(19) Europäisches Patentamt
European Patent Office
Office européen des brevets

(11) Numéro de publication : **0 431 991 B1**

# (12) FASCICULE DE BREVET EUROPEEN

(45) Date de publication du fascicule du brevet : **12.01.94 Bulletin 94/02**

(21) Numéro de dépôt : **90403047.5**

(22) Date de dépôt : **29.10.90**

(51) Int. Cl.$^5$ : **C07D 471/04,** A61K 31/44, // (C07D471/04, 221:00, 221:00)

## (54) Nouveaux dérivés de benzonaphtyridine-1,8 leur préparation et les compositions qui les contiennent.

(30) Priorité : **30.10.89 FR 8914203**
**10.07.90 FR 9008757**

(43) Date de publication de la demande : **12.06.91 Bulletin 91/24**

(45) Mention de la délivrance du brevet : **12.01.94 Bulletin 94/02**

(84) Etats contractants désignés : **AT BE CH DE DK ES FR GB GR IT LI LU NL SE**

(56) Documents cités :
**EP-A- 0 169 993**
**EP-A- 0 230 053**
**EP-A- 0 379 412**

(73) Titulaire : **Société anonyme dite: LABORATOIRE ROGER BELLON**
**159, avenue A. Peretti**
**F-92201 Neuilly-sur-Seine (FR)**

(72) Inventeur : **Antoine, Michel**
**184, Rue Nationale**
**F-75013 Paris (FR)**
Inventeur : **Barreau, Michel**
**24 bis, Avenue du Clos de Sénart**
**F-91230 Montgeron (FR)**
Inventeur : **Desconclois, Jean-François**
**12, Rue Beccaria**
**F-75012 Paris (FR)**
Inventeur : **Girard, Philippe**
**7, Rue du Bois-Gaudron Ollainville**
**F-91290 Arpajon (FR)**
Inventeur : **Picaut, Guy**
**152, Rue de Chevilly**
**F-94800 Villejuif (FR)**

(74) Mandataire : **Savina, Jacques et al**
**RHONE-POULENC SANTE, Service Brevets,**
**20 Avenue Raymond Aron**
**F-92165 Antony Cédex (FR)**

EP 0 431 991 B1

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Jouve, 18, rue Saint-Denis, 75001 PARIS

## Description

La présente invention concerne des dérivés de la benzo[b]naphtyridine-1,8 de formule générale :

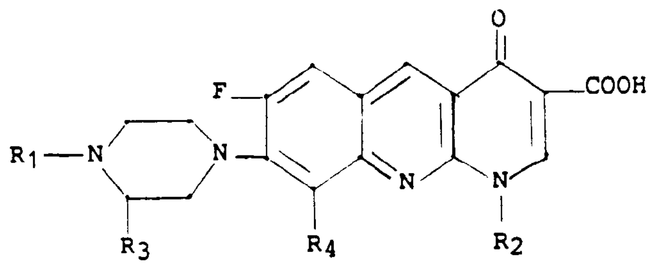

(I)

leurs sels, leur préparation et les compositions qui les contiennent.

Dans les brevets US 4 229 456 et 4 133 885 ont été décrits des dérivés de naphtyridine de structure :

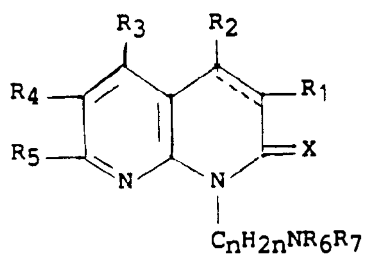

dans laquelle X peut être l'oxygène et deux des radicaux $R_1$ à $R_5$ adjacents peuvent former un cycle benzénique.

Ces produits sont utiles comme inhibiteurs des sécrétions gastriques acides.

La demande de brevet DE 3 302 126 décrit des hypotenseurs de formule générale :

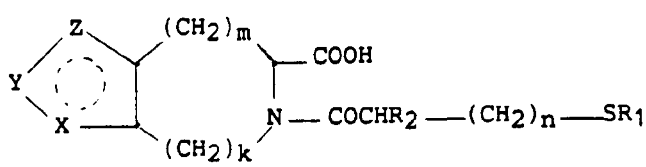

dans laquelle les radicaux X, Y et Z peuvent être O ou un radical $NR_4$ ou $CR_5$=$CR_5$ dans lequel les $R_5$ peuvent former un cycle benzénique.

Il a été trouvé que les produits de formule générale (I) dans laquelle :

- $R_1$ représente un atome d'hydrogène ou un radical hydroxy ou alcoyle,
- $R_2$ représente un atome d'hydrogène ou un radical alcoyle, fluoroalcoyle, cycloalcoyle contenant 3 à 6 atomes de carbone, alcoyloxy ou alcoylamino,
- $R_3$ représente un radical phényle ou phénylalcoyle éventuellement substitué par un ou plusieurs atomes d'halogène ou radicaux alcoyle, cycloalcoyle contenant 3 à 6 atomes de carbone, alcoyloxy, cyano, amino, alcoylamino, dialcoylamino, alcoyloxyalcoyle, hydroxyalcoyle, hydroxyalcoyloxy, méthylènedioxy, aminoalcoyle, alcoylaminoalcoyle ou dialcoylaminoalcoyle dont les parties alcoyle peuvent former avec l'atome d'azote auquel elles sont rattachées un hétérocycle à 5 ou 6 chainons ou représente un radical hétérocyclyle à 5 chaînons contenant 1 ou 2 hétéroatomes choisis parmi l'azote, l'oxygène ou le soufre, ou un radical N-alcoyl pyrrolyle, et
- $R_4$ représente un atome d'hydrogène ou un atome de fluor, ou bien
- $R_1$ est hydroxy, $R_2$ est méthyle, $R_3$ est un atome d'hydrogène et $R_4$ est défini comme ci-dessus,

et dont les radicaux alcoyle sont droits ou ramifiés et contiennent 1 à 4 atomes de carbone, ainsi que leurs sels, et leurs isomères, manifestent une activité antibactérienne particulièrement intéressante.

Les produits de formule générale (I) peuvent exister à l'état de forme hydratée, il est entendu que ces hydrates entrent aussi dans le cadre de la présente invention.

Dans la formule générale (I), lorsque $R_3$ représente un radical hétérocyclyle, ce dernier peut être avantageusement choisi parmi furyle, thiényle, pyrrolyle, N-alcoyl pyrrolyle, imidazolyle, pyrazolyle ou thiazolyle.

Selon l'invention, les produits de formule générale (I) peuvent être obtenus par substitution d'une pipérazine de formule générale :

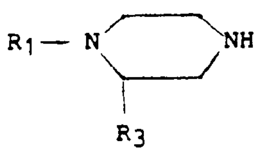

(II)

dans laquelle $R_1$ et $R_3$ sont définis comme précédemment, sur une benzo[b]naphtyridine-1,8 de formule générale :

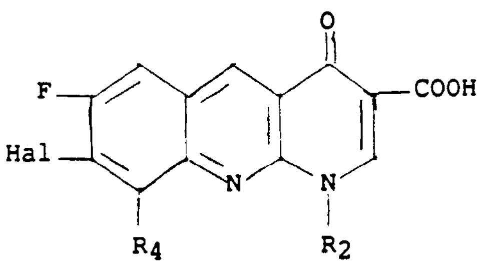

(III)

dans laquelle $R_2$ est défini comme précédemment, Hal est un atome de fluor, de chlore ou de brome si $R_4$ est hydrogène, ou bien Hal et $R_4$ sont simultanément des atomes de fluor, suivie éventuellement, si $R_1$ est un atome d'hydrogène et si l'on veut obtenir un dérivé de benzo[b]naphtyridine-1,8 dans laquelle $R_1$ est méthyle, de la transformation du produit obtenu en une (méthyl-4 pipérazinyl-1)-8 benzo[b]naphtyridine.

L'action du dérivé de la pipérazine de formule générale (II) s'effectue généralement en présence d'un excès de ce dérivé comme accepteur d'acide dans des solvants organiques convenables. Il est possible d'opérer avec ou sans solvant, à une température comprise entre 30 et 120°C. Lorsque l'on opère en présence d'un solvant, la réaction s'effectue avantageusement dans des solvants tels que la pyridine, le diméthylformamide, le diméthylsulfoxyde ou l'acétonitrile.

Il peut être également avantageux d'opérer en présence d'un accepteur d'acide comme par exemple une base organique azotée (triéthylamine notamment), un carbonate alcalin (carbonate de sodium par exemple) ou un hydroxyde de métal alcalin ou alcalino-terreux.

Il est entendu que, dans le cas où le symbole $R_2$ du produit de formule générale (III) est un atome d'hydrogène, ou lorsque $R_3$ contient un substituant amino, alcoylamino, aminoalcoyle ou alcoylaminoalcoyle, il est préférable de protéger préalablement le produit de départ. La protection et l'élimination du radical protecteur après la réaction, s'effectuent selon les méthodes habituelles.

La protection peut être réalisée par tout groupement compatible et dont la mise en oeuvre et l'élimination n'altère pas le reste de la molécule. Notamment, on opère selon les méthodes décrites par T.W. GREENE, Protective Groups in Organic Synthesis, A. Wiley-Interscience Publication (1981), ou par Mc OMIE, Protective Groups in Organic Chemistry, Plenum Press (1973).

A titre d'exemple, les groupements protecteurs peuvent être choisis parmi les radicaux triméthylsilyle, benzhydryle, tétrahydropyrannyle, formyle, acétyle, chloracétyle, trichloracétyle, trifluoroacétyle, éthoxycarbonyle, t.butoxycarbonyle, trichloréthoxycarbonyle.

Le cas échéant, l'opération subséquente de méthylation du radical pipérazinyle s'effectue avantageusement par action du formol en présence d'acide formique. On opère généralement en milieu aqueux, à une température comprise entre 90 et 100°C.

Selon l'invention, les dérivés de benzo[b]naphtyridine-1,8 de formule générale (I) peuvent être aussi obtenus à partir de l'ester correspondant de formule générale :

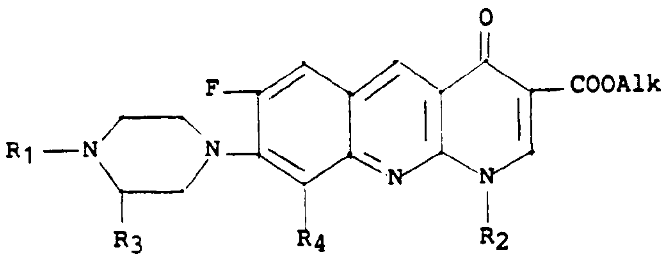

(IV)

dans laquelle $R_1$, $R_3$ et $R_4$ sont définis comme précédemment, $R_2$ est défini comme précédemment ou représente un radical alcoylamino protégé et Alk représente un radical alcoyle contenant 1 à 4 atomes de carbone en chaîne droite ou ramifiée, par toute méthode connue pour obtenir un acide à partir d'un ester sans toucher au reste de la molécule, suivie le cas échéant de l'élimination du groupement protecteur du radical alcoylamino, et/ou si l'on a obtenu un produit de formule générale (I) dans lequel $R_1$ est un atome d'hydrogène et si l'on veut obtenir le produit correspondant pour lequel $R_1$ est méthyle, suivie de la transformation du produit obtenu en une (méthyl-4 pipérazinyl-1)-8 benzo[b]naphtyridine.

La préparation de l'acide à partir de l'ester s'effectue généralement par hydrolyse acide. On opère avantageusement dans un mélange acide acétique - acide chlorhydrique, dans l'acide sulfurique ou dans l'acide méthanesulfonique à une température comprise entre 20 et 100°C. Il est également possible d'opérer par saponification en présence de potasse ou de soude, en milieu hydro-alcoolique, à une température comprise entre 20 et 80°C.

Le cas échéant, la méthylation du radical pipérazinyle s'effectue, comme décrit précédemment.

Lorsque $R_2$ représente un radical alcoylamino protégé, le radical protecteur peut être tout groupement protecteur d'amino compatible avec la molécule. Il est particulièrement avantageux de choisir un radical protecteur qui peut être éliminé simultanément à l'hydrolyse de l'ester.

Le dérivé de la benzo[b]napthyridine-1,8 de formule générale (III) peut être obtenu à partir de l'ester correspondant de formule générale :

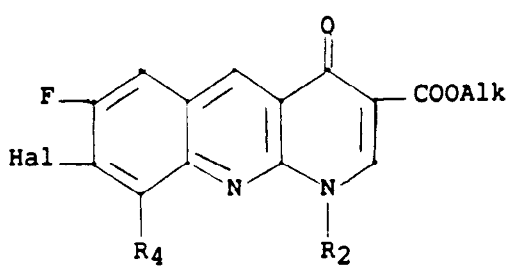

(V)

dans laquelle $R_4$, Hal et Alk sont définis comme précédemment, et $R_2$ est défini comme précédemment ou représente un radical alcoylamino protégé, par toute méthode connue pour obtenir un acide à partir d'un ester, sans toucher au reste de la molécule, suivie éventuellement de l'élimination du groupement protecteur du radical alcoylamino.

On opère notamment dans les conditions décrites précédemment pour obtenir un produit de formule générale (I) à partir d'un ester de formule générale (IV).

L'ester dérivé de la benzo[b]naphtyridine-1,8 de formule générale (V) peut être préparé par action de l'amino-3 triazine-1,2,4 pour obtenir un produit pour lequel $R_2$ est un atome d'hydrogène ou par action d'un produit de formule générale :

$$R_2\text{-}NH_2 \qquad \text{(VI)}$$

dans laquelle $R_2$ est alcoyle, fluoroalcoyle, cycloalcoyle, alcoyloxy ou alcoylamino éventuellement protégé sur un dérivé de la quinoléine de formule générale :

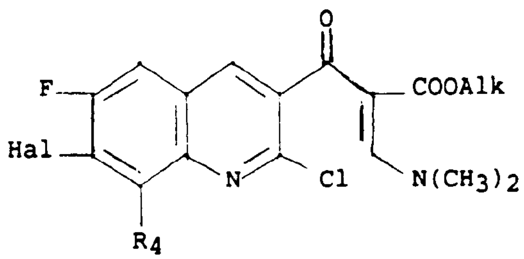

(VII)

dans laquelle $R_4$, Hal et Alk sont définis comme précédemment, suivie de la cyclisation par action d'un agent accepteur d'acide.

Généralement, la réaction de l'amino-3 triazine-1,2,4 ou du produit de formule générale (VI) est mise en oeuvre dans un solvant organique comme un alcool (éthanol, méthanol par exemple) ou un solvant chloré (trichlorométhane par exemple), à une température comprise entre 10 et 25°C.

La cyclisation s'effectue dans un alcool à chaîne droite ou ramifiée contenant 1 à 4 atomes de carbone, à une température comprise entre 20°C et la température de reflux du mélange réactionnel.

L'agent accepteur d'acide peut être notamment choisi parmi les bases azotées (triéthylamine par exemple), le diaza-1,8-bicy clo[5.4.0] undécène-7, ou un excès de l'amine employée.

Le dérivé de la quinoléine de formule générale (VII) peut être obtenu à partir du cétoester de formule générale :

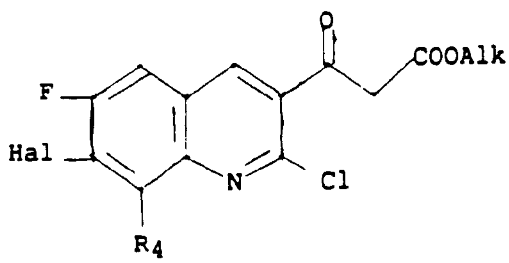

(VIII)

dans laquelle $R_4$, Hal et Alk sont définis comme précédemment, par action d'un NN-diméthylformamide acétal de formule générale :

$$(CH_3)_2N - CH(OAlk_1)_2 \qquad (IX)$$

dans laquelle $Alk_1$ est un radical alcoyle contenant 1 à 4 atomes de carbone en chaîne droite ou ramifiée.

La réaction s'effectue généralement dans un solvant organique tel qu'un ester (acétate d'éthyle par exemple) à une température comprise entre 60 et 75°C.

Le cétoester de formule générale (VIII) pour lequel $R_4$ est un atome d'hydrogène et Hal est défini comme précédemment peut être obtenu à partir de l'acide dichloro-2,7 fluoro-6 quinoléine carboxylique-3 ou de l'acide chloro-2 difluoro-6,7 quinoléine carboxylique-3 comme décrit ci-après à l'exemple 1 ou à partir de l'acide bromo-7 chloro-2 fluoro-6 quinoléine carboxylique-3 par analogie avec cette méthode. Dans ce cas, la bromo-3 fluoro-4 aniline utilisée comme produit de départ, peut être préparée selon la méthode décrite par W.B. Austin et coll., J. Org. Chem., 46(11), 2280 (1981).

Le cétoester de formule générale (VIII) pour lequel $R_4$ et Hal sont simultanément des atomes de fluor, peut être obtenu à partir de l'acide chloro-2 trifluoro-6,7,8 carboxylique-3 comme décrit ci-après, à l'exemple 25.

Le dérivé de benzo[b]naphtyridine-1,8 de formule générale (IV) peut être obtenu à partir de la benzo[b]naphtyridine de formule générale (V), par substitution d'un dérivé de la pipérazine de formule générale (II).

Lorsque $R_3$ représente un radical phényle substitué par amino, le dérivé de benzo [b] naphtyridine de formule générale (IV) peut aussi être obtenu par réduction du dérivé nitré correspondant, comme décrit ci-après à l'exemple 35.

On opère avantageusement dans les conditions décrites précédemment pour obtenir un produit de formule générale (I) à partir d'une pipérazine de formule générale (II) et d'une benzo[b] naphtyridine-1,8 de formule générale (III).

La pipérazine de formule générale (II) peut être obtenue par application des méthodes décrites par :

- E. Jucker et coll., Helv. Chim. Acta, 45(7), 2383 (1962) ;
- demande de brevet européen 230 053

- demande de brevet FR 2 351 108
- demande de brevet japonais 01 117 869
- Toschio Uno et Coll., J. Het. Chem., 26 393 (1988) ;
- W.R. Roderick et Coll., J. Med. Chem., 9, 181 (1966) ;
- J.D. Behan et Coll, J. Org. Chem, 26, 3379 (1961) ;
- J.W.H. Watthey et Coll., J. Med. Chem.,26, 1116 (1983) ;
- L.J. Kitchen et Coll., J. Am. Chem. Soc., 69, 854 (1947) et J. Org. Chem., 8, 342 (1943) ;
- J.D. Behun et Coll., J. Org. Chem., 26, 3379 (1961)
- ou par application ou par analogie avec les méthodes décrites ci-après dans les exemples. Il est entendu qu'il est parfois nécessaire d'effectuer la chromatographie de la pipérazine obtenue.

Les pipérazines de formule générale (II) pour lesquelles $R_1$ est un radical hydroxy et $R_3$ est autre que l'atome d'hydrogène, peuvent être préparées par oxydation de la pipérazine correspondantes dans laquelle l'atome d'azote en position -4 a été préalable-ment protégé, selon la méthode décrite par A.J. Biloski et Coll., Synthesis, 537 (1983).

Lorsque $R_3$ représente un radical phényle substitué par un radical dialcoylaminométhyle, la pipérazine de formule générale (II) peut être obtenue à partir d'une (hydroxyphényl)-2 pipérazine dont les atomes d'azote ont été préalablement protégés par tout groupement compatible, par application de la méthode décrite par M. Tromontini, Synthesis, 703-775 (1973).

Selon l'invention, lorsque l'on veut obtenir les isomères des dérivés de la benzonaphtyridine de formule générale (I), on effectue la séparation des formes isomères des pipérazines de formule générale (II) par toute méthode connue et compatible avec la molécule. A titre d'exemple, la séparation s'effectue par acylation au moyen d'un acide ou d'un dérivé réactif d'un acide chiral, séparation des isomères par chromatographie liquide hautes performances, puis désacylation par hydrolyse acide.

Dans les exemples qui suivent, on appelle isomère (-) l'isomère(S) du dérivé de la benzonaphtyridine de formule générale (I) dont le pouvoir rotatoire en solution dans l'acide acétique est négatif, obtenu à partir du dérivé de la pipérazine dont le pouvoir rotatoire dans l'éthanol est positif ; on appelle isomère (+), l'isomère (R) du dérivé de la benzonaphtyridine de formule générale (I) dont le pouvoir rotatoire en solution dans l'acide acétique est positif, obtenu à partir du dérivé de la pipérazine dont le pouvoir rotatoire, en solution dans l'éthanol, est négatif.

Il est également possible d'effectuer directement la synthèse de la pipérazine chirale, comme décrit ci-après aux exemples 20 et 21.

Les nouveaux produits selon la présente invention ainsi que leurs intermédiaires de synthèse peuvent être éventuellement purifiés par des méthodes physiques telles que la cristallisation ou la chromatographie.

Les produits selon la présente invention peuvent être transformés en sels métalliques ou en sels d'addition avec les bases azotées selon les méthodes connues en soi. Ces sels peuvent être obtenus par action d'une base métallique (par exemple alcaline ou alcalino-terreuse), de l'ammoniac ou d'une amine sur un produit selon l'invention dans un solvant approprié tel qu'un alcool, un éther ou l'eau ou par réaction d'échange avec un sel d'un acide organique. Le sel formé précipite après concentration éventuelle de sa solution, il est séparé par filtration, décantation ou lyophilisation.

Les nouveaux produits selon l'invention peuvent être également transformés en sels d'addition avec les acides. Les produits de formule générale (I) obtenus sous forme de ces sels, peuvent être libérés et transformés en sels d'autres acides selon les méthodes habituelles.

Comme exemples de sels pharmaceutiquement acceptables, peuvent être cités les sels avec les métaux alcalins (sodium, potassium, lithium) ou avec les métaux alcalino-terreux (magnésium, calcium), le sel d'ammonium, les sels de bases azotées (éthanolamine, diéthanolamine, triméthylamine, triéthylamine, méthylamine, propylamine, diisopropylamine, NN-diméthyléthanolamine, benzylamine, dicyclohexylamine, N-benzyl-β-phénéthylamine, NN'-dibenzyléthylènediamine, diphénylènediamine, benzhydrylamine, quinine, choline, arginine, lysine, leucine, dibenzylamine), ainsi que les sels d'addition avec des acides minéraux (chlorhydrates, bromhydrates, sulfates, nitrates, phosphates) ou organiques (succinates, fumarates, maléates, méthanesulfonates, p.toluènesulfonate, iséthionates).

Les nouveaux dérivés de benzo[b]naphtyridine-1,8 de formule générale (I) selon la présente invention et leurs sels pharmaceutiquement acceptables présentent des propriétés antibactériennes particulièrement intéressantes. Ils manifestent une activité remarquable in vitro et in vivo sur les germes gram-positifs et d'une manière générale sur les germes responsables de la plupart des infections des voies aériennes hautes et basses.

In vitro, les produits de formule générale (I) se sont montrés actifs à une concentration comprise entre 0,12 et 4 μg/cm$^3$ sur staphylococcus aureus IP 8203.

In vivo, les produits de formule générale (I) se sont montrés actifs sur les infections expérimentales de la

souris à staphylococcus aureus IP 8203 à des doses comprises entre 2 et 200 mg/kg par voie sous cutanée ou à des doses comprises entre 4 et 150 mg/kg par voie orale.

Un autre intérêt des produits selon l'invention est leur faible toxicité. Leur $DL_{50}$ est généralement supérieure à 500 mg/kg par voie sous cutanée chez la souris.

Par ailleurs les produits selon l'invention se sont révélés particulièrement intéressants pour la prophylaxie et le traitement du SIDA (syndrome d'immunodéficience acquise) et de syndromes associés [ARC (AIDS related complex)].

Les produits étudiés inhibent l'effet cytopathogène du virus HIV en culture cellulaire à des concentrations dépourvues d'effet cytotoxique ou cytostatique.

Activité vis-à-vis de l'effet cytopathogène du virus HIV.

Les produits en poudre ont été mis en solution à raison de 2 mg de produit par ml (environ $4x10^{-3}$ M) dans un mélange de diméthylsulfoxide (DMSO) et de L-lysine (base) à (1:19, vol:vol). On ajoute d'abord 1 volume de DMSO et on solubilise le produit au mieux puis on ajoute 19 volumes d'une solution de L-lysine base à $4x10^{-3}$ M dans de l'eau distillée. Le mélange est alors maintenu 15 minutes à 60°C. De cette manière on obtient une solution stock de produit à 5% de DMSO et contenant un rapport molaire (produit/lysine) proche de 1. Le test est réalisé sur la lignée lymphoblastoïde CEM clone 13. Dans une microplaque de 96 puits on dépose 25 µl/puit d'une solution de produit à tester dans du tampon phosphate isotonique (TPI) ou de TPI seul dans le cas des contrôles. Les produits sont étudiés à différentes concentrations (souvent 8), à raison de 6 puits par concentration. On ajoute alors 125 µl d'une suspension de cellules CEM (entre 5 et $8x10^4$ cellules par mil dans le milieu RPMI contenant 10 % de sérum de veau foetal, 100 UI/ml de pénicilline, 100 µg/ml de streptomycine et 2 µmoles/ml de glutamine et les microplaques sont incubées une heure à 37°C, sous une atmosphère contenant 5 % de gaz carbonique. Pour chaque concentration, l'essai est partagé en deux parties : une partie (3 puits) sur cellules infectées, pour la détermination de l'activité antivirale et l'autre partie (3 puits) sur cellules non infectées, pour déterminer la cytotoxicité des produits. On infecte alors la première série avec HIV-1 (100 µl par puit d'une suspension de virus LAV-1-BRU contenant 200-300 TCID50) tandis que l'autre série reçoit 100 µl de milieu RPMI tel que défini précédemment. Au bout de 7 jours d'incubation, 150 µl de cellules sont prélevés pour mesurer la viabilité cellulaire [déterminée selon une modification de la technique décrite par R. Pauwels et coll., J. Virol. Meth., 20, 309-321 (1988)]. On ajoute à ce prélèvement 10 µl d'une solution contenant 7 mg de MTT [bromure de (4,5-diméthyl thiazol-2 yl)-2,5-diphényltétrazolium] par ml de tampon phosphate isotonique. Après 3 heures d'incubation à 37°C le surnageant est enlevé.Le MTT est converti en un sel de formazan (bleu) à l'intérieur des cellules vivantes et proportionnellement à leur nombre, alors que rien n'apparaît dans les puits ne contenant plus que des cellules mortes. On ajoute alors 100 µl d'isopropanol (contenant 0,04 mole/l d'acide chlorhydrique) et les microplaques sont agitées jusqu'à la solubilisation du bleu de formazan. L'absorbance à 540 nm est lue avec un lecteur automatique de réactions ELISA en microplaques. Cette absorbance est proportionnelle à la quantité de cellules vivantes. Les résultats obtenus au jour 14 sont donnés dans le tableau I ci-après.

Tableau I

| Produit étudié | Concentration du produit étudié µg/ml | Résultats à J14 Densité optique (DO) | | % de protection |
|---|---|---|---|---|
| | | cellules infectées | cellules non infectées | |
| Exemple 1 | 0 | 321 | 1151 | 0 % |
| | 0,014 | 300 | 1285 | -2 % |
| | 0,04 | 285 | 1310 | -4 % |
| | 0,12 | 335 | 1285 | 1 % |
| | 0,37 | 425 | 1188 | 12 % |
| | 1,11 | 842 | 1010 | 76 % |
| | 3,33 | 747 | 867 | 78 % |
| | 10 | 850 | 844 | 101 % |
| Exemple 21 | 0 | 489 | 1227 | 0 % |
| | 0,014 | 543 | 1196 | 8 % |
| | 0,04 | 1086 | 1228 | 81 % |
| | 0,12 | 1104 | 1162 | 91 % |
| | 0,37 | 1132 | 1145 | 98 % |
| | 1,11 | 945 | 1021 | 86 % |

Le taux de protection (en %) d'un produit donné est déterminé par la formule :

$$\frac{\text{DO(cell. traitées et inf.)} - \text{DO(cell. non traitées et inf.)}}{\text{DO(cell. traitées et non inf.)} - \text{DO(cell. non traitées et inf.)}}$$

D'un interêt particulier sont les produits de formule générale (I) dans laquelle :

- $R_1$ représente un atome d'hydrogène ou un radical hydroxy ou alcoyle,
- $R_2$ représente un atome d'hydrogène ou un radical alcoyle, fluoroalcoyle, cycloalcoyle contenant 3 à 6 atomes de carbone ou alcoyloxy,
- $R_3$ représente un radical phényle ou phénylalcoyle éventuellement substitué par un ou plusieurs atomes d'halogène ou radicaux alcoyle, cycloalcoyle contenant 3 à 6 atomes de carbone, alcoyloxy, cyano, amino, alcoyloxyalcoyle, hydroxyalcoyloxy, méthylènedioxy, ou représente un radical hétérocyclyle à 5 chaînons contenant 1 ou 2 hétéroatomes choisis parmi l'azote, l'oxygène ou le soufre, ou un radical N-alcoyle pyrrolyle, et
- $R_4$ représente un atome d'hydrogène ou un atome de fluor, ou bien
- $R_1$ est hydroxy, $R_2$ est méthyl, $R_3$ est un atome d'hydrogène et $R_4$ est défini comme ci-dessus.

Parmi ces produits les dérivés de benzonaphtyridine suivants sont plus spécialement intéressants :

- acide fluoro-7 méthyl-1 oxo-4 (phényl-3 pipérazinyl-1)-8 dihydro-1,4 benzo [b] naphtyridine-1,8 carboxylique-3 sous ses formes isomères et leurs mélanges;
- acide fluoro-7 méthyl-1 [(méthyl-4 phényl)-3 pipérazinyl-1]-8 oxo-4 dihydro-1,4 benzo [b] naphtyridine-1,8 carboxylique-3 sous ses formes isomères et leurs mélanges;
- acide fluoro-7 [(méthoxy-4 phényl)-3 pipérazinyl-1]-8 méthyl-1 oxo-4 dihydro-1,4 benzo [b] naphtyridine-1,8 carboxylique-3 sous ses formes isomères et leurs mélanges;
- acide difluoro-7,9 [(méthoxy-4 phényl)-3 pipérazinyl-1]-8 méthyl-1 oxo-4 dihydro-1,4 benzo [b] naphtyridine-1,8 carboxylique-3 sous ses formes isomères et leurs mélanges;
- acide fluoro-7 [(furyl-2)-3 pipérazinyl-1]-8 méthyl-1 oxo-4 dihydro-1,4 benzo[b]naphtyridine-1,8 carboxylique-3 sous ses formes isomères et leurs mélanges;

Les exemples suivants donnés à titre non limitatif illustrent la présente invention.

EXEMPLE 1 (RP 65358)

Une suspension de 1,84 g d'acide chloro-8 fluoro-7 méthyl-1 oxo-4 dihydro-1,4 benzo [b] naphtyridine-1,8 carboxylique-3 dans 20 $cm^3$ de pyridine, 5,83 g de phényl-2 pipérazine (RS) et 1,24 g de triéthylamine est chauffée à une température voisine de 115°C pendant 5 heures. Après refroidissement à environ 20°C, le précipité formé est essoré, lavé par 2 fois 5 $cm^3$ de pyridine, 2 fois 5 $cm^3$ d'alcool isopropylique, 2 fois 5 $cm^3$ d'éthanol et 1 fois 20 $cm^3$ d'éther éthylique. Après 1 recristallisation dans un mélange de 40 $cm^3$ de diméthylformamide et de 40 $cm^3$ d'éthanol, on obtient 0,920 g d'acide fluoro-7 méthyl-1 oxo-4 (phényl-3 pipérazinyl-1)-8 dihydro-1,4 benzo [b] naphtyridine-1,8 carboxylique-3-(RS) sous forme d'un solide jaune fondant à 265°C.

L'acide chloro-8 fluoro-7 méthyl-1 oxo-4 dihydro-1,4 benzo[b]naphtyridine-1,8 carboxylique-3 peut être préparé de la manière suivante :

Une suspension de 15 g de chloro-8 éthoxycarbonyl-3 fluoro-7 méthyl-1 oxo-4 dihydro-1,4 benzo[b]naphtyridine-1,8 dans 150 $cm^3$ d'acide acétique et 150 $cm^3$ d'acide chlorhydrique en solution aqueuse à 17,5 % est chauffée à une température voisine de 100°C sous agitation pendant 4 heures. Après refroidissement à température voisine de 20°C, le produit est essoré, lavé par 2 fois 100 $cm^3$ d'eau, lavé par 2 fois 150 $cm^3$ d'éthanol puis 2 fois 100 $cm^3$ d'éther éthylique. On obtient 12,7 g d'acide chloro-8 fluoro-7 méthyl-1 oxo-4 dihydro-1,4 benzo[b]naphtyridine-1,8 carboxylique-3 sous forme d'un solide beige se sublimant à 400-405°C qui est utilisé sans autre purification pour les étapes ultérieures.

La chloro-8 éthoxycarbonyl-3 fluoro-7 méthyl-1 oxo-4 dihydro-1,4 benzo[b]naphtyridine-1,8 est préparée de la manière suivante :

Dans une suspension sous agitation de 19,3 g de (dichloro-2,7 fluoro-6 quinoléinecarbonyle-3)-2 diméthylamino-3 acrylate d'éthyle dans 250 $cm^3$ d'éthanol, maintenue entre 10 et 15°C, on fait barboter de la méthylamine jusqu'à absorption de 16 g de gaz. On laisse la température remonter à environ 20°C, ajoute 0,8 g de diaza-1,8-bicyclo[5.4.0.]undécène-7 (DBU) et chauffe à une température voisine de 75°C pendant 2 heures. Après refroidissement à environ 20°C, le produit est essoré, lavé par 2 fois 150 $cm^3$ d'éthanol et 2 fois 100 $cm^3$ d'éther éthylique. On obtient 15 g de chloro-8 éthoxycarbonyl-3 fluoro-7 méthyl-1 oxo-4 dihydro-1,4 benzo[b]naphtyridine-1,8 sous forme d'un solide jaune fondant à 360-362°C qui est utilisé sans autre purification pour les étapes ultérieures.

Le (dichloro-2,7 fluoro-6 quinoléinecarbonyle-3)-2 diméthylamino-3 acrylate d'éthyle est préparé de la manière suivante :

Une suspension de 16,5 g de (dichloro-2,7 fluoro-6 quinolyl-3)-3 oxo-3 propionate d'éthyle dans 160 $cm^3$ d'acétate d'éthyle et 19 $cm^3$ de N,N-diméthylformamide diméthylacétal est chauffée à une température voisine de 75°C, sous agitation, pendant 2 heures. Le mélange réactionnel est concentré à sec sous pression réduite (20 kPa) à 50°C. L'extrait sec est repris par 50 $cm^3$ d'éther isopropylique, essoré, lavé par 2 fois 10 $cm^3$ d'éther isopropylique. On obtient 16,57 g de (dichloro-2,7 fluoro-6 quinoléinecarbonyle-3)-2 diméthylamino-3 acrylate d'éthyle sous forme d'un solide orangé fondant à 122°C. Ce produit est utilisé sans autre purification pour les étapes ultérieures.

Le (dichloro-2,7 fluoro-6 quinolyl-3)-3 oxo-3 propionate d'éthyle est préparé de la manière suivante :

Une suspension de 38,75 g d'acide dichloro-2,7 fluoro-6 quinoléine carboxylique-3 dans 410 $cm^3$ de trichlorométhane et 24 $cm^3$ de chlorure de thionyle est chauffée à une température voisine de 60°C, sous agitation, pendant 6 heures. La solution obtenue est concentrée à sec sous pression réduite (20 kPa) à 50°C. L'extrait sec est repris 2 fois par au total 200 $cm^3$ de toluène et concentré de nouveau sous pression réduite dans les mêmes conditions que précédemment. Le solide jaune obtenu fondant à 124°C est dissous dans 230 $cm^3$ de tétrahydrofuranne anhydre. La solution obtenue est introduite, goutte à goutte, sous agitation, en 30 minutes, entre 5 et 10°C, dans 200 $cm^3$ d'une solution de chélate de magnésium dans le tétrahydrofuranne. On laisse la température remonter à 20°C et agite 1 heure et demie à cette température. La solution obtenue est introduite, goutte à goutte, sous forte agitation à une température voisine de 5°C, dans 1 litre d'acide sulfurique 0,5N. On laisse la température de la suspension obtenue remonter à 20°C et agite encore 2 heures à cette température. On extrait par 1 litre d'acétate d'éthyle, filtre les phases organique et aqueuse sur silice diatomée pour filtration ce qui permet d'éliminer un léger insoluble, extrait la phase aqueuse encore 2 fois par 500 $cm^3$ d'acétate d'éthyle. Les extraits organiques réunis sont lavés par 2 fois 500 $cm^3$ d'eau, séchés sur sulfate de magnésium, filtrés et concentrés à sec sous pression réduite (20 kPa) à 40°C. Le résidu est repris par 100 $cm^3$ d'éther isopropylique à 20°C, essoré et lavé par 2 fois 30 $cm^3$ d'éther isopropylique. On obtient 40,55

g de (dichloro-2,7 fluoro-6 quinolyl-3)-3 oxo-3 propionate d'éthyle sous forme d'un solide beige fondant à 112-114°C. Ce produit est utilisé sans autre purification pour les étapes ultérieures.

Préparation du chélate de magnésium du monomalonate d'éthyle:

A 6,9 g de magnésium en tournure on ajoute progressivement 5 $cm^3$ d'éthanol absolu, 0,2 $cm^3$ de tétrachlorométhane et 2 g de monomalonate d'éthyle. Après échauffement, on ajoute, en 15 minutes, une solution de 23,8 g de monomalonate d'éthyle dans 450 $cm^3$ d'éthanol. Le mélange est chauffé 20 heures à une température voisine de 78°C, concentré sous pression réduite (20 kPa) à 50°C. Le résidu est repris par 2 fois 100 $cm^3$ de toluène et concentré sous pression réduite dans les mêmes conditions que précédemment. La poudre grise obtenue est mise en solution par addition de tétrahydrofuranne anhydre de façon à obtenir un volume total de 200 $cm^3$.

Le monomalonate d'éthyle a été préparé selon la méthode décrite par D.S. Breslow, E. Baumgarten, C.R. Hauser., J. Am. Chem. Soc., 66, 1287 (1944) et distillé sous pression réduite (point d'ébullition=132°C/2,7 kPa).

L'acide dichloro-2,7 fluoro-6 quinoléine carboxylique-3 est préparé de la manière suivante :

A une suspension, sous agitation, refroidie à 10°C, de 69,5 g de dichloro-2,7 fluoro-6 formyl-3 dihydro-1,4 quinoléine dans 282 $cm^3$ de potasse aqueuse 2N et 282 $cm^3$ d'eau, on ajoute en 1 heure en maintenant la température entre 10 et 14°C, une solution de 89,3 g de permanganate de potassium dans 1,4 litre d'eau. On laisse la température remonter à environ 20°C et agite encore 30 minutes à cette température. On ajoute 26 g de dithionite de sodium, agite 10 minutes à température voisine de 20°C, filtre sur silice diatomée pour filtration, lave par 2 fois 250 $cm^3$ d'eau. Le filtrat et les phases aqueuses de lavage sont réunis et additionnés de 90 $cm^3$ d'acide chlorhydrique en solution aqueuse à 35 %. Le précipité formé est extrait par 4 fois 500 $cm^3$ d'acétate d'éthyle. Les extraits organiques réunis sont lavés par 3 fois 500 $cm^3$ d'eau, séchés sur sulfate de magnésium, filtrés et concentrés sous pression réduite (20 kPa) à 50°C. Le résidu est repris par 350 $cm^3$ d'éther éthylique, essoré, lavé par 2 fois 200 $cm^3$ d'éther éthylique. On obtient 45 g d'acide dichloro-2,7 fluoro-6 quinoléine carboxylique-3 sous forme d'un solide beige fondant à 230°C qui est utilisé sans autre purification pour les étapes ultérieures.

La dichloro-2,7 fluoro-6 formyl-3 dihydro-1,4 quinoléine a été préparée de la manière suivante :

A un mélange de 250 $cm^3$ de trichlorométhane et de 54 $cm^3$ de diméthylformamide, on ajoute en 30 minutes, sous agitation, entre 10 et 15°C, 55,6 $cm^3$ de chlorure de phosphoryle et agite 1 heure à température voisine de 20°C. A la solution obtenue, on ajoute en 10 minutes, à environ 20°C, progressivement, sous forte agitation, 52 g de chloro-7 fluoro-6 dihydro-3,4 carbostyrile. La suspension obtenue est chauffée à une température voisine de 60°C et maintenue encore 2 heures, sous agitation à cette température. Le mélange réactionnel est concentré sous pression réduite (20 kPa) à 50°C jusqu'à obtention d'un mélange pâteux. Sous forte agitation, on ajoute un mélange de 250 $cm^3$ d'eau et 250 g de glace pilée. Le solide obtenu est essoré à environ 5°C et lavé par 4 fois 125 $cm^3$ d'eau à 5°C. Le produit humide obtenu et 58 g d'acétate de sodium sont ajoutés simultanément, en 1 heure, à 500 $cm^3$ d'eau à 90°C de manière à maintenir le pH à environ 6. On agite encore 15 minutes à 90°C, laisse la température redescendre à environ 50°C, essore à cette température et lave par 3 fois 250 $cm^3$ d'eau à environ 20°C. On obtient 54,3 g de dichloro-2,7 fluoro-6 formyl-3 dihydro-1,4 quinoléine sous forme d'un solide jaune fondant à 260°C qui est utilisé tel quel pour les étapes ultérieures.

Le chloro-7 fluoro-6 dihydro-3,4 carbostyrile est préparé de la manière suivante :

A 174,4 g de chloro-3' fluoro-4' N-chloro-3 propionanilide, on ajoute sous forte agitation en 5 minutes, 350 g de chlorure d'aluminium. Le mélange solide est chauffé en 30 minutes à environ 60°C. La température monte d'elle-même à environ 80°C et le mélange réactionnel devient liquide. On chauffe alors à 110°C en 15 minutes et maintient entre 110 et 120°C pendant 3 heures. Le mélange réactionnel (à environ 110°C) est versé, en 10 minutes, sous forte agitation, dans un mélange de 550 $cm^3$ d'acide chlorhydrique à 35 % et de 500 g de glace pilée. On laisse la température remonter à 20°C, essore, lave par 6 fois 500 $cm^3$ d'eau.

Le produit humide est recristallisé dans 1,2 litre d'éthanol. On obtient 108 g de chloro-7 fluoro-6 dihydro-3,4 carbostyrile sous forme d'un solide beige fondant à 215°C.

La chloro-3' fluoro-4' N-chloro-3 propionanilide a été préparée de la manière suivante :

A une solution, à température voisine de 55°C, de 291 g de chloro-3 fluoro-4 aniline dans 500 $cm^3$ d'acétone, on ajoute, sous agitation, en 35 minutes, une solution de 127 g de chlorure de l'acide chloro-3 propionique dans 200 $cm^3$ d'acétone et maintient à cette température pendant 2 heures. Après refroidissement à environ 20°C, l'insoluble est éliminé par filtration et lavé par 2 fois 200 $cm^3$ d'acétone. Le filtrat et les lavages réunis sont versés sous agitation dans 2 litres d'eau et 1 kg de glace. On laisse la température remonter à environ 20°C, extrait par 4 fois 500 $cm^3$ de dichlorométhane. Les extraits organiques réunis sont lavés par 3 fois 500 $cm^3$ d'eau, séchés sur sulfate de magnésium, agités 15 minutes avec 6 g de charbon végétal Norit, filtrés sur silice diatomée pour filtration et concentrés sous pression réduite (2,7 kPa) à 50°C. Le solide obtenu est recristallisé dans un mélange de 133 $cm^3$ de cyclohexane et de 67 $cm^3$ d'éther isopropylique. On obtient 176 g de chloro-3'fluoro-4' N-chloro-3 propionanilide sous forme d'un solide beige fondant à 94°C qui est utilisé tel

quel pour les étapes ultérieures.

EXEMPLE 2

L'acide cyclopropyl-1 fluoro-7 oxo-4 (phényl-3 pipérazinyl-1)-8 dihydro-1,4 benzo [b] naphtyridine-1,8 carboxylique-3-(RS) a été préparé dans les conditions de l'exemple 11 ci-après, mais à partir de 2 g d'acide chloro-8 cyclopropyl-1 fluoro-7 oxo-4 dihydro-1,4 benzo [b] naphtyridine-1,8 carboxylique-3, de 4,9 g de phényl-2 pipérazine-(RS) et de 1,7 $cm^3$ de triéthylamine. Après recristallisation dans un mélange de 40 $cm^3$ de diméthylformamide et de 50 $cm^3$ d'éthanol, on obtient 1,16 g d'acide cyclopropyl-1 fluoro-7 oxo-4 (phényl-3 pipérazinyl-1)-8 dihydro-1,4 benzo [b] naphtyridine-1,8 carboxylique-3-(RS), sous forme d'un solide jaune fondant à 254°C.

L'acide chloro-8 cyclopropyl-1 fluoro-7 oxo-4 dihydro-1,4 benzo[b]naphtyridine-1,8 carboxylique-3 est préparé dans les conditions de l'exemple 1, mais à partir de 6,1 g de chloro-8 cyclopropyl-1 éthoxycarbonyl-3 fluoro-7 oxo-4 dihydro-1,4 benzo[b]naphtyridine-1,8. On obtient 4,85 g d'acide chloro-8 cyclopropyl-1 fluoro-7 oxo-4 dihydro-1,4 benzo[b]naphtyridine-1,8 carboxylique-3 sous forme d'un solide jaune fondant à 330°C qui est utilisé sans autre purification pour les étapes ultérieures.

La chloro-8 cyclopropyl-1 éthoxycarbonyl-3 fluoro-7 oxo-4 dihydro-1,4 benzo[b]naphtyridine-1,8 est préparée dans les conditions suivantes :

Une solution de 20,6 g de (dichloro-2,7 fluoro-6 quinoléinecarbonyle-3)-2 diméthylamino-3 acrylate d'éthyle et de 6 g de cyclopropylamine dans 100 $cm^3$ de trichlorométhane est agitée à une température voisine de 20°C pendant 24 heures. Le mélange réactionnel est concentré sous pression réduite (2,7 kPa) à 50°C. Le résidu est repris par 180 $cm^3$ d'éthanol et 10 g de DBU et la solution obtenue chauffée à une température voisine de 78°C pendant 4 heures. Après refroidissement à une température voisine de 20°C, le précipité obtenu est essoré et lavé par 2 fois 60 $cm^3$ d'éthanol. On obtient 13,65 g de chloro-8 cyclopropyl-1 éthoxycarbonyl-3 fluoro-7 oxo-4 dihydro-1,4 benzo[b]naphtyridine-1,8 sous forme d'un solide jaune pâle fondant à 256°C qui est utilisé sans autre purification pour les étapes ultérieures.

EXEMPLE 3

L'acide fluoro-7 (fluoro-2 éthyl)-1 oxo-4 (phényl-3 pipérazinyl-1)-8 oxo-4 dihydro-1,4 benzo [b] naphtyridine-1,8 carboxylique-3-(RS) a été préparé dans les conditions suivantes:

Une suspension de 0,95 g d'acide difluoro-7,8 (fluoro-2 éthyl)-1 oxo-4 dihydro-1,4 benzo [b] naphtyridine-1,8 carboxylique-3 et de 1,05 g de phényl-2 pipérazine-(RS) dans 10 $cm^3$ de diméthylsulfoxyde est chauffée, à une température voisine de 100°C, sous agitation, pendant 20 minutes. Après refroidissement à environ 20°C, l'insoluble est essoré et lavé par 3 fois 10 $cm^3$ d'éthanol à environ 70°C. On obtient 1,3 g du produit attendu, sous forme d'un solide jaune se décomposant à 305°C.

L'acide difluoro-7,8 (fluoro-2 éthyl)-1 oxo-4 dihydro-1,4 benzo[b]naphtyridine-1,8-carboxylique-3 a été préparé de la manière suivante:

Une suspension de 2,3 g d'éthoxycarbonyl-3 difluoro-7,8 (fluoro-2 éthyl)-1 oxo-4 dihydro-1,4 benzo[b]naphtyridine-1,8 dans 20 $cm^3$ d'acide acétique et 20 $cm^3$ d'acide chlorydrique 5N est chauffée, sous agitation, à une température voisine de 100°C pendant 1 heure. L'insoluble est essoré à environ 70°C et lavé par 3 fois 10 $cm^3$ d'eau et 3 fois 10 $cm^3$ d'éthanol. On obtient 1,47 g du produit attendu, sous forme d'un solide beige fondant à 291°C.

L'éthoxycarbonyl-3 difluoro-7,8 (fluoro-2 éthyl)-1 oxo-4 dihydro-1,4 benzo [b] naphtyridine-1,8 a été préparée de la manière suivante:

A un mélange, sous agitation, de 1,46 g de chlorhydrate de fluoro-2 éthylamine et de 2,06 $cm^3$ de triéthylamine dans 30 $cm^3$ de trichlorométhane sont ajoutés à environ 10°C, 2,58 g de (chloro-2 difluoro-6,7 quinoléinecarbonyle-3)-2 diméthylamino-3 acrylate d'éthyle. Après 16 heures d'agitation à environ 20°C, le mélange est concentré sous pression réduite (20 kPa) à une température voisine de 50°C. Le résidu est mis en solution dans 30 $cm^3$ d'éthanol et additionné de 2,3 $cm^3$ de triéthylamine. Le mélange est chauffé sous agitation à environ 75°C. L'insoluble est essoré, lavé par 3 fois 5 $cm^3$ d'éthanol; on obtient 2,3 g du produit attendu, sous forme d'un solide beige fondant à 266°C qui a été utilisé sans autre purification pour les étapes ultérieures.

Le (chloro-2 difluoro-6,7 quinoléinecarbonyle-3)-2 diméthylamino-3 acrylate d'éthyle peut être obtenu comme décrit ci-après à l'exemple 39.

EXEMPLE 4

Une suspension de 1,6 g d'acide chloro-8 fluoro-7 méthyl-1 oxo-4 dihydro-1,4 benzo [b] naphtyridine-1,8

carboxylique-3 et de 3,7 g de (fluoro-4 phényl)-2 pipérazine-(RS) dans 16 $cm^3$ de pyridine est chauffée à une température voisine de 115°C pendant 24 heures. Le mélange réactionnel est concentré sous pression réduite (20 kPa) à environ 60 °C. Le résidu est repris par 40 $cm^3$ d'éthanol et concentré de nouveau sous pression réduite dans les conditions précédentes. Le solide obtenu est repris par 10 $cm^3$ d'eau, additionné de 1,75 $cm^3$ d'acide acétique à 10 %, essoré, lavé par 2 fois 10 $cm^3$ d'eau et 2 fois 10 $cm^3$ d'éthanol. Après 2 recristallisations dans 10 $cm^3$ de diméthylformamide à chaque fois, on obtient 1,1 g d'acide fluoro-7 [(fluoro-4 phényl)-3 pipérazinyl-1]-8 méthyl-1 oxo-4 dihydro-1,4 benzo [b] naphtyridine-1,8 carboxylique-3-(RS) sous forme d'un solide jaune fondant à 270-275°C.

La (fluoro-4 phényl)-2 pipérazine-(RS) a été préparée selon le procédé décrit pour la phényl-2 pipérazine-(RS) par R. Roderick et coll., J. Med. Chem., 9, 181 (1966).

A partir de 20 g de (fluoro-4 phényl)-2 oxo-3 pipérazine-(RS) et de 7,8 g d'hydrure d'aluminium lithium dans 1,5 litre d'éther éthylique. On obtient 11 g de (fluoro-4 phényl)-2 pipérazine-(RS) sous forme d'un solide beige fondant à 110-112°C.

A partir de 65 g de bromo-1 (fluoro-4' phényl)-1 acétate d'éthyle et de 30 g d'éthylène diamine, on obtient 30 g de (fluoro-4 phényl)-2 oxo-3 pipérazine-(RS) sous forme d'un solide incolore fondant à 115°C.

Le bromo-1 (fluoro-4' phényl)-1 acétate d'éthyle a été préparé selon J.W. Epstein et coll., J. Med. Chem., 24, 481 (1981) à partir du (fluoro-4 phényl) acétate d'éthyle.

Le (fluoro-4 phényl) acétate d'éthyle a été préparé selon la méthode décrite par J.W. Corse et coll., J. Am. Chem. Soc., 70, 2837 (1948).

## EXEMPLE 5

Une solution de 2,1 g d'acide fluoro-7 [(fluoro-4 phényl)-3 pipérazinyl-1]-8 méthyl-1 oxo-4 dihydro-1,4 benzo [b] naphtyridine-1,8 carboxylique-3-(RS) dans 1,8 $cm^3$ d'acide formique à 98 % et 4,4 $cm^3$ de formaldéhyde en solution aqueuse à 30 % est chauffée à une température voisine de 100°C pendant 2 heures. Le mélange réactionnel est concentré sous pression réduite (20 kPa) à environ 50°C, additionné de 10 $cm^3$ d'eau, de 1,2 $cm^3$ de potasse aqueuse 2N et chauffé à environ 100°C pendant 2 minutes. Après refroidissement à une température voisine de 20°C, l'insoluble est essoré et lavé par 2 fois 20 $cm^3$ d'eau. Après 2 recristallisations dans 15 $cm^3$ de diméthylformamide à chaque fois, on obtient 1,3 g d'acide fluoro-7 [(fluoro-4 phényl)-3 méthyl-4 pipérazinyl-1]-8 methyl-1 oxo-4 dihydro-1,4 benzo [b] naphtyridine-1,8 carboxylique-3-(RS) sous forme d'un solide jaune se décomposant à 313-314°C.

## EXEMPLE 6

En opérant dans les conditions de l'exemple 16, mais à partir de 1,3 g d'acide difluoro-7,8 méthyl-1 oxo-4 dihydro-1,4 benzo [b] naphtyridine-1,8 carboxylique-3 et de 1,8 g de (fluoro-3 phényl)-2 pipérazine-(RS). Après 1 recristallisation dans 50 $cm^3$ de diméthylformamide à 50 % d'éthanol, on obtient 1,74 g d'acide fluoro-7 [(fluoro-3 phényl)-3 pipérazinyl-1]-8 méthyl-1 oxo-4 dihydro-1,4 benzo [b] naphtyridine-1,8 carboxylique-3-(RS), sous forme d'un solide jaune fondant à 254°C.

## EXEMPLE 7

On opère dans les conditions de l'exemple 4, mais à partir de 1,5 g d'acide chloro-8 éthyl-1 fluoro-7 oxo-4 dihydro-1,4 benzo [b] naphtyridine-1,8 carboxylique-3 et de 3,4 g de (fluoro-4 phényl)-2 pipérazine-(RS) dans 15 $cm^3$ de pyridine. On obtient 0,92 g d'acide éthyl-1 fluoro-7 oxo-4 [(fluoro-4 phényl)-3 pipérazinyl-1]-8 dihydro-1,4 benzo [b] naphtyridine-1,8 carboxylique-3-(RS), sous forme d'un solide jaune fondant à 298°C.

L'acide chloro-8 éthyl-1 fluoro-7 oxo-4 dihydro-1,4 benzo[b] naphtyridine-1,8 carboxylique-3 a été préparé dans les conditions de l'exemple 1, mais à partir de 10,5 g de chloro-8 fluoro-7 éthoxycarbonyl-3 éthyl-1 oxo-4 dihydro-1,4 benzo [b] naphtyridine-1,8. On obtient 9,3 g d'acide chloro-8 éthyl-1 fluoro-7 oxo-4 dihydro-1,4 benzo [b] naphtyridine-1,8 carboxylique-3 sous forme d'un solide beige fondant à 380°C qui est utilisé sans autre purification pour les étapes ultérieures.

La chloro-8 éthoxycarbonyl-3 éthyl-1 fluoro-7 oxo-4 dihydro-1,4 benzo [b] naphtyridine-1,8 a été préparée de la manière suivante :

Dans une suspension sous agitation, de 13,5 g de (dichloro-2,7 fluoro-6 quinoléinecarbonyle-3)-2 diméthylamino-3 acrylate d'éthyle dans 135 $cm^3$ d'éthanol, on ajoute, en 5 minutes, entre 10 et 15°C, 16 g d'éthylamine, laisse la température remonter à environ 20°C, ajoute 0,5 g de DBU et chauffe sous agitation, pendant 2 heures, à une température voisine de 75°C. Après refroidissement à une température voisine de 20°C, le précipité est essoré, lavé par 2 fois 100 $cm^3$ d'éthanol, 2 fois 100 $cm^3$ d'éther éthylique. On obtient 10,4 g de

chloro-8 éthoxycarbonyl-3 éthyl-1 fluoro-7 oxo-4 dihydro-1,4 benzo [b] naphtyridine-1,8 sous forme d'un solide jaune fondant à 300-301°C, qui est utilisé sans autre purification pour les étapes ultérieures.

EXEMPLE 8

En opérant dans les conditions de l'exemple 16, mais à partir de 1,5 g d'acide éthyl-1 difluoro-7,8 oxo-4 dihydro-1,4 benzo [b] naphtyridine-1,8 carboxylique-3 et de 1,98 g de (fluoro-3 phényl)-2 pipérazine-(RS), on obtient 2 g d'acide éthyl-1 fluoro-7 [(fluoro-3 phényl)-3 pipérazinyl-1]-8 oxo-4 dihydro-1,4 benzo [b] naphtyridine-1,8 carboxylique-3-(RS) sous forme d'un solide jaune fondant à 284°C.

L'acide éthyl-1 difluoro-7,8 oxo-4 dihydro-1,4 benzo [b] naphtyridine-1,8 carboxylique-3 a été préparé dans les mêmes conditions que dans l'exemple 17, mais à partir de 8 g d'éthoxycarbonyl-3 éthyl-1 difluoro-7,8 oxo-4 dihydro-1,4 benzo [b] naphtyridine-1,8. On obtient 6,70 g de produit attendu, sous forme d'un solide jaune se décomposant à 330°C.

L'éthoxycarbonyl-3 éthyl-1 difluoro-7,8 oxo-4 dihydro-1,4 benzo [b]naphtyridine-1,8 peut être obtenue comme décrit ci-après à l'exemple 32.

La (fluoro-3 phényl)-2 pipérazine-(RS) a été préparée selon la méthode décrite dans la demande de brevet français 2 351 108. A partir de 24 g de fluoro-3 phénylglyoxal, on obtient 6,3 g du produit attendu sous forme d'une huile jaune.

Le fluoro-3 phénylglyoxal a été préparé selon la méthode décrite par Nathan Kornblum et Coll., J.Am. Chem.Soc.,79, 6562 (1957). A partir de 40 g de fluoro-3' bromo-2 acétophénone, on obtient 24 g du produit attendu sous forme d'une huile jaune.

La fluoro-3' bromo-2 acétophénone a été préparée selon la méthode décrite par D.V.C. Awang et Coll. Canad.J.Chem. 47, 706, (1969). A partir de 25,8 g de fluoro-3' acétophénone, on obtient 40 g du produit attendu, sous forme d'une huile verdâtre.

EXEMPLE 9

En opérant dans les conditions de l'exemple 16, mais à partir de 1,5 g d'acide éthyl-1 difluoro-7,8 oxo-4 dihydro-1,4 benzo [b] naphtyridine-1,8 carboxylique-3 et de 1,5 g de (fluoro-2 phényl)-2 pipérazine-(RS), on obtient 2 g d'acide éthyl-1 fluoro-7 [(fluoro-2 phényl)-3 pipérazinyl-1]-8 oxo-4 dihydro-1,4 benzo [b] naphtyridine-1,8 carboxylique-3-(RS) sous forme d'un solide jaune fondant à 306°C.

La (fluoro-2 phényl)-2 pipérazine-(RS) a été préparée selon les mêmes méthodes que celles utilisées dans l'exemple 8.

A partir de 26,8 g de fluoro-2 phénylglyoxal, on obtient 6 g de (fluoro-2 phényl)-2 pipérazine-(RS) fondant à 70°C.

A partir de 40,3 g de fluoro-2' bromo-2 acétophénone, on obtient 26,8 g de fluoro-2 phénylglyoxal utilisé sans autre purification pour les étapes ultérieures.

A partir de 20 g de fluoro-2' acétophénone, on obtient 32,6 g de fluoro-2' bromo-2 acétophénone sous forme d'une huile verdâtre utilisée sans autre purification pour les étapes ultérieures.

EXEMPLE 10

L'acide cyclopropyl-1 fluoro-7 [(fluoro-4 phényl)-3 pipérazinyl-1]-8 oxo-4 dihydro-1,4 benzo [b] naphtyridine-1,8 carboxylique-3-(RS) a été préparé dans les conditions de l'exemple 11 ci-après, mais à partir de 2 g d'acide chloro-8 cyclopropyl-1 fluoro-7 oxo-4 dihydro-1,4 benzo [b] naphtyridine-1,8 carboxylique-3 et de 6,534 g de (fluoro-4 phényl)-2 pipérazine (RS) et de 9 $cm^3$ de triéthylamine. Le mélange réactionnel est concentré sous pression réduite (20 kPa) à une température voisine de 60°C. L'extrait sec est repris par 20 $cm^3$ d'eau et 0,5 $cm^3$ d'acide acétique. L'insoluble est essoré et lavé par 2 fois 5 $cm^3$ d'eau. Après 1 recristallisation dans un mélange de 37 $cm^3$ de diméthylformamide et de 37 $cm^3$ d'éthanol, on obtient 1,07 g d'acide cyclopropyl-1 fluoro-7 [(fluoro-4 phényl)-3 pipérazinyl-1]-8 oxo-4 dihydro-1,4 benzo [b] naphtyridine-1,8 carboxylique-3-(RS) sous forme d'un solide jaune fondant à 306°C.

EXEMPLE 11

Une suspension de 1,84 g d'acide chloro-8 fluoro-7 méthyl-1 oxo-4 dihydro-1,4 benzo [b] naphtyridine-1,8 carboxylique-3 dans 20 $cm^3$ de pyridine, 5,83 g de (méthyl-4 phényl)-2 pipérazine-(RS) et 1,24 g de triéthylamine est chauffée à une température voisine de 115°C pendant 37 heures. Après refroidissement à environ 20°C, l'insoluble est essoré, lavé par 2 fois 5 $cm^3$ d'éthanol et 2 fois 5 $cm^3$ d'éther éthylique. Après re-

cristallisation dans 25 $cm^3$ de diméthylformamide, on obtient 0,8 g d'acide fluoro-7 méthyl-1 [(méthyl-4 phényl)-3 pipérazinyl-1)-8 oxo-4 dihydro-1,4 benzo [b] naphtyridine-1,8 carboxylique-3-(RS) sous forme d'un solide jaune se décomposant à 282°C.

La (méthyl-4 phényl)-2 pipérazine a été préparée selon la méthode décrite dans la demande de brevet FR 2 351 108 : à partir de 38,8 g de méthyl-4 phénylglyoxal (préparé à partir de la méthyl-4 acétophénone) on obtient 11,55 g de méthyl-4 phényl-2 pipérazine (RS) sous forme d'un solide jaune fondant à 96-97°C.

EXEMPLE 12

Une suspension de 1,45 g d'acide difluoro-7,8 méthyl-1 oxo-4 dihydro-1,4 benzo [b] naphtyridine-1,8 carboxylique-3 et de 1,92 g de (méthoxy-4 phényl)-2 pipérazine-(RS) dans 14 $cm^3$ de diméthylsulfoxyde sont chauffés, sous agitation, à une température voisine de 90°C pendant 2 heures. Après refroidissement à environ 20°C, on ajoute au mélange réactionnel 20 $cm^3$ d'eau. L'insoluble est essoré et lavé par 2 fois 5 $cm^3$ d'eau. Après 1 recristallisation dans 150 $cm^3$ de diméthylformamide, on obtient 1,35 g d'acide fluoro-7 [(méthoxy-4 phényl)-3 pipérazinyl-1]-8 méthyl-1 oxo-4 dihydro-1,4 benzo [b] naphtyridine-1,8 carboxylique-3-(RS) sous forme d'un solide jaune se décomposant à 312°C.

L'acide difluoro-7,8 méthyl-1 oxo-4 dihydro-1,4 benzo [b] naphtyridine-1,8 carboxylique-3, est préparé de la manière suivante :

Une suspension de 8 g d'éthoxycarbonyl-3 difluoro-7,8 méthyl-1 oxo-4 dihydro-1,4 benzo [b] naphtyridine-1,8 dans 80 $cm^3$ d'acide chlorhydrique en solution aqueuse à 17,5 % et 80 $cm^3$ d'acide acétique est chauffée, sous agitation, à une température voisine de 100°C pendant 1 heure et demie. Après refroidissement à environ 20°C, le solide est essoré et lavé par 6 fois 100 $cm^3$ d'eau. Après 1 recristallisation dans 160 $cm^3$ de diméthylformamide, on obtient 6,44 g d'acide difluoro-7,8 méthyl-1 oxo-4 dihydro-1,4 benzo [b] naphtyridine-1,8 carboxylique-3, sous forme d'un solide jaune se décomposant à 360°C.

L'éthoxycarbonyl-3 difluoro-7,8 méthyle-1 oxo-4 dihydro-1,4 benzo[b] naphtyridine-1,8 peut être obtenue comme décrit ci-après, à l'exemple 19.

La (méthoxy-4 phényl)-2 pipérazine-(RS) a été préparée selon la méthode décrite dans la demande de brevet FR 2 351 108, à partir de 23,4 g de (méthoxy-4 phényl)-2 glyoxal et de 10,26 g d'éthylènediamine on obtient 6,21 g de (méthoxy-4 phényl)-2 pipérazine-(RS) sous forme d'un produit huileux qui est utilisé tel quel.

Le méthoxy-4 phénylglyoxal peut être préparé selon la méthode décrite par Nathan Kornblum et coll., J. Am. Chem. Soc., 79, 6562 (1957). A partir de 45,4 g de bromo-2 méthoxy-4' acétophénone dans 200 $cm^3$ de diméthylsulfoxyde, on obtient 23,4 g de méthoxy-4 phénylglyoxal sous forme d'une huile orange qui est utilisée sans autre purification pour les étapes ultérieures.

La bromo-2 méthoxy-4' acétophénone a été préparée selon NG.PH. Buu-Hoï et coll., J. Chem. Soc., 255, (1951).

EXEMPLE 13

Une suspension de 2 g d'acide difluoro-7,8 méthoxy-1 oxo-4 dihydro-1,4 benzo [b] naphtyridine-1,8 carboxylique-3 et de 2,1 g de (phényl)-2 pipérazine-(RS) dans 30 $cm^3$ de diméthylsulfoxyde est chauffée, sous agitation pendant 15 minutes, à environ 50°C. Après refroidissement à environ 20°C, le mélange réactionnel est versé dans 100 $cm^3$ d'eau et additionné de 1,2 $cm^3$ d'acide acétique. L'insoluble est essoré, lavé par 3 fois 10 $cm^3$ d'eau et recristallisé dans 80 $cm^3$ de diméthylformamide. On obtient 2 g d'acide fluoro-7 méthoxy-1 oxo-4 [(phényl)-3 pipérazinyl-1]-8 dihydro-1,4 benzo [b] naphtyridine-1,8 carboxylique-3-(RS) sous forme d'un solide jaune se décomposant à 284°C.

L'acide difluoro-7,8 méthoxy-1 oxo-4 dihydro-1,4 benzo[b] naphtyridine-1,8 carboxylique-3 est préparé dans les conditions suivantes :

Une suspension de 2,78 g d'éthoxycarbonyl-3 difluoro-7,8 méthoxy-1 oxo-4 dihydro-1,4 benzo [b] naphtyridine-1,8 dans 30 $cm^3$ d'acide chlorhydrique à 17,5 % et 30 $cm^3$ d'acide acétique est chauffée à une température voisine de 100°C pendant 1 heure. Après refroidissement à environ 20°C, le mélange réactionnel est versé dans 100 $cm^3$ d'eau. Le précipité formé est essoré, lavé par 3 fois 30 $cm^3$ d'eau et 2 fois 5 $cm^3$ d'éthanol. Après 1 recristallisation dans 100 $cm^3$ de diméthylformamide à 20 % d'éthanol, on obtient 2,03 g d'acide difluoro-7,8 méthoxy-1 oxo-4 dihydro-1,4 benzo[b] naphtyridine-1,8 carboxylique-3 sous forme d'un solide jaune fondant à 325-327°C.

L'éthoxycarbonyl-3 difluoro-7,8 méthoxy-1 oxo-4 dihydro-1,4 benzo [b] naphtyridine-1,8 dans les conditions suivantes :

Une suspension de 1,7 g de chlorhydrate de méthoxylamine dans 40 $cm^3$ de trichlorométhane est additionnée de 2,13 g de triéthylamine. Après 15 minutes d'agitation à une température voisine de 20°C, la solution

obtenue est additionnée de 3,69 g (chloro-2 difluoro-6,7 quinoléinecarbonyle-3)-2 diméthylamino-3 acrylate d'éthyle et agitée pendant 4 heures et demie à environ 20°C. Le mélange réactionnel est concentré à sec sous pression réduite (20 kPa) à une température voisine de 50°C. Le résidu est repris par 70 $cm^3$ d'éthanol, 3,6 g de triéthylamine et chauffé pendant 30 minutes à une température voisine de 75°C. Après refroidissement à environ 20°C, le précipité obtenu est essoré et lavé par 3 fois 30 $cm^3$ d'éthanol. On obtient 2,67 g d'éthoxycarbonyl-3 difluoro-7,8 méthoxy-1 oxo-4 dihydro-1,4 benzo [b] naphtyridine-1,8 sous forme d'un solide jaune pâle fondant à 266-268°C.

Le (chloro-2 difluoro-6,7 quinoleinecarbonyle-2)-2 diméthylamino-3 acrylate d'éthyle) peut être obtenu comme décrit ci-après à l'exemple 20.

EXEMPLE 14

L'acide [(cyano-4 phényl)-3 pipérazinyl-1)-8 fluoro-7 méthyl-1 oxo-4 dihydro-1,4 benzo [b] naphtyridine-1,8 carboxylique-3-(RS) a été préparé dans les conditions de l'exemple 11, mais à partir de 1,84 g d'acide chloro-8 fluoro-7 méthyl-1 oxo-4 dihydro-1,4 benzo [b] naphtyridine-1,8 carboxylique-3, de 5,61 g de (cyano-4 phényl)-2 pipérazine (RS) et de 1,7 $cm^3$ de triéthylamine. On obtient 1,15 g d'acide [(cyano-4 phényl)-3 pipérazinyl-1]-8 fluoro-7 méthyl-1 oxo-4 dihydro-1,4 benzo [b] naphtyridine-1,8 carboxylique-3-(RS), sous forme d'un solide jaune se décomposant à 332°C.

La (cyano-4 phényl)-2 pipérazine-(RS) peut être préparée selon la méthode décrite dans la demande de brevet FR 2 351 108, à partir de 45 g de cyano-4 phénylglyoxal, on obtient 9,4 g de (cyano-4 phényl)-2 pipérazine sous forme d'une huile orange qui est utilisée telle quelle pour les étapes ultérieures. Le cyano-4 phényl glyoxal est préparé à partir de la cyano-4 acétophénone.

EXEMPLE 15

En opérant comme dans l'exemple 16, mais à partir de 1,16 g d'acide difluoro-7,8 méthyl-1 oxo-4 dihydro-1,4 benzo [b] naphtyridine-1,8 carboxylique-3 et de 1,82 g de (méthoxyméthyl-4 phényl)-2 pipérazine-(RS), on obtient 1,70 g d'acide fluoro-7 [(méthoxyméthyl-4 phényl)-3 pipérazinyl-1]-8 méthyl-1 oxo-4 dihydro-1,4 benzo [b] naphtyridine-1,8 carboxylique-3-(RS) sous forme d'un solide jaune se décomposant à 284°C.

La (méthoxyméthyl-4 phényl)-2 pipérazine-(RS) a été préparée selon la méthode décrite dans la demande de brevet FR 2 351 108, mais à partir de 33,5 g de méthoxyméthyl-4 phénylglyoxal et de 13,48 g d'éthylènediamine. Le produit brut a été purifié par chromatographie sur 750 g de silice 230-400 mesh en suspension dans un mélange de 70 % de dichlorométhane, 26 % d'éthanol, 4 % de triéthylamine et élution par 1,8 litre du même mélange. Après concentration de l'éluat sous pression réduite (20 kPa) à environ 50°C, on obtient 12,15 g du produit attendu sous forme d'un solide orange fondant à 79°C.

Le méthoxyméthyl-4 phénylglyoxal a été préparé selon la méthode décrite dans la demande de brevet FR 2 351 108, mais à partir de 30,8 g de méthoxyméthyl-4 acétophénone et de 24 g de dioxyde de sélénium. On obtient 30 g du produit attendu, sous forme d'une huile brune qui a été utilisé, sans autre purification, pour les étapes ultérieures.

La méthoxyméthyl-4 acétophénone a été préparée selon la méthode décrite par H.B.HASS et Coll., J.Am. Chem.Soc. 71, 1767, (1949) à partir du méthoxyméthyl-4 cyanobenzène obtenu à partir du bromure de 4-cyanobenzyle.

EXEMPLE 16

Une suspension de 1,015 g d'acide difluoro-7,8 méthyl-1 oxo-4 dihydro-1,4 benzo[b]naphtyridine-1,8 carboxylique-3 et de 1,7 g d'[(hydroxy-2 éthoxy)-4 phényl]-2 pipérazine-(RS) dans 15 $cm^3$ de diméthylsulfoxyde est chauffée, sous agitation, à une température voisine de 100°C, pendant 2 heures et demie. Après refroidissement à environ 20°C, la suspension est additionnée de 30 $cm^3$ d'eau, essorée, lavée par 3 fois 10 $cm^3$ d'eau. Après une recristallisation dans 15 $cm^3$ de diméthylformamide, on obtient 1,2 g d'acide fluoro-7 {[( hydroxy-2 éthoxy)-4 phényl]-3 pipérazinyl-1}-8 méthyl-1 oxo-4 dihydro-1,4 benzo[b]naphtyridine-1,8 carboxylique-3-(RS) sous forme d'un solide jaune fondant à 276°C.

L'(hydroxy-2 éthoxyl-4 phényl]-2 pipérazine-(RS) peut être préparée selon la méthode décrite dans la demande de brevet FR 2 351 108, à partir de 18,5 g d'[(hydroxy-2 éthoxy)-4 phényl]-2 glyoxal et de 6,86 g d'éthylènediamine. On obtient 4 g d'(hydroxy-2 éthoxy)-4 phényl]-2 pipérazine-(RS) sous forme d'un solide beige fondant à 141°C.

L'[(hydroxy-2 éthoxy)-4 phényl]-2 glyoxal peut être préparé selon la méthode décrite par Nathan kornblum et Coll., J. Am. Chem. Soc., 79, 6562 (1957). A partir de 30,5 g d'(hydroxy-2' éthoxy)-4' bromo-2 acétophénone,

on obtient 18,5 g d'[(hydroxy-2 éthoxy)-4 phényl]-2 glyoxal sous forme d'une huile jaune qui est utilisée sans autre purification pour les étapes ultérieures.

L'(hydroxy-2' éthoxy)-4' bromo-2 acétophénone peut être préparée selon la méthode décrite par NG.PH. Buu-Hoï et Coll., J. Chem.Soc., 255 (1951). A partir de 27 g d'(hydroxy-2 éthoxy)-4 acétophénone, on obtient 28,85 g d'(hydroxy-2' éthoxy)-4' bromo-2 acétophénone sous forme d'un solide beige fondant à 78°C.

L'(hydroxy-2 éthoxy)-4 acétophénone a été préparée selon C. Rufer et Coll., J. Med. Chem., 18(3), 253, (1975).

EXEMPLE 17

En opérant dans les conditions de l'exemple 16, mais à partir de 1,45 g d'acide difluoro-7,8 méthyl-1 oxo-4 dihydro-1,4 benzo [b] naphtyridine-1,8 carboxylique-3 et de 2,09 g de (diméthyl-3,4 phényl)-2 pipérazine-(RS), on obtient 2,15 g d'acide fluoro-7 méthyl-1 [(diméthyl-3,4 phényl)-3 pipérazinyl-1]-8 oxo-4 dihydro-1,4 benzo [b] naphtyridine-1,8 carboxylique-3-(RS) sous forme d'un solide jaune fondant à 276°C.

La (diméthyl-3,4 phényl)-2 pipérazine-(RS) a été préparée selon la méthode décrite dans la demande de brevet FR 2 351 108. A partir de 42,4 g de diméthyl-3,4 phénylglyoxal, on obtient 7,9 g de (diméthyl-3,4 phényl)-2 pipérazine (RS) sous forme d'un solide beige fondant à 98-100°C.

Le diméthyl-3,4 phénylglyoxal peut être préparé selon la méthode décrite par Nathan Kornblum et Coll., J.Am.Chem.Soc., 79, 6562 (1957). A partir de 60 g de diméthyl-3',4' bromo-2 acétophénone, on obtient 40,4 g de diméthyl-3,4 phényl glyoxal sous forme d'huile.

La diméthyl-3',4' bromo-2 acétophénone a été préparée selon la méthode décrite par R.M. Laird et R.E. Parker. J.Am.Chem.Soc. 83, 4277, (1961).

EXEMPLE 18

En opérant dans les conditions de l'exemple 16, mais à partir de 1,16 g d'acide difluoro-7,8 méthyl-1 oxo-4 dihydro-1,4 benzo [b] naphtyridine-1,8 carboxylique-3 et de 1,82 g d'(amino-4 méthoxy-3 phényl)-2 pipérazine-(RS), on obtient 1,40 g d'acide [(amino-4 méthoxy-3 phényl)-3 pipérazinyl-1]-8 méthyl-1 oxo-4 dihydro-1,4 benzo [b] naphtyridine-1,8 carboxylique-3-(RS), sous forme d'un solide jaune se décomposant à 259°C.

L'(amino-4 méthoxy-3 phényl)-2 pipérazine-(RS) a été préparée de la manière suivante:

Dans une solution de 18 g de (méthoxy-3 nitro-4 phényl)-2 pipérazine-(RS) dans 150 $cm^3$ d'éthanol chauffée, sous agitation, à environ 75°C, on ajoute, en 10 minutes, une solution de 53 g d'hydrosulfite de sodium dans 200 $cm^3$ d'eau. Le mélange est maintenu, sous agitation, à la même température, pendant 1 heure. Le mélange réactionnel est concentré sous pression réduite (20 kPa) à environ 60°C. Le résidu est repris par 150 $cm^3$ d'une solution aqueuse de soude 3,75 N et 50 g de carbonate de potassium. Le mélange est extrait par 4 fois 100 $cm^3$ de trichlorométhane. Les extraits organiques réunis sont séchés sur sulfate de magnésium, filtrés et concentrés, sous pression réduite (20 kPa) à environ 40°C. On obtient 13 g du produit attendu, sous forme d'une huile brune qui est utilisée sans autre purification pour les étapes ultérieures.

La (méthoxy-3 nitro-4 phényl)-2 pipérazine-(RS) a été préparée selon la méthode décrite dans la demande de brevet FR 2 351 108, mais à partir de 36 g de méthoxy-3 nitro-4 phénylglyoxal et de 12,1 g d'éthylènediamine. Le produit brut est purifié par chromatographie sur 800 g de silice 230-400 mesh en suspension dans un mélange de 70 % de dichlorométhane, 26% d'éthanol, 4 % de triéthylamine et élution par 2 litres du même mélange. Après concentration de l'éluat, sous pression réduite (20 kPa) à environ 50°C, on obtient 18 g du produit attendu, sous forme d'une huile rouge qui a été utilisée sans autre purification pour les étapes ultérieures.

Le méthoxy-3 nitro-4 phénylglyoxal a été préparé selon la méthode décrite dans la demande de brevet FR 2 351 108, mais à partir de 33,6 g de méthoxy-3 nitro-4 acétophénone et de 22 g de dioxyde de sélénium. On obtient 35 g du produit attendu sous forme d'une huile brune qui a été utilisée sans autre purification pour les étapes ultérieures.

La méthoxy-3 nitro-4 acétophénone a été préparée selon la méthode décrite dans le brevet S. Africain 67 06 465.

EXEMPLE 19

Une solution de 1,07 g d'éthoxycarbonyl-3 fluoro-7 méthyl-1 oxo-4 (phényl-3 pipérazinyl-1)-8 dihydro-1,4 benzo[b] naphtyridine-1,8-(RS) dans 10 $cm^3$ d'acide acétique et 10 $cm^3$ d'acide chlorhydrique en solution aqueuse à 17,5 % est chauffée à une température voisine de 100°C pendant 40 minutes. Le mélange réactionnel est concentré à sec sous pression réduite (20 kPa) à environ 60°C. L'extrait sec est repris par 10 $cm^3$

d'éthanol, essoré, lavé par 2 fois 10 $cm^3$ d'éthanol et 2 fois 10 $cm^3$ d'éther éthylique. Le solide obtenu est mis en suspension dans 30 $cm^3$ d'eau et chauffé à une température voisine de 95°C. Après addition de 1,35 $cm^3$ de potasse aqueuse 2 N et agitation pendant 30 minutes, le solide est essoré à environ 95°C, lavé par 3 fois 20 $cm^3$ d'eau à environ 80°C, 3 fois 15 $cm^3$ d'éthanol à environ 60°C et 3 fois 20 $cm^3$ d'éther éthylique. Après 1 recristallisation dans un mélange de 12 $cm^3$ de diméthylformamide et 6 $cm^3$ d'éthanol, on obtient 0,7 g d'acide fluoro-7 méthyl-1 oxo-4 (phényl-3 pipérazinyl-1)-8 dihydro-1,4 benzo [b] naphtyridine-1,8 carboxylique-3-(R,S) sous forme d'un solide jaune fondant à 265°C.

L'éthoxycarbonyl-3 fluoro-7 méthyl-1 oxo-4 (phényl-3 pipérazinyl-1)-8 dihydro-1,4 benzo [b] naphtyridine-1,8 (RS) peut être préparé de la manière suivante :

Une suspension de 0,8 g d'éthoxycarbonyl-3 difluoro-7,8 méthyl-1 oxo-4 dihydro-1,4 benzo [b] naphtyridine-1,8, de 0,265 g de carbonate de sodium et de 0,5 g de phényl-2 pipérazine (RS) dans 25 $cm^3$ de diméthylsulfoxyde est chauffée à une température voisine de 95°C pendant 2 heures. Après refroidissement à environ 20°C, le mélange réactionnel est versé dans 75 $cm^3$ d'eau et extrait par 4 fois 50 $cm^3$ de dichlorométhane. Les extraits organiques réunis sont lavés par 3 fois 40 $cm^3$ d'eau, séchés sur sulfate de magnésium, filtrés et concentrés à sec sous pression réduite (20 kPa) à environ 50°C. On obtient 1,07 g d'éthoxycarbonyl-3 fluoro-7 oxo-4 (phényl-3 pipérazinyl-1)-8 dihydro-1,4 benzo [b] naphtyridine-1,8-(RS) sous forme d'un solide jaune fondant à 220-222°C qui est utilisé sans autre purification pour les étapes ultérieures.

L'éthoxycarbonyl-3 difluoro-7,8 méthyle-1 oxo-4 dihydro-1,4 benzo [b] naphtyridine-1,8 est préparée de la manière suivante :

Dans une suspension sous agitation de 22,3 g de (chloro-2 difluoro-6,7 quinoléinecarbonyle-3)-2 diméthylamino-3 acrylate d'éthyle dans 480 $cm^3$ d'éthanol, maintenue à une température voisine de 0°C, on ajoute en 10 minutes entre 0 et 5°C, une solution de 11,3 g à environ 0°C de méthylamine dans 50 $cm^3$ d'éthanol, agite 1 heure entre 0 et 5°C, laisse la température remonter à environ 25°C et agite encore 16 heures à cette même température. L'insoluble est essoré, lavé par 3 fois 100 $cm^3$ d'éthanol et 2 fois 100 $cm^3$ d'éther éthylique. Après 1 recristallisation dans 250 $cm^3$ de diméthylformamide, on obtient 16 g d'éthoxycarbonyl-3 difluoro-7,8 méthyl-1 oxo-4 dihydro-1,4 benzo [b] naphtyridine-1,8 sous forme d'un solide jaune fondant à 323-324°C.

Le (chloro-2 difluoro-6,7 quinoléinecarbonyle-3)-2 diméthylamino-3 acrylate d'éthyle est préparé de la manière suivante :

Une suspension de 6,17 g de (chloro-2 difluoro-6,7 quinolyl-3)-oxo-3 propionate d'éthyle dans 7,15 g de N,N-diméthylformamidediméthylacétal et 60 $cm^3$ d'acétate d'éthyle est chauffée à une température voisine de 75°C pendant 1 heure 15 minutes.Le mélange réactionnel est concentré à sec sous pression réduite (20 kPa) à environ 50°C. Le résidu est repris par 50 $cm^3$ d'éther isopropylique, essoré, lavé par 3 fois 25 $cm^3$ du même solvant. On obtient 6,65 g de (chloro-2 difluoro-6,7 quinoléinecarbonyle-3)-2 diméthylamino-3 acrylate d'éthyle sous forme d'un solide orangé fondant à 140°C.

Le (chloro-2 difluoro-6,7 quinolyl-3)-3 oxo-3 propionate d'éthyle est préparé de la manière suivante :

Une suspension de 14,13 g d'acide chloro-2 difluoro-6,7 quinoléine carboxylique-3 dans 29 $cm^3$ de chlorure de thionyle et 220 $cm^3$ de trichlorométhane est chauffée à une température voisine de 60°C pendant 4 heures. La solution obtenue est concentrée à sec sous pression réduite (20 kPa) à environ 60°C. Le résidu obtenu est repris par 75 $cm^3$ de n-hexane, essoré, lavé par 2 fois 60 $cm^3$ du même solvant. Les 14,4 g de solide jaune obtenu sont mis en solution dans 115 $cm^3$ de tétrahydrofuranne. Cette solution est introduite, goutte à goutte, sous agitation, en 35 minutes, entre 5 et 10°C, dans 70 $cm^3$ d'une solution de chélate de magnésium du monomalonate d'éthyle dans le tétrahydrofuranne préparé dans les conditions décrites ci-après. On laisse la température remonter à environ 20°C, et agite encore 2 heures dans ces conditions. La solution obtenue est introduite, goutte à goutte, sous agitation, en 30 minutes, à une température voisine de 5°C, dans 560 $cm^3$ d'acide sulfurique 0,5 N. On laisse la température de la suspension obtenue remonter à 20°C et agite encore 1 heure et demie à cette température. On extrait par 3 fois 250 $cm^3$ d'acétate d'éthyle. Les extraits organiques réunis sont lavés par 2 fois 250 $cm^3$ d'eau, séchés sur sulfate de magnésium, filtrés, et concentrés à sec, sous pression réduite (20 kPa) à 50°C. Le résidu obtenu est repris par 50 $cm^3$ de n-hexane à 20 % d'éther isopropylique, essoré, lavé par 10 $cm^3$ du même mélange et recristallisé dans 60 $cm^3$ d'isopropanol à 30 % de n-hexane. On obtient 11,84 g de (chloro-2 difluoro-6,7 quinolyl-3)-3 oxo-3 propionate d'éthyle sous forme d'un solide crème fondant à 107°C.

Préparation du chélate de magnésium du monomalonate d'éthyle :

A 2,78 g de magnésium en tournure on ajoute progressivement 2 $cm^3$ d'éthanol absolu, 0,1 $cm^3$ de tétrachlorométhane et 1 g de monomalonate d'éthyle. Après échauffement, on ajoute, en 15 minutes, une solution de 9 g de monomalonate d'éthyle dans 180 $cm^3$ d'éthanol. Le mélange est chauffé 20 heures à une température voisine de 75°C, concentré sous pression réduite (20 kPa) à 50°C. Le résidu est repris par 2 fois 100 $cm^3$ de toluène et concentré sous pression réduite dans les mêmes conditions que précédemment. La poudre grise obtenue est mise en solution par addition de tétrahydrofuranne anhydre de façon à obtenir un volume total de

70 $cm^3$.

L'acide chloro-2 difluoro-6,7 quinoléine carboxylique-3 a été préparé de la manière suivante :

A une suspension, sous agitation, refroidie à 10°C, de 70,18 g de chloro-2 difluoro-6,7 formyl-3 dihydro-1,4 quinoléine dans 970 $cm^3$ de potasse aqueuse N, on ajoute, en 1 heure, en maintenant la température entre 10 et 14°C, une solution de 115 g de permanganate de potassium dans 1,215 litre d'eau. On laisse la température remonter à environ 20°C et agite encore 30 minutes à cette température. On ajoute 38,5 g de dithionite de sodium, agite 10 minutes à une température voisine de 20°C, filtre sur silice diatomée, lave par 3 fois 200 $cm^3$ d'eau. Le filtrat et les phases aqueuses de lavage sont réunis et additionnés de 140 $cm^3$ d'acide chlorhydrique en solution aqueuse à 35 %. Le précipité formé est extrait par 4 fois 800 $cm^3$ d'acétate d'éthyle. Les extraits organiques réunis sont lavés par 2 fois 500 $cm^3$ d'eau, séchés sur sulfate de magnésium, filtrés et concentrés sous pression réduite (20 kPa) à 50°C. Le résidu est repris par 400 $cm^3$ d'éther éthylique, essoré, lavé par 2 fois 200 $cm^3$ du même solvant. On obtient 49,2 g d'acide chloro-2 difluoro-6,7 quinoléine carboxylique-3 sous forme d'un solide beige fondant à 232°C qui est utilisé, sans autre purification, pour les étapes ultérieures.

La chloro-2 difluoro-6,7 formyl-3 dihydro-1,4 quinoléine a été préparée de la manière suivante :

A un mélange de 800 $cm^3$ de trichlorométhane et de 74,35 $cm^3$ de diméthylformamide, on ajoute en 30 minutes, sous agitation, entre 10 et 15°C, 76,9 $cm^3$ de chlorure de phosphoryle et agite 1 heure à température voisine de 20°C. A la solution obtenue, on ajoute en 10 minutes à environ 20°C, sous forte agitation, 65,8 g de difluoro-6,7 dihydro-3,4 carbostyrile. La suspension obtenue est chauffée à une température voisine de 60°C et maintenue à cette température pendant 2 heures. Le mélange réactionnel est concentré sous pression réduite (20 kPa) à 50°C jusqu'à obtention d'un mélange pâteux. Sous forte agitation on ajoute un mélange de 500 g de glace et 500 $cm^3$ d'eau. Le solide obtenu est essoré à environ 5°C et lavé par 3 fois 300 $cm^3$ d'eau à 5°C. Le produit humide obtenu et 60 g d'acétate de sodium sont ajoutés simultanément, en 1 heure, à 1,5 litre d'eau à 90°C, de manière à maintenir le pH à environ 6. On agite encore 30 minutes à 90°C, laisse la température redescendre à environ 50°C, essore à cette température et lave par 3 fois 300 $cm^3$ d'eau à environ 20°C. On obtient 70,18 g de chloro-2 difluoro-6,7 formyl-3 dihydro-1,4 quinoléine sous forme d'un solide jaune fondant à 260°C qui est utilisé tel quel pour les étapes ultérieures.

Le difluoro-6,7 dihydro-3,4 carbostyrile est obtenu de la manière suivante :

A 67 g de difluoro-3',4' N-chloro-3 propionanilide, on ajoute sous forte agitation, 134 g de chlorure d'aluminium, puis après environ 2 minutes, on ajoute, de nouveau, par petites fractions, en 15 minutes, 135,9 g de difluoro-3',4' N-chloro-3 propionanilide et 272 g de chlorure d'aluminium. La température monte d'ellemême à environ 60°C et le mélange réactionnel devient liquide. On chauffe alors à 110°C en 20 minutes et maintient entre 110 et 120°C pendant 2 heures. Le mélange réactionnel (à environ 110°C) est versé, en 10 minutes, sous forte agitation dans un mélange de 840 $cm^3$ d'acide chlorhydrique à 35 % et de 1 kg de glace pilée. On laisse la température remonter à environ 20°C, essore, lave par 600 $cm^3$ d'eau 2 fois, 300 $cm^3$ d'éthanol à 5°C et 2 fois 400 $cm^3$ d'éther éthylique à environ 20°C. On obtient 131,58 g de difluoro-6,7 dihydro-1,4 carbostyrile sous forme d'un solide beige fondant à 216°C qui est utilisé tel quel pour les étapes ultérieures.

Le difluoro-3',4' N-chloro-3 propionanilide est préparé de la manière suivante :

A une solution de 125 g de 3,4 difluoroaniline dans 80 $cm^3$ de pyridine et 1,5 litre d'acétone chauffée à une température voisine de 55°C, on ajoute, sous agitation, en 1 heure et demie, 139,16 g de chlorure de l'acide chloro-3 propionique et maintient à cette température pendant 1 heure et demie. Après refroidissement à environ 20°C, la solution est versée, sous agitation, dans un mélange de 1 litre d'eau et 500 g de glace pilée. On laisse la température remonter à environ 20°C et extrait par 3 fois 500 $cm^3$ de dichlorométhane. Les extraits organiques réunis sont lavés par 500 $cm^3$ d'acide chlorhydrique N, 5 fois 500 $cm^3$ d'eau, séchés sur sulfate de magnésium, filtrés et concentrés sous pression réduite (20 kPa) à environ 50°C. Le solide obtenu est repris par 500 $cm^3$ de n-hexane, essoré, lavé par 2 fois 100 $cm^3$ du même solvant. On obtient 202,9 g de difluoro-3',4' N-chloro-3 propionanilide sous forme d'un solide beige fondant à 76°C qui est utilisé sans autre purification pour les étapes ultérieures.

La phényl-2 pipérazine-(RS) a été préparée selon le procédé décrit par W.R. Roderick et coll., J. Med. Chem., 9, 181 (1966).

EXEMPLE 20

En opérant dans les conditions de l'exemple 25 mais à partir de 0,6 g d'éthoxycarbonyl-3 fluoro-7 méthyl-1 oxo-4 (phényl-3 pipérazinyl-1)-8 dihydro-1,4 benzo [b] naphtyridine-1,8-(S), on obtient après recristallisation dans 13 $cm^3$ de diméthylformamide à 30 % d'éthanol, 0,44 g d'acide fluoro-7 méthyl-1 oxo-4 (phényl-3 pipérazinyl-1)-8 dihydro-1,4 benzo [b] naphtyridine-1,8 carboxylique-3-(S) sous forme d'un solide jaune se décomposant à 276-278°C.

$[\alpha]_D^{20}$ = -39,3 + 0,8 (c=1; acide acétique)

En opérant dans les conditions de l'exemple 25, mais à partir de 0,8 g d'éthoxycarbonyl-3 difluoro-7,8 méthyl-1 oxo-4 dihydro-1,4 benzo [b] naphtyridine-1,8 et de 0,5 g de phényl-2 pipérazine-(S),on obtient 0,85 g d'éthoxycarbonyl-3 fluoro-7 méthyl-1 oxo-4 (phényl-3 pipérazinyl-1)-8 dihydro-1,4 benzo[b] naphtyridine-1,8-(S), sous forme d'un solide jaune fondant à 226°C.

$[\alpha]_D^{20}$ = -45° ± 1 (c=0,5; acide acétique).

La synthèse de la phényl-2 pipérazine-(S) a été effectuée selon le même procédé que celui décrit dans l'exemple 22.

A partir de 14,9 g phényl-2 di(toluène p-sulfonyl)-1,4 pipérazine-(S), on obtient 3,31 g de phényl-2 pipérazine-(S) sous forme d'un solide incolore fondant à 117°C.

$[\alpha]_D^{20}$ = +39°± 0,6 (c=2; éthanol).

A partir de 16,3 g de phényl-2 di(toluène p-sulfonyl)-1,4 éthylène diamine-(S), on obtient 15,4 g de phényl-2 di(toluène p-sulfonyl)-1,4 pipérazine-(S) sous forme d'un solide incolore fondant à 258°C.

$[\alpha]_D^{20}$ = -9,9°± 0,9 (c=0,5; diméthylformamide).

A partir de 17,5 g de phényl-2 éthylènediamine-(S), on obtient 22,5 g de phényl-2 di(toluène p-sulfonyl)-1,4 éthylène diamine-(S) sous forme d'un solide jaune fondant à 164°C.

$[\alpha]_D^{20}$ = +17,2 ± 0,7 (c=0,7; diméthylformamide)

A partir de 20 g d'α-aminophénylacétamide-(S), on obtient 17,5 g de phényl-2 éthylènediamine-(S) sous forme d'une huile jaune qui est utilisée immédiatement pour l'étape ultérieure.

A partir de 45,1 g de phényl-2 glycinate de méthyle-(S), on obtient 21,5 g d'α-aminophénylacétamide-(S) sous forme d'un solide incolore fondant à 137-138°C.

$[\alpha]_D^{20}$ = +114° ± 2 (c=1;éthanol).

A partir de 45,3 g de phényl-2 glycine-(S), on obtient 45,1 g de phényl-2 glycinate de méthyle-(S)sous forme d'une huile jaune.

## EXEMPLE 21

En opérant dans les conditions de l'exemple 20, mais à partir de 66,3 g d'éthoxycarbonyl-3 fluoro-7 méthyl-1 oxo-4 (phényl-3 pipérazinyl-1)-8 dihydro-1,4 benzo [b] naphtyridine-1,8-(R), on obtient sans recristallisation 59,15 g d'acide fluoro-7 méthyl-1 oxo-4 (phényl-3 pipérazinyl-1)-8 dihydro-1,4 benzo [b] naphtyridine-1,8 carboxylique-3-(R) sous forme d'un solide jaune se décomposant à 276-278°C.

$[\alpha]_D^{20}$ = +39,8 ±0,6 (c=1;acide acétique).

En opérant dans les conditions de l'exemple 20, mais à partir de 51,6 g d'éthoxycarbonyl-3 difluoro-7,8 méthyl-1 oxo-4 dihydro-1,4 benzo [b] naphtyridine-1,8 et de 31,7 g de phényl-2 pipérazine-(R),on obtient 66,86 g d'éthoxycarbonyl-3 fluoro-7 méthyl-1 oxo-4 (phényl-3 pipérazinyl-1)-8 dihydro-1,4 benzo [b] naphtyridine.1,8-(R), sous forme d'un solide jaune fondant à 221°C.

$[\alpha]_D^{20}$ = +43° ± 1 (c=0,5; acide acétique).

La phényl-2 pipérazine-(R) peut être préparée de la manière suivante:

Un mélange de 10 g phényl-2 di(toluène p-sulfonyl)-1,4 pipérazine-(R) et de 9,4 g de phénol dans 100 $cm^3$ d'acide bromhydrique à 48 % est chauffé sous très forte agitation à une température voisine de 120°C, pendant 5 heures. Après refroidissement à environ 80°C, le mélange réactionnel est additionné de 250 $cm^3$ d'eau, refroidi vers 20°C et lavé par 5 fois 100 $cm^3$ de dichlorométhane. La phase aqueuse est concentrée, sous pression réduite (20 kPa) à environ 80°C. Le résidu est repris par 100 cm 3 d'acétate d'éthyle, refroidi à 5°C et additionné entre 5 et 20°C de 100 $cm^3$ de soude aqueuse à 30 % . La phase organique est séparée et la phase aqueuse extraite de nouveau par 3 fois 100 $cm^3$ d'acétate d'éthyle. Les extraits organiques réunis sont lavés par 4 fois 20 $cm^3$ de soude aqueuse 5 N, séchés sur sulfate de magnésium, filtrés et concentrés à sec, sous pression réduite (20 kPa) à environ 30°C. Le résidu obtenu est repris par 15 $cm^3$ d'éther isopropylique à environ 0°C, essoré et lavé à cette même température par 10 $cm^3$ du même solvant. On obtient 2,3 g de phényl-2 pipérazine-(R) sous forme d'un solide incolore fondant à 117°C.

$[\alpha]_D^{20}$ = 38° ± 0,6 (c=2;éthanol).

La phényl-2 di(toluène p-sulfonyl)-1,4 pipérazine-(R) a été préparée de la manière suivante:

Un mélange de 11 g de phényl-2 di(toluène p-sulfonyl)-1,4 éthylènediamine-(R) et de 13,82 g de carbonate de potassium dans 110 $cm^3$ de diméthylformamide est chauffé, sous agitation à 60°C, pendant 30 minutes, puis additionné de 18,8 g de dibromo-1,2 éthane et chauffé à 115°C pendant 1 heure. Après refroidissement à environ 20°C, le mélange réactionnel est versé sur 250 $cm^3$ d'eau et extrait par 3 fois 200 $cm^3$ de dichlorométhane. Les extraits organiques réunis sont lavés par 3 fois 120 $cm^3$ d'eau, séchés sur sulfate de magnésium

et concentrés sous pression réduite (20 kPa) à environ 30°C. Le résidu est repris par 50 $cm^3$ d'éther éthylique, essoré, lavé par 3 fois 15 $cm^3$ du même solvant. On obtient 10,55 g du produit attendu, sous forme d'un solide incolore fondant à 258°C.

$[\alpha]_D^{20}$ = + 9,6° ± 0,8 (c=0,5; diméthylformamide).

La phényl-2 di(toluène p-sulfonyl)-1,4 éthylènediamine-(R) a été préparée selon la méthode décrite par Douglas G. Neilson et Coll., J.Chem.Soc.,393 (1966), mais à partir de 7,5 g de phényl-2 éthylènediamine-(R). On obtient 12,9 g du produit attendu, sous forme d'un solide jaune fondant à 164°C.

$[\alpha]_D^{20}$ = - 18,2 ± 0,7 (c=0,7 diméthylformamide)

La phényl-2 éthylènediamine-(R) a été préparée selon la méthode décrite par H.C.Brown et Coll. J.Am. Chem.Soc.86,3566 (1964), mais à partir de 11 g d'α-aminophénylacétamide-(R) et de 293 $cm^3$ d'une solution 1 M de borane dans le tétrahydrofuranne. On obtient 7,5 g de produit attendu sous forme d'une huile jaune instable qui est utilisé immédiatement pour l'étape ultérieure.

L'α-aminophénylacétamide-(R) a été préparé selon la méthode décrite par Douglas G. Neilson et Coll.J.Chem.Soc.,393 (1966), A partir de 27,9 g de phényl-2 glycinate de méthyle-(R), on obtient, après 1 recristallisation dans 135 $cm^3$ d'acétate d'éthyle à 26 % de méthanol, 12,93 g du produit attendu sous forme d'un solide incolore fondant à 137-138°C.

$[\alpha]_D^{20}$ = - 115° ± 3 (c=0,5; éthanol).

Le phényl-2 glycinate de méthyle-(R) a été préparé selon la méthode décrite par Douglas G. Neilson et Coll., J.Chem.Soc.,393 (1966) mais à partir de 43 g de phényl-2 glycine-(R) et de 22,8 $cm^3$ de chlorure de thionyle dans 85 $cm^3$ de méthanol sous forme d'une huile jaune.

Le monométhanesulfonate de l'acide fluoro-7 méthyl-1 oxo-4 (phényl-3 pipérazinyl-1)-8 dihydro-1,4 benzo [b] naphtyridine-1,8 carboxylique-3-(R) a été préparé de la manière suivante:

Une suspension de 3,5 g d'acide fluoro-7 méthyl-1 oxo-4 (phényl-3 pipérazinyl-1)-8 dihydro-1,4 benzo [b] naphtyridine-1,8 carboxylique-3-(R) dans 70 $cm^3$ d'eau est additionnée de 100 $cm^3$ d'une solution aqueuse d'acide méthanesulfonique 0,1N et chauffée à environ 90°C. Après refroidissement à environ 20°C, l'insoluble est essoré et lavé par 3 fois 25 $cm^3$ d'eau. On obtient 3,9 g du sel attendu sous forme d'un solide jaune se décomposant à 335-340°C.

$[\alpha]_D^{20}$ = -19° ± 2 (c=0,2; eau à 50 % d'éthanol).

Le fluoro-7 méthyl-1 oxo-4 (phényl-3 pipérazinyl-1)-8 dihydro-1,4 benzo [b] naphtyridine-1,8 carboxylate-3 de choline-(R) a été préparé de la manière suivante:

A 3,5 g d'acide fluoro-7 méthyl-1 oxo-4 (phényl-3 pipérazinyl-1)-8 dihydro-1,4 benzo [b] naphtyridine-1,8 carboxylique-3-(R) en suspension dans 30 $cm^3$ de méthanol, sont ajoutés 2,72 $cm^3$ d'une solution de choline à 45 % dans le méthanol. A la solution obtenue sont additionnés 200$cm^3$ d'éther isopropylique. Le précipité est essoré, lavé par 3 fois 60 $cm^3$ du même solvant, puis par 3 fois 60 $cm^3$ d'acétone. On obtient 3,97 g du sel attendu sous forme d'un solide jaune fondant à 234°C.

$[\alpha]_D^{20}$ = -33,9° ± 0,9 (c=1; méthanol).

Le monoiséthionate de l'acide fluoro-7 méthyl-1 oxo-4 (phényl-3 pipérazinyl-1)-8 dihydro-1,4 benzo [b] naphtyridine-1,8 carboxylique-3-(R) a été préparé de la manière suivante: A une suspension de 1 g de l'acide fluoro-7 méthyl-1 oxo-4 (phényl-3 pipérazinyl-1)-8 dihydro-1,4 benzo [b] naphtyridine-1,8 carboxylique-3-(R) dans 40 $cm^3$ d'eau à 50 % d'éthanol, sont ajoutés 2,6 $cm^3$ d'une solution aqueuse d'acide iséthionique 1N. Après dissolution à environ 80°C, puis refroidissement à environ 10°C, l'insoluble est essoré, lavé par 2 fois 15 $cm^3$ d'eau à 50 % d'éthanol, 2 fois 15 $cm^3$ d'eau et 3 fois 15 $cm^3$ d'éthanol. On obtient 1 g du sel attendu sous forme d'un solide jaune se décomposant à 334°C.

$[\alpha]_D^{20}$ = +111° ± 6 (c=0,1; diméthylsulfoxyde).

## EXEMPLE 22

L'acide fluoro-7 méthyl-1 oxo-4 (phényl-3 pipérazinyl-1)-8 dihydro-1,4 benzo [b] naphtyridine-1,8 carboxylique-3-(-) isomère (S) a été préparé de la même manière que l'acide fluoro-7 méthyl-1 oxo-4 (phényl-3 pipérazinyl-1)-8 dihydro-1,4 benzo [b] naphtyridine-1,8 carboxylique-3-(RS), (exemple 19), mais à partir de 4,4 g d'éthoxycarbonyl-3 difluoro-7,8 méthyl-1 oxo-4 dihydro-1,4 benzo [b] naphtyridine-1,8, de 2,7 g de phényl-2 pipérazine-(+) ; $[\alpha]_D^{20}$ = +35,4°±0,5 (c=2;éthanol) et de 1,48 g de carbonate de sodium. On obtient 6,3 g d'un solide jaune fondant à 226°C.

Après hydrolyse acide dans les mêmes conditions que celles décrites pour l'éthoxycarbonyl-3 fluoro-7 méthyl-1 oxo-4 (phényl-3 pipérazinyl-1)-8 dihydro-1,4 benzo [b] naphtyridine-1,8-(R,S), on obtient 3,95 g d'acide fluoro-7 méthyl-1 oxo-4 (phényl-3 pipérazinyl-11-8 dihydro-1,4 benzo [b] naphtyridine-1,8 carboxylique-3-(-)

isomère (S) sous forme d'un solide jaune fondant à 276-278°C.

$[\alpha]_D^{20}$=-40,4°±1 (c=1;acide acétique).

Les phényl-2 pipérazine-(-) isomère (R) et phényl-2 pipérazine-(+) isomère (S) ont été préparées par hydrolyse acide de 2 composés diastéréoisomériques (R et S) correspondant à la structure d'une (trifluorométhyl-2' méthoxy-2' phénylacétyl)-1 phényl-3 pipérazine appelés arbitrairement A et B. Ces 2 derniers composés sont obtenus par acylation de la phényl-2 pipérazine-(RS) par un chlorure d'acide chiral, (chlorure de l'acide trifluorométhyl-2 méthoxy-2 phénylacétique-(R)-(-). La préparation est la suivante :

A une solution de 12,77 g de phényl-2 pipérazine-(RS) dans 220 $cm^3$ de trichlorométhane refroidie à environ -25°C, on ajoute, goutte à goutte, en 25 minutes, sous agitation, une solution de 19,89 g du chlorure de l'acide trifluorométhyl-2 méthoxy-2 phénylacétique-(R)-(-) dans 80 $cm^3$ du même solvant en maintenant la température entre -30 et -25°C. Après 15 minutes d'agitation dans ces conditions, on laisse la température remonter à environ 0°C, ajoute, entre 0 et 5°C, 50 $cm^3$ de soude aqueuse 2N, laisse la température remonter à environ 20°C et ajoute 150 $cm^3$ d'eau. La phase organique est séparée et la phase aqueuse extraite de nouveau par 2 fois 200 $cm^3$ de trichlorométhane. Les extraits organiques réunis sont lavés par 1 fois 200 $cm^3$ de soude 0,5 N, 4 fois 200 $cm^3$ d'eau, séchés sur sulfate de magnésium, filtrés et concentrés à sec sous pression réduite (20 kPa) à environ 40°C. Le résidu (29,8 g) contenant les 2 diastéréoisomères A et B est mis en solution dans 500 $cm^3$ de dichlorométhane. Ce mélange est séparé par chromatographie liquide haute performance, sur 2 colonnes de 57 mm de diamètre et de 300 mm de longueur, contenant chacune 300 g de silice 55-105 μ, en 5 injections de 100 $cm^3$. L'élution est effectuée par un mélange de dichlorométhane à 2% d'éthanol. Le diastéréoisomère A est élué par 1 litre de ce mélange dans la fraction comprise entre 2,5 et 3,5 litres. Le diastéréoisomère B est élué par 2 litres du même mélange dans la fraction comprise entre 4 et 6 litres. Après concentration sous pression réduite (20 kPa) à environ 50°C, de chacune des 2 fractions, on obtient respectivement 13,62 g de composé A sous forme d'un solide incolore fondant à 102°C et 14 g de composé B, de même aspect fondant à 130°C. Les 13,62 g de composé A sont repris par un mélange de 140 $cm^3$ d'une solution aqueuse à 48% d'acide bromhydrique et de 26 $cm^3$ d'acide acétique et chauffés pendant 30 heures à une température voisine de 110°C. Le mélange réactionnel est concentré à sec sous pression réduite (20 kPa) à environ 80°C. Le résidu obtenu est repris par 200 $cm^3$ d'acétate d'éthyle. La suspension obtenue est refroidie à environ 10°C et additionnée entre 10 et 20°C de 160 $cm^3$ de soude aqueuse à 30%. La phase organique est séparée et la phase aqueuse extraite de nouveau par 3 fois 100 $cm^3$ d'acétate d'éthyle. Les extraits organiques réunis sont lavés par 1 fois 80 $cm^3$ de soude aqueuse 4 N, 3 fois 80 $cm^3$ d'une solution aqueuse à 30% de chlorure de sodium et concentrés à sec sous pression réduite. Après reprise du résidu obtenu par 80 $cm^3$ d'une solution aqueuse à 10% d'acide méthanesulfonique et extraction par 3 fois 100 $cm^3$ d'acétate d'éthyle, la phase aqueuse est additionnée de 120 $cm^3$ de soude aqueuse à 30% et extraite par 4 fois 150 $cm^3$ d'acétate d'éthyle. Les extraits organiques réunis sont lavés par 3 fois 80 $cm^3$ d'une solution aqueuse à 30% de chlorure de sodium, séchés sur sulfate de magnésium, filtrés et concentrés sous pression réduite (20 kPa) à une température voisine de 50°C. Le résidu obtenu est repris par 30 $cm^3$ d'éther isopropylique, essoré et lavé par 10 $cm^3$ du même solvant. On obtient 2,45 g de phényl-2 pipérazine-(+) sous forme d'un solide incolore fondant à 114°C.

$[\alpha]_D^{20}$= + 35,4°±0,5 (c=2; éthanol).

Les 14 g de composé a traités dans les mêmes conditions que celles décrites pour le composé A conduisent à 3,08 g de phényl-2 pipérazine-(-) sous forme d'un solide incolore fondant à 114°C.

$[\alpha]_D^{20}$= -37°±0,5 (c=2; éthanol).

EXEMPLE 23

L'acide fluoro-7 méthyl-1 oxo-4 (phényl-3 pipérazinyl-1)-8 dihydro-1,4 benzo [b] naphtyridine-1,8 carboxylique-3-(+) a été préparé de la même manière que l'acide fluoro-7 méthyl-1 oxo-4 (phényl-3 pipérazinyl-1)-8 dihydro-1,4 benzo [b] naphtyridine-1,8 carboxylique-3-(RS), (exemple 19) à partir de 4,4 g d'éthoxycarbonyl-3 difluoro-7,8 méthyl-1 oxo-4 dihydro-1,4 benzo (b) naphtyridine-1,8, de 2,7 g de phényl-2 pipérazine-(-), $[\alpha]_D^{20}$= -37°±0,5 (c=2;éthanol) et de 1,48 g de carbonate de sodium. On obtient 6,3 g d'un solide jaune fondant à 226°C. Après hydrolyse acide dans les mêmes conditions que celles décrites dans l'exemple 12 pour l'éthoxycarbonyl-3 fluoro-7 méthyl-1 oxo-4 (phényl-3 pipérazinyl-1)-8 dihydro-1,4 benzo [b] naphtyridine-1,8-(RS), on obtient 4,26 g d'acide fluoro-7 méthyl-1 oxo-4 (phényl-3 pipérazinyl-1)-8 dihydro-1,4 benzo [b] naphtyridine-1,8 carboxylique-3-(+) isomère (R) sous forme d'un solide jaune fondant à 276-278°C.

$[\alpha]_D^{20}$= + 40,6°± 1 (c=1; acide acétique).

EXEMPLE 24

Une suspension de 2 g d'éthoxycarbonyl-3 difluoro-7,9 méthyl-1 oxo-4 (phényl-3 pipérazinyl-1)-8 dihydro-1,4 benzo [b] naphtyridine-1,8-(RS) dans 25 $cm^3$ d'éthanol et 15 $cm^3$ de potasse aqueuse N est chauffée sous agitation à une température voisine de 75°C pendant 2 heures. La solution obtenue, à environ 75°C, est additionnée de 9 g d'une solution aqueuse d'acide acétique à 10 %. L'insoluble obtenu est essoré à une température voisine de 75°C et lavé 3 fois par 30 $cm^3$ d'eau à environ 20°C. Après 1 recristallisation dans 40 $cm^3$ de diméthylformamide, on obtient 1,5 g d'acide difluoro-7,9 méthyl-1 oxo-4 (phényl-3 pipérazinyl-1)-8 dihydro-1,4 benzo [b] naphtyridine-1,8 carboxylique-3-(RS) sous forme d'un solide orange fondant à 298°C.

L'éthoxycarbonyl-3 difluoro-7,9 méthyl-1 oxo-4 (phényl-3 pipérazinyl-1)-8 dihydro-1,4 benzo [b] naphtyridine-1,8-(RS) est préparé de la manière suivante :

Une suspension de 1,8 g d'éthoxycarbonyl-3 trifluoro-7,8,9 méthyl-1 oxo-4 dihydro-1,4 benzo [b] naphtyridine-1,8 et de 3,2 g de phényl-2 pipérazine-(RS) dans 30 $cm^3$ de diméthylsulfoxyde est chauffée à une température voisine de 100°C, sous agitation pendant 1 heure et demie. La solution obtenue, à environ 100°C, est versée dans un mélange, sous agitation de 150 $cm^3$ d'eau et 50 g de glace. La suspension obtenue est extraite, à environ 20°C, par 3 fois 40 $cm^3$ de trichlorométhane. Les extraits organiques réunis sont lavés par 2 fois 50 $cm^3$ d'eau, séchés sur sulfate de magnésium, filtrés et concentrés à sec sous pression réduite (20 kPa) à environ 50°C. Le solide obtenu est recristallisé dans un mélange de 40 $cm^3$ de diméthylformamide et de 40 $cm^3$ d'éthanol. On obtient 2 g d'éthoxycarbonyl-3 difluoro-7,9 méthyl-1 oxo-4 (phényl-3 pipérazinyl-1)-8 dihydro-1,4 benzo [b] naphtyridine-1,8-(RS) sous forme d'un solide jaune fondant à 216°C.

L'éthoxycarbonyl-3 trifluoro-7,8,9 méthyl-1 oxo-4 dihydro-1,4 benzo [b] naphtyridine-1,8 est préparé de la manière suivante :

Dans une suspension sous agitation de 19,3 g de (chloro-2 trifluoro-6,7,8 quinoléinecarbonyle-3)-2 diméthylamino-3 acrylate d'éthyle dans 150 $cm^3$ d'éthanol maintenue à une température voisine de 5°C, on ajoute en 10 minutes entre 5 et 10°C, une solution à environ 5°C de 10 g de méthylamine dans 50 $cm^3$ d'éthanol, agite 1 heure entre 5 et 10°C et laisse la température remonter à environ 20°C. La solution obtenue est additionnée de 7,6 g de D.B.U. et chauffée à environ 30°C pendant 1 heure. Après refroidissement à une température voisine de 20°C, le produit est essoré, lavé par 2 fois 100 $cm^3$ d'éthanol et 2 fois 100 $cm^3$ d'éther isopropylique. On obtient 13,4 g d'éthoxycarbonyl-3 trifluoro-7,8,9 méthyl-1 oxo-4 dihydro-1,4 benzo [b] naphtyridine-1,8 sous forme d'un solide jaune fondant à 320°C qui est utilisé sans autre purification pour les étapes ultérieures.

Le (chloro-2 trifluoro-6,7,8 quinoléinecarbonyle-3)-2 diméthylamino-3 acrylate d'éthyle peut être préparé de la manière suivante :

Une suspension de 26,7 g de (chloro-2 trifluoro-6,7,8 quinolyl-3)-3 oxo-3 propionate d'éthyle dans 270 $cm^3$ d'acétate d'éthyle et 32 $cm^3$ de N,N diméthylformamidediméthylacétal est chauffée à une température voisine de 75°C, sous agitation, pendant 2 heures. Le mélange réactionnel est concentré à sec sous pression réduite (20 kPa) à environ 50°C. L'extrait sec est repris par 175 $cm^3$ d'éther isopropylique, essoré, lavé par 2 fois 85 $cm^3$ du même solvant. On obtient 19,32 g de (chloro-2 trifluoro-6,7,8 quinoléinecarbonyle-3)-2 diméthylamino-3 acrylate d'éthyle sous forme d'un solide orange fondant à 118°C qui est utilisé sans autre purification pour les étapes ultérieures.

Le (chloro-2 trifluoro-6,7,8 quinolyl-3)-3 oxo-3 propionate d'éthyle est préparé de la manière suivante :

Une suspension de 46,3 g d'acide chloro-2 trifluoro-6,7,8 quinoléine carboxylique-3 dans 640 $cm^3$ de trichlorométhane et 84 $cm^3$ de chlorure de thionyle est chauffée, sous agitation, à une température voisine de 60°C pendant 6 heures. La solution obtenue est concentrée à sec sous pression réduite (20 kPa) à environ 50°C. L'extrait sec obtenu est repris par 140 $cm^3$ d'éther de pétrole (40-60), essoré, lavé par 2 fois 60 $cm^3$ du même solvant. Les 47,61 g du solide jaune obtenu sont mis en solution dans 400 $cm^3$ de tétrahydrofuranne. Cette solution est introduite, goutte à goutte, sous agitation, en 1 heure et demie, entre 5°C et 10°C, dans 250 $cm^3$ d'une solution de chélate de magnésium du monomalonate d'éthyle dans le tétrahydrofuranne préparé dans les conditions de l'exemple 19. On laisse la température remonter à environ 20°C et agite encore 2 heures dans ces conditions. La solution obtenue, est introduite, goutte à goutte, sous forte agitation, en 1 heure, à une température voisine de 5°C dans 1750 $cm^3$ d'acide sulfurique 0,5 N. On agite encore 2 heures à cette température, extrait à environ 5°C par 3 fois 600 $cm^3$ d'éther éthylique. Les phases organiques réunies sont lavées par 3 fois 500 $cm^3$ d'eau, séchées sur sulfate de magnésium, et concentrées sous pression réduite (20 kPa) à une température voisine de 30°C. L'extrait sec est repris par un mélange de 135 $cm^3$ d'éther isopropylique et de 15 $cm^3$ de n-hexane, essoré à environ 5°C, lavé par 2 fois 115 $cm^3$ du même mélange à la même température. On obtient 47,4 g de (chloro-2 trifluoro-6,7,8 quinolyl-3)-3 oxo-3 propionate d'éthyle sous forme d'un solide beige fondant à 78-80°C qui est utilisé sans autre purification pour les étapes ultérieures.

L'acide chloro-2 trifluoro-6,7,8 quinoléine carboxylique-3 est préparé de la manière suivante :

A une suspension, sous agitation, refroidie à environ 10°C, de 45,7 g de chloro-2 trifluoro-6,7,8 formyl-3 dihydro-1,4 quinoléine dans 585 $cm^3$ de potasse N, on ajoute en 1 heure en maintenant la température entre 10 et 14°C, une solution de 69,65 g de permanganate de potassium dans 730 $cm^3$ d'eau. On laisse encore agiter 30 minutes à environ 10°C. On ajoute 12 g de dithionite de sodium, agite 10 minutes à une température voisine de 10°C, filtre sur silice diatomée et lave par 3 fois 400 $cm^3$ d'eau. Le filtrat et les lavages sont réunis et additionnés de 70 $cm^3$ d'acide chlorhydrique en solution aqueuse à 35 %. Le précipité formé est extrait par 3 fois 500 $cm^3$ d'acétate d'éthyle. Les extraits organiques réunis sont séchés sur sulfate de magnésium, filtrés et concentrés sous pression réduite (20 kPa) à 50°C. Le résidu est repris par un mélange de 100 $cm^3$ d'éther éthylique et 100 $cm^3$ d'éther isopropylique, essoré, lavé par 100 $cm^3$ du même mélange. On obtient 46,43 g d'acide chloro-2 trifluoro-6,7,8 quinoléine carboxylique-3 sous forme d'un solide incolore se décomposant à 225-230°C qui est utilisé sans autre purification pour les étapes ultérieures.

La chloro-2 trifluoro-6,7,8 formyl-3 dihydro-1,4 quinoléine est préparée de la manière suivante :

A un mélange de 525 $cm^3$ de trichlorométhane et de 49 $cm^3$ de diméthylformamide, on ajoute en 40 minutes, sous agitation, entre 5 et 10°C, 50 $cm^3$ de chlorure de phosphoryle, agite 15 minutes à cette température et laisse la température remonter à environ 20°C. A la solution obtenue, on ajoute en 20 minutes à environ 20°C, progressivement, sous forte agitation, 46,8 g de trifluoro-6,7,8 dihydro-3,4 carbostyrile. On laisse agiter 30 minutes à une température voisine de 20°C, chauffe à environ 60°C et maintient à cette même température pendant 2 heures et demie. Le mélange réactionnel est concentré sous pression réduite (20 kPa) à environ 50°C. Le résidu huileux est versé sous forte agitation dans 500 g de glace. On ajoute, par petites fractions, en 30 minutes, 100 g d'acétate de sodium. La suspension obtenue est versée en 15 minutes, sous forte agitation dans 1 litre d'eau préalablement chauffée à environ 90°C et agite encore 15 minutes à cette même température. L'insoluble est essoré à environ 90°C et lavé par 3 fois 250 $cm^3$ d'eau. On obtient 47,7 g de chloro-2 trifluoro-6,7,8 formyl-3 dihydro-1,4 quinoléine sous forme d'un solide incolore se décomposant à 220°C.

Le trifluoro 6,7,8 dihydro-3,4 carbostyrile est préparé de la manière suivante :

24,35 g de trifluoro-6,7,8 carbostyrile en suspension dans un mélange de 450 $cm^3$ d'éthanol et 150 $cm^3$ de diméthylformamide sont hydrogénés, sous agitation, à environ 50°C, en présence de 5 g de nickel de Raney sous une pression de 1 atmosphère, jusqu'à fin d'absorption d'hydrogène. Le nickel de Raney utilisé de qualité W-2 est préalablement lavé par 50 $cm^3$ d'une solution d'acide acétique aqueux à 2 %, par 2 fois 50 $cm^3$ d'eau et par 3 fois 50 $cm^3$ d'éthanol. Le mélange réactionnel est additionné de 250 $cm^3$ de diméthylformamide, filtré à environ 50°C sur silice diatomée. Le filtrat est concentré sous pression réduite (20 kPa) à environ 70°C. L'extrait sec est repris par 150 $cm^3$ d'eau, essoré et lavé par 2 fois 50 $cm^3$ d'eau. On obtient 23,6 g de trifluoro 6,7,8 dihydro-3,4 carbostyrile sous forme d'un solide beige clair fondant à 217°C qui est utilisé sans autre purification pour les étapes ultérieures.

Le trifluoro-6,7,8 carbostyrile est préparé de la manière suivante :

60,83 g de chloro-4 trifluoro-6,7,8 carbostyrile en suspension dans 520 m3 d'acide acétique et 38,15 $cm^3$ de triéthylamine sont hydrogénés sous une pression de 1 atmosphère, en présence de 5,25 g de palladium sur charbon à 10 % jusqu'à fin d'absorption d'hydrogène, à une température voisine de 25°C. Le mélange réactionnel est ensuite chauffé à environ 40°C, filtré à cette même température, sur silice diatomée pour filtration. Le filtrat est concentré sous pression réduite (20 kPa) à une température voisine de 50°C. L'extrait sec est repris par 400 $cm^3$ d'eau ; l'insoluble est essoré, lavé par 4 fois 170 $cm^3$ d'eau, 2 fois 110 $cm^3$ d'éthanol et 2 fois par 100 $cm^3$ d'éther isopropylique. On obtient 48,35 g de trifluoro-6,7,8 carbostyrile, sous forme d'un solide incolore se sublimant à 288°C, qui est utilisé sans autre purification pour les étapes ultérieures.

Le chloro-4 trifluoro-6,7,8 carbostyrile est préparé de la manière suivante :

Une suspension de 70,4 g de chloro-4 éthoxy-2 trifluoro-6,7,8 quinoléine dans 170 $cm^3$ d'une solution aqueuse d'acide chlorhydrique à 35 %, 420 $cm^3$ d'acide acétique et 250 $cm^3$ d'eau est chauffée sous agitation à une température voisine de 100°C pendant 2 heures et demie. Après refroidissement à environ 20°C, le mélange réactionnel est versé dans 1100 $cm^3$ d'eau à environ 5°C, agité 15 minutes à cette même température, puis l'insoluble est essoré et lavé par 3 fois 220 $cm^3$ d'eau. On obtient 61 g de chloro-4 trifluoro-6,7,8 carbostyrile, sous forme d'un solide crème fondant à 213°C qui est utilisé sans autre purification pour les étapes ultérieures.

La chloro-4 éthoxy-2 trifluoro-6,7,8 quinoléine est préparée de la manière suivante :

Une suspension de 69,5 g d'éthoxy-2 trifluoro-6,7,8 hydroxy-4 quinoléine dans 430 $cm^3$ de chlorure de phosphoryle est chauffée, sous agitation à une température voisine de 100°C pendant 30 minutes. La solution obtenue est concentrée, sous pression réduite (20 kPa) à environ 60°C, jusqu'à un volume de 100 $cm^3$. Le résidu est repris par 750 $cm^3$ d'acétate d'éthyle ; la solution obtenue est versée sous agitation, en 10 minutes, dans un mélange de 400 $cm^3$ d'eau et 200 g de glace et laisse agiter dans ces conditions pendant 30 minutes. Après séparation de l'extrait organique, la phase aqueuse est de nouveau extraite par 2 fois 250 $cm^3$ d'acétate d'éthyle. Les extraits organiques réunis sont lavés par 3 fois 250 $cm^3$ d'eau, séchés sur sulfate de magnésium

et concentrés sous pression réduite (20 kPa) à environ 40°C. Le résidu huileux obtenu est repris par 370 $cm^3$ d'éther de pétrole ((40-60). Après filtration sur silice diatomée, le filtrat est concentré à sec, sous pression réduite (20 kPa) à environ 30°C. On obtient 70,7 g de chloro-4 éthoxy-2 trifluoro-6,7,8 quinoléine, sous forme d'un solide beige fondant à 45°C qui est utilisé sans autre purification pour les étapes ultérieures.

L'éthoxy-2 trifluoro-6,7,8 hydroxy-4 quinoléine peut être préparée de la manière suivante :

Une solution de 122 g de trifluoro-2,3,4 N-[(éthoxy-1' éthoxycarbonyl-2') éthylidène]-aniline dans 120 $cm^3$ d'oxyde de phényle est introduite, goutte à goutte, en 25 minutes, sous agitation, dans 600 $cm^3$ d'oxyde de phényle à une température voisine de 250°C tout en éliminant l'éthanol formé par distillation. Après agitation pendant 15 minutes à cette même température, la solution est refroidie à environ 20°C et additionnée de 750 $cm^3$ de n-hexane. Le précipité formé est essoré et lavé 3 fois par 200 $cm^3$ de n-hexane. On obtient 69,5 g d'éthoxy-2 trifluoro-6,7,8 hydroxy-4 quinoléine sous forme d'un solide beige fondant à 171°C qui est utilisé sans autre purification pour les étapes ultérieures.

La trifluoro-2,3,4 N-[(éthoxy-1' éthoxycarbonyl-2') éthylidène]-aniline peut être préparée de la manière suivante :

A une solution de 90 g de chlorhydrate d'(éthoxycarbonyl-2 éthoxy-1) éthylidèneamine dans 820 $cm^3$ d'éthanol, on ajoute en une seule fois, sous agitation, 58,8 g de trifluoro-2,3,4 aniline. Après 48 heures d'agitation à une température voisine de 20°C, la suspension obtenue est filtrée ; le filtrat est concentré sous pression réduite (20 kPa) à une température voisine de 50°C. Le résidu huileux est repris par 250 $cm^3$ d'eau. Le mélange obtenu est extrait par 3 fois 200 $cm^3$ d'éther éthylique. Les extraits organiques réunis sont lavés par 4 fois 150 $cm^3$ d'eau, séchés sur sulfate de magnésium et concentrés sous pression réduite (20 kPa) à environ 30°C. On obtient 122 g de trifluoro-2,3,4 N-[(éthoxy-1' éthoxycarbonyl-2') éthylidène]-aniline sous forme d'une huile jaune qui est utilisée sans autre purification pour les étapes ultérieures.

Le chlorhydrate d'(éthoxycarbonyl-2 éthoxy-1) éthylidèneamine a été préparé selon la méthode décrite par A. Pinner, et coll., Ber. Dtsch. Chem. Ges., 28, 478 (1895).

## EXEMPLE 25

En opérant dans les conditions de l'exemple 39, mais à partir de 0,978 g d'éthoxycarbonyl-3 difluoro-7,9 méthyl-1 oxo-4 (phényl-3 pipérazinyl-1)-8 dihydro-1,4 benzo [b] naphtyridine-1,8-(R), on obtient, après 1 recristallisation dans 25 $cm^3$ de diméthyl- formamide, 0,540 g d'acide difluoro-7,9 méthyl-1 oxo-4 (phényl-3 pipérazinyl-1)-8 dihydro-1,4 benzo [b] naphtyridine-1,8 carboxylique-3-(R) sous forme d'un solide jaune se décomposant à 270-272°C.

$[\alpha]_D^{20}$ = + 113° ± 5 (c=2; trichlorométhane).

En opérant dans les conditions de l'exemple 39, mais à partir de 1,21 g d'éthoxycarbonyl-3 trifluoro-7,8,9 méthyl-1 oxo-4 dihydro-1,4 benzo [b] naphtyridine-1,8 et de 0,7 g de phényl-2 pipérazine-(R), après recristallisation dans 22 $cm^3$ d'éthanol à 30% de diméthylformamide, on obtient 1,02 g d'éthoxycarbonyl-3 difluoro-7,9 méthyl-1 oxo-4 (phényl-3 pipérazinyl-1)-8 dihydro-1,4 benzo [b] naphtyridine-1,8-(R), sous forme d'un solide jaune fondant à 216°C.

$[\alpha]_D^{20}$ = + 98° ± 2 (c=0,5; acide acétique).

## EXEMPLE 26

En opérant dans les conditions de l'exemple 25, mais à partir de 1,33 g d'éthoxycarbonyl-3 difluoro-7,9 méthyl-1 oxo-4 (phényl-3 pipérazinyl-1)-8 dihydro-1,4 benzo [b] naphtyridine-1,8-(S), on obtient 0,779 g d'acide difluoro-7,9 méthyl-1 oxo-4 (phényl-3 pipérazinyl-1)-8 dihydro-1,4 benzo [b] naphtyridine-1,8 carboxylique-3-(S) sous forme d'un solide jaune se décomposant a 270-272°C.

$[\alpha]_D^{20}$ = + 118° ± 5 (c=0,2;trichlorométhane)

En opérant dans les conditions de l'exemple 26, mais à partir de 1,38 g d'éthoxycarbonyl-3 trifluoro-7,8,9 méthyl-1 oxo-4 dihydro-1,4 benzo [b] naphtyridine-1,8 et de 0,8 g de phényl-2 pipérazine-(S),on obtient 1,02 g d'éthoxycarbonyl-3 difluoro-7,9 méthyl-1 oxo-4 (phényl-3 pipérazinyl-1)-8 dihydro-1,4 benzo [b] naphtyridine-1,8-(S), sous forme d'un solide jaune fondant à 216°C.

$[\alpha]_D^{20}$ = - 99°± 2 (c=0,5; acide acétique).

## EXEMPLE 27

L'acide cyclopropyl-1 difluoro-7,9 oxo-4 (phényl-3 pipérazinyl-1)-8 que-3-(RS) a été préparé dans les conditions de l'exemple 24, mais à partir de 1,8 g de cyclopropyl-1 éthoxycarbonyl-3 difluoro-7,9 oxo-4 (phényl-3 pipérazinyl-1)-8 dihydro-1,4 benzo [b] naphtyridine-1,8-(RS). Après une recristallisation dans un mélange de 30 $cm^3$ de diméthylformamide et de 20 $cm^3$ d'éthanol, on obtient 1,2 g d'acide cyclopropyl-1 difluoro-7,9 oxo-4 (phényl-3 pipérazinyl-1)-8 dihydro-1,4 benzo [b] naphtyridine-1,8 carboxylique-3-(RS), sous forme d'un solide jaune fondant à 274°C.

Le cyclopropyl-1 éthoxycarbonyl-3 difluoro-7,9 oxo-4 (phényl-3 pipérazinyl-1)-8 dihydro-1,4 benzo [b] naphtyridine-1,8 (RS) a été préparé dans les conditions de l'exemple 19, mais à partir de 1,8 g de cyclopropyl-1 éthoxycarbonyl-3 trifluoro-7,8,9 oxo-4 dihydro-1,4 benzo [b] naphtyridine-1,8 et de 3,2 g de phényl-2 pipérazine (RS). Après recristallisation dans un mélange de 5 $cm^3$ de diméthylformamide et de 40 $cm^3$ d'éthanol, on obtient 1,8 g de cyclopropyl-1 éthoxycarbonyl-3 difluoro-7,9 oxo-4 (phényl-3 pipérazinyl-1)-8 dihydro-1,4 benzo [b] naphtyridine-1,8-(RS) sous forme d'un solide jaune fondant à 242°C.

Le cyclopropyl-1 éthoxycarbonyl-3 trifluoro-7,8,9 oxo-4 dihydro-1,4 benzo [b] naphtyridine-1,8 peut être préparé dans les conditions suivantes :

Dans une solution de 7 g de (chloro-2 trifluoro-6,7,8 quinoléinecarbonyle-3)-2 diméthylamino-3 acrylate d'éthyle dans 100 $cm^3$ de trichlorométhane maintenue à une température voisine de 20°C, on ajoute en 5 minutes 4,12 g de cyclopropylamine et agite encore 4 heures à cette même température. Le mélange réactionnel est concentré sous pression réduite (20 kPa) à environ 50°C. Le résidu huileux obtenu est repris par 100 $cm^3$ d'éthanol et 3 g de D.B.U. Le mélange est chauffé à 80°C et maintenu, sous agitation, à cette même température pendant 1 heure et demie. Après refroidissement à environ 20°C l'insoluble est essoré, lavé par 2 fois 30 $cm^3$ d'éthanol et 2 fois 30 $cm^3$ d'éther isopropylique. On obtient 4,5 g de cyclopropyl-1 éthoxycarbonyl-3 trifluoro-7,8,9 oxo-4 dihydro-1,4 benzo [b] naphtyridine-1,8 sous forme d'un solide incolore fondant à 260°C.

EXEMPLE 28

En opérant dans les conditions de l'exemple 39, mais à partir de 2,4 g d'éthoxycarbonyl-3 difluoro-7,9 (fluoro-2 éthyl)-1 oxo-4 (phényl-3 pipérazinyl-1)-8 dihydro-1,4 benzo [b] naphtyridine-1,8-(RS), on obtient 1,6 g d'acide difluoro-7,9 (fluoro-2 éthyl)-1 oxo-4 (phényl-3 pipérazinyl-1)-8 dihydro-1,4 benzo [b] naphtyridine-1,8 carboxylique-3-(RS) sous forme d'un solide jaune se décomposant à 307-310°C.

L'éthoxycarbonyl-3 difluoro-7,9 (fluoro-2 éthyl)-1 oxo-4 (phényl-3 pipérazinyl-1)-8 dihydro-1,4 benzo [b] naphtyridine-1,8-(RS) a été préparé dans les conditions suivantes: Une suspension de 2,8 g d'éthoxycarbonyl-3 (fluoro-2 éthyl)-1 trifluoro-7,8,9 oxo-4 dihydro-1,4 benzo [b] naphtyridine-1,8, de 1,7 g de phényl-2 pipérazine-(RS) et de 1,1 g de carbonate de sodium dans 40 $cm^3$ de diméthylsulfoxyde est chauffée sous agitation à une température voisine de 100°C, pendant 2 heures. Après refroidissement à environ 20°C, le mélange réactionnel est versé dans 200 $cm^3$ d'eau et extrait par 3 fois 100 $cm^3$ de trichlorométhane. Les extraits organiques réunis sont lavés par 1 fois 50 $cm^3$ d'eau et concentrés sous pression réduite (20 kPa) à une température voisine de 40°C. Le résidu huileux est repris par 100 $cm^3$ d'éthanol et concentré de nouveau sous pression réduite dans les conditions précédentes. Le solide obtenu est recristallisé dans 100 $cm^3$ d'éthanol à 20 % de diméthylformamide, essoré et lavé par 2 fois 20 $cm^3$ d'éthanol. On obtient 2,4 g du produit attendu sous forme d'un solide jaune fondant à 208°C.

L'éthoxycarbonyl-3 (fluoro-2 éthyl)-1 trifluoro-7,8,9 oxo-4 dihydro-1,4 benzo [b] naphtyridine-1,8 a été préparé dans des conditions voisines de l'exemple 3, mais à partir de 3,9 g de (chloro-2 trifluoro-6,7,8 quinoléinecarbonyle-3)-2 diméthylaminoacrylate d'éthyle, de 3 g de chlorhydrate de fluoro-2 éthylamine, de 4,2 $cm^3$ de triéthylamine dans 100 $cm^3$ d'éthanol. Après addition de 1,8 $cm^3$ de D.B.U.la solution est chauffée 2 heures à 80°C. On obtient, après le même traitement du mélange réactionnel que dans l'exemple 24, 2,8 g d'éthoxycarbonyl-3 trifluoro-7,8,9 (fluoro-2 éthyl)-1 oxo-4 dihydro-1,4 benzo [b] naphtyridine-1,8 sous forme d'un solide jaune fondant à 302-304°C.

Le (chloro-2 trifluoro-6,7,8 quinoléinecarbonyle-3)-2 diméthylaminoacrylate d'éthyle peut être préparé comme décrit à l'exemple 24.

EXEMPLE 29

Une suspension de 2,65 g (benzyl-3 pipérazinyl-1)-8 éthoxycarbonyl-3 fluoro-7 méthyl-1 oxo-4 dihydro-1,4 benzo [b] naphtyridine-1,8-(RS) dans 14 $cm^3$ de potasse aqueuse N et 20 $cm^3$ d'éthanol est chauffée à une température voisine de 80°C pendant 20 minutes, additionnée, à cette même température, de 32 $cm^3$ d'acide acétique à 5 % et agitée pendant 20 minutes. L'insoluble est essoré à environ 80°C, lavé par 2 fois 20 $cm^3$ d'eau, 2 fois 20 $cm^3$ d'éthanol et 2 fois 20 $cm^3$ d'éther éthylique. Après 2 recristallisations dans 50 $cm^3$ de diméthylformamide à chaque fois, on obtient 2,05 g d'acide (benzyl-3 pipérazinyl-1)-8 fluoro-7 méthyl-1 oxo-4 dihydro-1,4 benzo [b] naphtyridine-1,8 carboxylique-3-(RS) sous forme d'un solide jaune se décomposant à 270-275°C.

Le (benzyl-3 pipérazinyl-1)-8 éthoxycarbonyl-3 fluoro-7 méthyl-1 oxo-4 dihydro-1,4 benzo [b] naphtyridine-1,8-(RS) est préparé de la manière suivante :

Une suspension de 1,3 g d'éthoxycarbonyl-3 difluoro-7,8 méthyl-1 oxo-4 dihydro-1,4 benzo [b] naphtyridine-1,8 et 1,3 g de benzyl-2 pipérazine-(RS) dans 25 $cm^3$ de diméthylsulfoxyde est chauffée à environ 90°C sous agitation pendant 1 heure 45 minutes. Après refroidissement à une température voisine de 20°C, le mélange réactionnel est additionné de 100 $cm^3$ d'eau, extrait par 3 fois 30 $cm^3$ de trichlorométhane. Les extraits organiques réunis sont lavés par 3 fois 30 $cm^3$ d'eau, séchés sur sulfate de magnésium, filtrés et concentrés à sec à environ 50°C sous pression réduite (20 kPa). Après 1 recristallisation dans un mélange de 4 $cm^3$ de diméthylformamide et de 1 $cm^3$ d'éthanol, on obtient 1,6 g de (benzyl-3 pipérazinyl-1)-8 éthoxycarbonyl-3 fluoro-7 méthyl-1 oxo-4 dihydro-1,4 benzo [b] naphtyridine-1,8-(RS) sous forme d'un solide jaune fondant à 190°C.

La benzyl-2 pipérazine-(RS) peut être préparée par hydrogénation de la benzyl-2 pyrazine obtenue selon la méthode décrite par J.D. BEHUN et R. LEVINE, J. Org. Chem., 26, 3379, (1961).

Une solution de 8 g de benzyl-2 pyrazine dans 60 $cm^3$ d'acide acétique est additionnée de 0,8 g de dioxyde de platine et hydrogénée sous une pression de 1 atmosphère à environ 20°C. Après la fin de l'absorption d'hydrogène, le catalyseur est éliminé par filtration sur silice diatomée. La solution est concentrée à sec sous pression réduite (20 kPa) à environ 60°C. L'extrait sec est mis en suspension dans 40 $cm^3$ d'éthanol, additionné à environ 20°C d'une solution d'éthylate de sodium préparée à partir de 1,49 g de sodium dans 40 $cm^3$ d'éthanol. Après 2 heures d'agitation à environ 20°C, la suspension est concentrée à sec sous pression réduite (20 kPa) à environ 30°C. L'extrait sec est repris par 60 $cm^3$ d'éther éthylique. L'insoluble est éliminé par filtration sur silice diatomée. Le filtrat est concentré à sec sous pression réduite dans les mêmes conditions que précédemment. L'extrait sec est repris par 20 $cm^3$ d'éther isopropylique, essoré et lavé par 2 fois 5 $cm^3$ du même solvant. On obtient 5,32 g de benzyl-2 pipérazine-(RS) sous forme d'un solide beige clair fondant à 90°C.

## EXEMPLE 30

En opérant dans les conditions de l'exemple 39, mais à partir de 1,3 g d'éthoxycarbonyl-3 fluoro-7 [(fluoro-2 phényl)-3 pipérazinyl-1]-8 méthyl-1 oxo-4 dihydro-1,4 benzo [b] naphtyridine-1,8-(RS), on obtient 0,97 g d'acide fluoro-7 [(fluoro-2 phényl)-3 pipérazinyl-1]-8 méthyl-1 oxo-4 dihydro-1,4 benzo [b] naphtyridine-1,8 carboxylique-3-(RS) sous forme d'un solide jaune fondant à 264°C.

L'éthoxycarbonyl-3 fluoro-7 [(fluoro-2 phényl)-3 pipérazinyl-1]-8 méthyl-1 oxo-4 dihydro-1,4 benzo [b] naphtyridine-1,8-(RS) a été préparée dans les conditions de l'exemple 39, mais à partir de 1,59 g d'éthoxycarbonyl-3 difluoro-7,8 méthyl-1 oxo-4 dihydro-1,4 benzo [b] naphtyridine-1,8 et de 1,08 g de (fluoro-2 phényl)-2 pipérazine-(RS). Après recristallisation dans 60 $cm^3$ de diméthylformamide à 50 % d'éthanol, on obtient 1,3 g du produit attendu sous forme d'un solide jaune fondant à 228°C.

## EXEMPLE 31

L'acide difluoro-7,9 [(fluoro-4 phényl)-3 pipérazinyl-1]-8 méthyl-1 oxo-4 dihydro-1,4 benzo [b] naphtyridine-1,8 carboxylique-3-(RS) a été préparé dans les conditions de l'exemple 24, mais à partir de 1,5 g d'éthoxycarbonyl-3 difluoro-7,9 [(fluoro-4 phényl)-3 pipérazinyl-1]-8 méthyl-1 oxo-4 dihydro-1,4 benzo [b] naphtyridine-1,8-(RS). Après 1 recristallisation dans 30 $cm^3$ de diméthylformamide, on obtient 1 g d'acide difluoro-7,9 [(fluoro-4 phényl)-3 pipérazinyl-11-8 méthyl-1 oxo-4 dihydro-1,4 benzo [b] naphtyridine-1,8 carboxylique-3-(RS) sous forme d'un solide jaune fondant à 266°C.

L'éthoxycarbonyl-3 difluoro-7,9 [(fluoro-4 phényl)-3 pipérazinyl-1]-8 méthyl-1 oxo-4 dihydro-1,4 benzo [b] naphtyridine-1,8-(RS) a été préparé dans les conditions de l'exemple 39, mais à partir de 2 g d'éthoxycarbonyl-3 trifluoro-7,8,9 méthyl-1 oxo-4 dihydro-1,4 benzo [b] naphtyridine-1,8 et de 4 g de (fluoro-4 phényl)-2 pipérazine(RS). Après 1 recristallisation dans un mélange de 3,5 $cm^3$ de diméthylformamide et de 31,5 $cm^3$ d'éthanol, on obtient 2,1 g d'éthoxycarbonyl-3 difluoro-7,9 [(fluoro-4 phényl)-3 pipérazinyl-1]-8 méthyl-1 oxo-4 dihydro-1,4 benzo [b] naphtyridine-1,8-(RS), sous forme d'un solide beige fondant à 242°C.

## EXEMPLE 32

L'acide éthyl fluoro-7 [(méthoxy-4 phényl)-3 pipérazinyl-1]-8 oxo-4 dihydro-1,4 benzo [b] naphtyridine-1,8 carboxylique-3-(RS) a été préparé dans les conditions décrites ci-après à l'exemple 33, mais à partir de 1,5 g d'éthoxycarbonyl-3 éthyl-1 fluoro-7 [(méthoxy-4 phényl)-3 pipérazinyl-1)-8 oxo-4 dihydro-1,4 benzo [b] naphtyridine-1,8-(RS). Après recristallisation dans 30 $cm^3$ de diméthylformamide, on obtient 0,85 g d'acide éthyl-1 fluoro-7 [(méthoxy-4 phényl)-3 pipérazinyl-1]-8 oxo-4 dihydro-1,4 benzo [b] naphtyridine-1,8 carboxylique-3-(RS), sous forme d'un solide jaune fondant à 270°C.

L'éthoxycarbonyl-3 éthyl-1 fluoro-7 [(méthoxy-4 phényl)-3 pipérazinyl-1]-8 oxo-4 dihydro-1,4 benzo [b] naphtyridine-1,8-(RS) a été préparé dans les conditions de l'exemple 33, mais à partir de 1,99 g d'éthoxycarbonyl-3 éthyl-1 difluoro-7,8 oxo-4 dihydro-1,4 benzo [b] naphtyridine-1,8 et de 2,3 g de (méthoxy-4 phényl)-2 pipérazine-(RS). On obtient 2,2 g d'éthoxycarbonyl-3 éthyl-1 fluoro-7 [(méthoxy-4 phényl)-3 pipérazinyl-1]-8 oxo-4 dihydro-1,4 benzo [b] naphtyridine-1,8- (RS) sous forme d'un solide beige fondant à 210-212°C.

L'éthoxycarbonyl-3 éthyl-1 difluoro-7,8 oxo-4 dihydro-1,4 benzo [b] naphtyridine-1,8 a été préparé dans les conditions suivantes :

Dans une suspension de 20 g de (chloro-2 difluoro-6,7 quinoléinecarbonyle-3)-2 diméthylamino-3 acrylate d'éthyle dans 200 $cm^3$ d'éthanol à environ 2°C, on ajoute, en 10 minutes, entre 2°et 5°C, sous agitation, une solution à environ 2°C de 14,6 g d'éthylamine dans 200 $cm^3$ d'éthanol, agite encore 40 minutes entre 2 et 5°C puis laisse la température remonter à environ 20°C en 2 heures. Après 24 heures à environ 20°C, l'insoluble est essoré, lavé par 2 fois 30 $cm^3$ d'éthanol et 2 fois 50 $cm^3$ d'éther isopropylique. On obtient 16,35 g d'éthoxycarbonyl-3 éthyl-1 difluoro-7,8 oxo-4 dihydro-1,4 benzo [b] naphtyridine-1,8 sous forme d'un solide beige fondant à 290°C.

EXEMPLE 33

L'acide cyclopropyl-1- fluoro-7 [(méthoxy-4 phényl)-3 pipérazinyl-1]-8 oxo-4 dihydro-1,4 benzo [b] naphtyridine-1,8 carboxylique-3-(RS) a été préparé dans les conditions de l'exemple 29, mais à partir de 1,5 g de cyclopropyl-1 éthoxycarbonyl-3 fluoro-7 [(méthoxy-4 phényl)-3 pipérazinyl-1]-8 oxo-4 dihydro-1,4 benzo [b] naphtyridine-1,8 -(RS) dans 18,4 $cm^3$ de potasse aqueuse N et 18,4 $cm^3$ d'éthanol. Après 1 recristallisation dans 50 $cm^3$ de diméthylformamide, on obtient 1,5 g d'acide cyclopropyl-1 fluoro-7 [(méthoxy-4 phényl)-3 pipérazinyl-1]-8 oxo-4 dihydro-1,4 benzo [b] naphtyridine-1,8 carboxylique-3-(RS) sous forme d'un solide jaune se décomposant à 287°C.

Le cyclopropyl-1 éthoxycarbonyl-3 fluoro-7 [(méthoxy-4 phényl)-3 pipérazinyl-1]-8 oxo-4 dihydro-1,4 benzo [b] naphtyridine-1,8 -(RS) a été préparé de la manière suivante :

Une suspension de 2 g de cyclopropyl-1 éthoxycarbonyl-3 difluoro-7,8 oxo-4 dihydro-1,4 benzo [b] naphtyridine-1,8 et de 2,3 g de (méthoxy-4 phényl)-3 pipérazine-(RS) dans 20 $cm^3$ de diméthylsulfoxyde est chauffée à une température voisine de 90°C pendant 1 heure et demie. Après refroidissement à environ 20°C, l'insoluble est essoré, lavé par 3 fois 15 $cm^3$ d'eau et recristallisé dans un mélange de 100 $cm^3$ d'éthanol et de 25 $cm^3$ de diméthylformamide. On obtient 2,1 g de cyclopropyl-1 éthoxycarbonyl-3 fluoro-7 [(méthoxy-4 phényl)-3 pipérazinyl-1]-8 oxo-4 dihydro-1,4 benzo [b] naphtyridine-1,8-(RS) sous forme d'un solide jaune fondant à 199°C.

La cyclopropyl-1 éthoxycarbonyl-3 difluoro-7,8 oxo-4 dihydro-1,4 benzo [b] naphtyridine-1,8 a été préparée de la manière suivante :

Une solution de 20 g de (chloro-2 difluoro-6,7 quinoléinecarbonyle-3)-2 diméthylamino-3 acrylate d'éthyle et de 9,25 g de cyclopropylamine dans 80 $cm^3$ de trichlorométhane est agitée à une température voisine de 20°C pendant 5 heures. La solution est concentrée sous pression réduite (20 kPa) à environ 40°C. Le résidu est repris par 300 $cm^3$ d'éthanol, additionné de 8,2 g de D.B.U. et chauffé, sous agitation, à environ 75°C pendant 30 minutes. Après refroidissement à environ 20°C, l'insoluble est essoré, lavé par 2 fois 30 $cm^3$ d'éthanol. On obtient 11,1 g de cyclopropyl-1 éthoxycarbonyl-3 difluoro-7,8 oxo-4 dihydro-1,4 benzo [b] naphtyridine-1,8 sous forme d'un solide jaune fondant à 230°C.

EXEMPLE 34

L'acide difluoro-7,9 [(méthoxy-4 phényl)-3 pipérazinyl-1]-8 méthyl-1 oxo-4 dihydro-1,4 benzo [b] naphtyridine-1,8 carboxylique-3-(RS) a été préparé dans des conditions voisines de l'exemple 39, à partir de 2,8 g d'éthoxycarbonyl-3 difluoro-7,9 [(méthoxy-4 phényl)-3 pipérazinyl-1]-8 méthyl-1 oxo-4 dihydro-1,4 benzo [b] naphtyridine-1,8-(RS) dans 24 $cm^3$ de potasse aqueuse N et 25 $cm^3$ d'éthanol. On obtient 2 g d'acide difluoro-7,9 [(méthoxy-4 phényl)-3 pipérazinyl-1]-8 méthyl-1 oxo-4 dihydro-1,4 benzo [b] naphtyridine-1,8 carboxylique-3-(RS) sous forme d'un solide jaune fondant à 288-290°C.

L'éthoxycarbonyl-3 difluoro-7,9 [(méthoxy-4 phényl)-3 pipérazinyl-1]-8 méthyl-1 oxo-4 dihydro-1,4 benzo [b] naphtyridine-1,8-(RS) a été préparé dans les conditions suivantes:

Une suspension de 2,5 g d'éthoxycarbonyl-3 trifluoro-7,8,9 méthyl-1 oxo-4 dihydro-1,4 benzo [b] naphtyridine-1,8 et de 3,5 g de (méthoxy-4 phényl)-2 pipérazine-(RS) dans 40 $cm^3$ de diméthylsulfoxyde est chauffée, sous agitation, à une température voisine de 95°C pendant 2 heures. Après traitement du mélange réactionnel selon les conditions décrites pour l'exemple 39, on obtient 2,8 g d'éthoxycarbonyl-3 difluoro-7,9 [(méthoxy-4 phényl)-3 pipérazinyl-1]-8 méthyl-1 oxo-4 dihydro-1,4 benzo [b] naphtyridine-1,8-(RS) sous forme d'un solide

jaune fondant à 209°C qui est utilisé sans autre purification pour les étapes ultérieures.

L'éthoxycarbonyl-3 trifluoro-7,8,9 méthyl-1 oxo-4 dihydro-1,4 benzo [b] naphtyridine-1,8 peut être préparée comme décrit à l'exemple 24.

La (méthoxy-4 phényl)-2 pipérazine-(RS) a été préparée selon la méthode décrite à l'exemple 12.

EXEMPLE 35

En opérant dans les conditions de l'exemple 39, mais à partir de 0,9 g d'[(amino-4 phényl)-3 pipérazinyl-1]-8 éthoxycarbonyl-3 fluoro-7 méthyl-1 oxo-4 dihydro-1,4 benzo [b] naphtyridine-1,8-(RS), on obtient 0,7 g d'acide [(amino-4 phényl)-3 pipérazinyl-1]-8 fluoro-7 méthyl-1 oxo-4 dihydro-1,4 benzo [b] naphtyridine-1,8 carboxylique-3-(RS) sous forme d'un solide jaune se décomposant à 315°C.

L'[(amino-4 phényl)-3 pipérazinyl-1]-8 éthoxycarbonyl-3 fluoro-7 méthyl-1 oxo-4 dihydro-1,4 benzo [b] naphtyridine-1,8-(RS) a été préparée dans les conditions suivantes:

Une suspension de 0,7 g d'éthoxycarbonyl-3 fluoro-7 méthyl-1 [(nitro-4 phényl-3 pipérazinyl-1]-8 oxo-4 dihydro-1,4 benzo [b] naphtyridine-1,8-(RS) et de 5 g de nickel de Raney dans 150 $cm^3$ d'éthanol est hydrogénée sous une pression de 1 atmosphère à environ 20°C pendant 7 heures ce qui permet d'absorber la quantité théorique d'hydrogène (100 $cm^3$ à 20°C et 1 atmosphère). Après addition de 50 $cm^3$ de diméthylformamide, le catalyseur est éliminé par filtration et le filtrat concentré à sec sous pression réduite (20 kPa) à environ 50°C. Le résidu obtenu (0,550 g) est chromatographié sur 15 g de silice (230-400 Mesh) en suspension dans le chloroforme à 20 % d'éthanol et élué par 200 $cm^3$ du même mélange de solvants. Après concentration à sec dans les mêmes conditions que précédemment, on obtient 0,3 g d'[(amino-4 phényl)-3 pipérazinyl-1]-8 éthoxycarbonyl-3 fluoro-7 méthyl-1 oxo-4 dihydro-1,4 benzo [b] naphtyridine-1,8-(RS) sous forme d'un solide fondant à 180-182°C.

L'éthoxycarbonyl-3 fluoro-7 méthyl-1 [(nitro-4 phényl-3 pipérazinyl-1]-8 oxo-4 dihydro-1,4 benzo [b] naphtyridine-1,8-(RS) a été préparée dans les conditions de l'exemple 39, mais à partir hydro-1,4 benzo [b] naphtyridine-1,8 et de 0,5 g de (nitro-4 phényl)-2 pipérazine-(RS). Après recristallisation dans 10 $cm^3$ de diméthylformamide, on obtient 0,7 g du produit attendu sous forme d'un solide jaune se décomposant à environ 300°C.

La (nitro-4 phényl)-2 pipérazine-(RS) a été préparée selon les mêmes méthodes que celles utilisées dans l'exemple 8.

A partir de 18 g de nitro-4 phénylglyoxal, on obtient 6,2 g de produit attendu sous forme d'une huile brune qui a été utilisée sans autre purification pour les étapes ultérieures.

A partir de 24,4 g de nitro-4' bromo-2 acétophénone, on obtient 9 g de nitro-4 phényl glyoxal sous forme d'une huile brune qui a été utilisée sans autre purification pour les étapes ultérieures.

EXEMPLE 36

En opérant dans les conditions de l'exemple 39, mais à partir de 2,2 g d'éthoxycarbonyl-3 difluoro-7,9 {[(hydroxy-2 éthoxy)-4 phényl]-3 pipérazinyl-1}-8 méthyl-1 oxo-4 dihydro-1,4 benzo [b] naphtyridine-1,8-(RS), on obtient 1,6 g d'acide difluoro-7,9 {[(hydroxy-2 éthoxy)-4 phényl]-3 pipérazinyl-1}-8 méthyl-1 oxo-4 dihydro-1,4 benzo [b] naphtyridine-1,8 carboxylique-3-(RS) sous forme d'un solide jaune fondant à 226-228°C.

L'éthoxycarbonyl-3 difluoro-7,9 {[(hydroxy-2 éthoxy)-4 phényl]-3 pipérazinyl-1}-8 méthyl-1 oxo-4 dihydro-1,4 benzo [b] naphtyridine-1,8-(RS) a été préparée dans les conditions de l'exemple 39, mais à partir de 1,9 g d'éthoxycarbonyl-3 trifluoro-7,8,9 méthyl-1 oxo-4 dihydro-1,4 benzo [b] naphtyridine-1,8 et de 1,6 g d' [(hydroxy-2 éthoxy)-4 phényl]-2 pipérazine-(RS). On obtient 2,2 g de produit attendu sous forme d'un solide jaune fondant à 183°C.

L'éthoxycarbonyl-3 trifluoro-7,8,9 méthyl-1 oxo-4 dihydro-1,4 benzo [b] naphtyridine-1,8, peut être préparée comme décrit ci-après à l'exemple 24.

EXEMPLE 37

En opérant dans des conditions voisines de l'exemple 39, mais à partir de 2,4 g de cyclopropyl-1 éthoxycarbonyl-3 difluoro-7,9 {[(hydroxy-2 éthoxy)-4 phényl]-3 pipérazinyl-1}-8 oxo-4 dihydro-1,4 benzo [b] naphtyridine-1,8-(RS), et en additionnant 100 $cm^3$ d'éthanol à 50 % d'eau au mélange réactionnel, avant introduction de l'acide méthane sulfonique, on obtient, 1,4 g d'acide cyclopropyl-1 difluoro-7,9 {[(hydroxy-2 éthoxy)-4 phényl]-3 pipérazinyl-1}-8 oxo-4 dihydro-1,4 benzo [b] naphtyridine-1,8 carboxylique-3-(RS) sous forme d'un solide jaune fondant à 228-230°C.

La cyclopropyl-1 éthoxycarbonyl-3 difluoro-7,9 {[(hydroxy-2 éthoxy)-4 phényl]-3 pipérazinyl-1}-8 oxo-4 dihydro-1,4 benzo [b] naphtyridine-1,8-(RS) a été préparée dans les conditions de l'exemple 39, mais à partir

de 2 g de cyclopropyl-1 éthoxycarbonyl-3 trifluoro-7,8,9 oxo-4 dihydro-1,4 benzo [b] naphtyridine-1,8 et de 1,6 g de [(hydroxy-2 éthoxy)-4 phényl]-2 pipérazine-(RS). On obtient 2,4 g de produit attendu, sous forme d'un solide jaune fondant à 225-226°C.

La cyclopropyl-1 éthoxycarbonyl-3 trifluoro-7,8,9 oxo-4 dihydro-1,4 benzo [b] naphtyridine-1,8 est préparée code décrit à l'exemple 27.

L'[(hydroxy-2 éthoxy)-4 phényl]-2 pipérazine-(RS) peut être préparée comme décrit à l'exemple 16.

EXEMPLE 38

En opérant dans les conditions de l'exemple 39, mais à partir de 2,1 g d'éthoxycarbonyl-3 fluoro-7 méthyl-1 {[(méthylènedioxy-3,4) phényl]-3 pipérazinyl-1}-8 oxo-4 dihydro-1,4 benzo [b] naphtyridine-1,8-(RS), on obtient après recristallisation dans 30 $cm^3$ de diméthylformamide: 1,6 g d'acide fluoro-7 méthyl-1 {[(méthylènedioxy-3,4) phényl]-3 pipérazinyl-1}-8 oxo-4 dihydro-1,4 benzo [b] naphtyridine-1,8 carboxylique-3-(RS) sous forme d'un solide jaune se décomposant à 255°C.

L'éthoxycarbonyl-3 fluoro-7 méthyl-1 {[(méthylènedioxy-3,4) phényl]-3 pipérazinyl-1}-8 oxo-4 dihydro-1,4 benzo [b] naphtyridine-1,8-(RS) a été préparée dans des conditions voisines de l'exemple 39, mais à partir de 1,59 g d'éthoxycarbonyl-3 difluoro-7,8 méthyl-1 oxo-4 dihydro-1,4 benzo [b] naphtyridine-1,8, de 1,25 g de (dioxyméthylène-3,4 phényl)-2 pipérazine-(RS) et de 0,64 g de carbonate de sodium. On obtient 2,15 g d'éthoxycarbonyl-3 fluoro-7 méthyl-1 {[(méthylènedioxy-3,4) phényl]-3 pipérazinyl-1}-8 oxo-4 dihydro-1,4 benzo [b] naphtyridine-1,8-(RS) sous forme d'un solide jaune fondant à 252°C.

La (méthylènedioxy-3,4 phényl)-2 pipérazine-(RS) a été préparée selon le procédé décrit dans la demande de brevet FR 2 351 108.

EXEMPLE 39

Une suspension de 1,3 g d'éthoxycarbonyl-3 fluoro-7 [(furyl-2)-3 pipérazinyl-1]-8 méthyl-1 oxo-4 dihydro-1,4 benzo[b]naphtyridine-1,8-(RS) dans 12 $cm^3$ de potasse aqueuse 0,5 N et 12 $cm^3$ d'éthanol est chauffée pendant 1 heure à une température voisine de 80°C, puis additionnée à cette même température de 6 $cm^3$ d'une solution aqueuse N d'acide méthanesulfonique. Après refroidissement à environ 20°C, l'insoluble est essoré, lavé par 3 fois 10 $cm^3$ d'eau, 3 fois par 10 $cm^3$ d'éthanol, 3 fois 10 $cm^3$ d'éther éthylique et recristallisé dans un mélange de 13 $cm^3$ d'éthanol et de 27 $cm^3$ de diméthylformamide. On obtient 0,570 g d'acide fluoro-7 [(furyl-2)-3 pipérazinyl-1]-8 méthyl-1 oxo-4 dihydro-1,4 benzo[b]naphtyridine-1,8 carboxylique-3-(RS) sous forme d'un solide jaune fondant à 258-260°C.

L'éthoxycarbonyl-3 fluoro-7 [(furyl-2)-3 pipérazinyl-1]-8 méthyl-1 oxo-4 dihydro-1,4 benzo[b]naphtyridine-1,8-(RS) a été préparée de la manière suivante:

Une suspension de 0,96 g d'éthoxycarbonyl-3 difluoro-7,8 méthyl-1 oxo-4 dihydro-1,4 benzo[b]naphtyridine-1,8, de 0,6 g de (furyl-2)-2 pipérazine-(RS) et de 0,38 g de carbonate de sodium dans 20 $cm^3$ de diméthylsulfoxyde est chauffée à environ 95°C pendant 5 heures et demie. Après refroidissement à environ 20°C, le mélange est versé dans 50 $cm^3$ d'eau à une température voisine de 5°C et extrait par 3 fois 100 $cm^3$ de dichlorométhane. Les extraits organiques réunis sont lavés par 3 fois 50 $cm^3$ d'eau, séchés sur sulfate de magnésium, filtrés et concentrés à sec, sous pression réduite (20 kPa), à environ 40°C. Le résidu obtenu est repris par 20 $cm^3$ d'éthanol, essoré, lavé par 2 fois 20 $cm^3$ d'éthanol et 3 fois 30 $cm^3$ d'éther éthylique. On obtient 1,3 g d'éthoxycarbonyl-3 fluoro-7 [(furyl-2)-3 pipérazinyl-1]-8 méthyl-1 oxo-4 dihydro-1,4 benzo[b]naphtyridine-1,8-(RS) sous forme d'un solide jaune fondant à 198-200°C qui est utilisé sans autre purification pour les étapes ultérieures.

L'éthoxycarbonyl-3 difluoro-7,8 méthyl-1 oxo-4 dihydro-1,4 benzo [b]naphtyridine-1,8 est préparée comme décrit à l'exemple 19.

La (furyl-2)-2 pipérazine-(RS) a été préparée par selon le procédé décrit dans la demande de brevet EP 230 053.

EXEMPLE 40

En opérant dans les conditions de l'exemple 39, mais à partir de 1,25 g de cyclopropyl-1 éthoxycarbonyl-3 fluoro-7 [(furyl-2)-3 pipérazinyl-1]-8 oxo-4 dihydro-1,4 benzo[b]naphtyridine-1,8-(RS), on obtient 0,95 g d'acide cyclopropyl-l fluoro-7 [(furyl-2)-3 pipérazinyl-1]-8 oxo-4 dihydro-1,4 benzo[b]naphtyridine-1,8 carboxylique-3-(RS) sous forme d'un solide jaune fondant à 240-241°C.

La cyclopropyl-1 éthoxycarbonyl-3 fluoro-7 [(furyl-2)-3 pipérazinyl-1]-8 oxo-4 dihydro-1,4 benzo[b]naphtyridine-1,8-(RS) a été préparée dans les conditions de l'exemple 39, mais à partir de 1,05 g de cyclopropyl-1 difluoro-7,8 éthoxycarbonyl-3 oxo-4 dihydro-1,4 benzo[b]naphtyridine-1,8 et de 0,6 g de (furyl-2)-2 pipérazine-(RS). On obtient 1,3 g de cyclopropyl-1 éthoxycarbonyl-3 fluoro-7 [(furyl-2)-3 pipérazinyl-1]-8 oxo-4 dihydro-1,4 benzo[b]naphtyridine-1,8-(RS) sous forme d'un solide jaune fondant à 182°C.

La cyclopropyl-1 éthoxycarbonyl-3 difluoro-7,8 oxo-4 dihydro-1,4 benzo[b]naphtyridine-1,8 a été préparée de la manière suivante :

Une solution de 20 g de (chloro-2 difluoro -6,7 quinoléinecarbonyle-3)-2 diméthylamino-3 acrylate d'éthyle et de 9,25 g de cyclopropylamine dans 80 $cm^3$ de trichlorométhane est agitée à une température voisine de 20°C pendant 5 heures. La solution est concentrée sous pression réduite (20 kPa) à environ 40°C. Le résidu est repris par 300 $cm^3$ d'éthanol, additionné de 8,2 g de D.B.U. et chauffé, sous agitation, à environ 75°C pendant 30 minutes. Après refroidissement à environ 20°C, l'insoluble est essoré, lavé par 2 fois 30 $cm^3$ d'éthanol. On obtient 11,1 g de cyclopropyl-1 éthoxycarbonyl-3 difluoro-7,8 oxo-4 dihydro-1,4 benzo[b]naphtyridine-1,8 sous forme d'un solide jaune fondant à 230°C.

EXEMPLE 41

En opérant dans les conditions de l'exemple 39, mais à partir de 1,58 g d'éthoxycarbonyl-3 difluoro-7,9 [(furyl-2)-3 pipérazinyl-1]-8 méthyl-1 oxo-4 dihydro-1,4 benzo[b]naphtyridine-1,8-(RS). On obtient 1,07 g d'acide difluoro-7,9 [(furyl-2)-3 pipérazinyl-1]-8 méthyl-1 oxo-4 dihydro-1,4 benzo[b]naphtyridine-1,8 carboxylique-3-(RS) sous forme d'un solide jaune fondant à 295-296°C.

L'éthoxycarbonyl-3 difluoro-7,9 [(furyl-2)-3 pipérazinyl-1]-8 méthyl-1 oxo-4 dihydro-1,4 benzo[b]naphtyridine-1,8-(RS) a été préparée dans les conditions de l'exemple 39, mais à partir de 1,85 g d'éthoxycarbonyl-3 trifluoro-7,8,9 méthyl-1 oxo-4 dihydro-1,4 benzo[b]naphtyridine-1,8 et de 1 g de (furyl-2)-2 pipérazine-(RS). Après concentration sous pression réduite des extraits organiques réunis (20 kPa, environ 40°C) le solide résiduel est recristallisé dans 100 $cm^3$ d'éthanol. On obtient 1,72 g d'éthoxycarbonyl-3 difluoro-7,9 [(furyl-2)-3 pipérazinyl-1]-8 méthyl-1 oxo-4 dihydro-1,4 benzo[b]naphtyridine-1,8-(RS) sous forme d'un solide jaune fondant à 208-210°C.

EXEMPLE 42

En opérant dans les conditions de l'exemple 39, mais à partir de 2 g d'éthoxycarbonyl-3 fluoro-7 méthyl-1 oxo-4 [(thiényl-2)-3 pipérazinyl-1]-8 dihydro-1,4 benzo[b]naphtyridine-1,8-(RS), on obtient sans recristallisation 1,8 g d'acide fluoro-7 méthyl-1 oxo-4 [(thiényl-2)-3 pipérazinyl-1]-8 dihydro-1,4 benzo[b]naphtyridine-1,8 carboxylique-3-(RS) pur sous forme d'un solide jaune fondant à 268-270°C.

L'éthoxycarbonyl-3 fluoro-7 méthyl-1 oxo-4 [(thiényl-2)-3 pipérazinyl-1]-8 dihydro-1,4 benzo[b]naphtyridine-1,8-(RS) a été préparée dans les conditions de l'exemple 39, mais à partir de 1,59 g d'éthoxycarbonyl-3 difluoro-7,8 méthyl-1 oxo-4 dihydro-1,4 benzo[b]naphtyridine-1,8 et de 1,02 g de (thiényl-2)-2 pipérazine-(RS). On obtient 2,25 g d'éthoxycarbonyl-3 fluoro-7 méthyl-1 oxo-4 [(thiényl-2)-3 pipérazinyl-1]-8 dihydro-1,4 benzo[b]naphtyridine-1,8-(RS) sous forme d'un solide jaune fondant à 228°C.

La (thiényl-2)-2 pipérazine-(RS) a été préparée selon les conditions décrites par Jeffrey W.H. Watthey et Coll., J. Med. Chem., 26, 1116 (1983).

EXEMPLE 43

En opérant dans les conditions de l'exemple 39, mais à partir de 2 g de cyclopropyl-1 éthoxycarbonyl-3 fluoro-7 oxo-4 [(thiényl-2)-3 pipérazinyl-1]-8 dihydro-1,4 benzo[b]naphtyridine-1,8-(RS), on obtient 1,75 g d'acide cyclopropyl-1 fluoro-7 oxo-4 [(thiényl-2)-3 pipérazinyl-1]-8 dihydro-1,4 benzo[b]naphtyridine-1,8 carboxylique-3-(RS) sous forme d'un solide jaune fondant à 245°C.

La cyclopropyl-1 éthoxycarbonyl-3 fluoro-7 oxo-4 [(thiényl-)-3 pipérazinyl-1]-8 dihydro-1,4 benzo[b]naphtyri dine-1,8-(RS) a été préparée dans les conditions de l'exemple 39, mais à partir de 1,7 g de cyclopropyl-1 difluoro-7,8 éthoxycarbonyl-3 oxo-4 dihydro-1,4 benzo[b]naphtyridine-1,8 et de 1 g de (thiényl-2)-2 pipérazine-(RS). On obtient 2,2 g de cyclopropyl-1 éthoxycarbonyl-3 fluoro-7 oxo-4 [(thiényl-2)-3 pipérazinyl-1]-8 dihydro-1,4 benzo[b] naphtyridine-1,8-(RS) sous forme d'un solide jaune fondant à 210-212°C.

EXEMPLE 44

En opérant dans les conditions de l'exemple 39, mais à partir de 1,6 g d'éthoxycarbonyl-3 difluoro-7,9 méthyl-1 oxo-4 [(thiényl-2)-3 pipérazinyl-1]-8 dihydro-1,4 benzo[b]naphtyridine-1,8-(RS), on obtient 0,75 g d'acide difluoro-7,9 méthyl-1 oxo-4 [(thiényl-2)-3 pipérazinyl-1]-8 dihydro-1,4 benzo[b]naphtyridine-1,8 carboxy-

lique-3-(RS) sous forme d'un solide jaune fondant à 292-294°C.

L'éthoxycarbonyl-3 difluoro-7,9 méthyl-1 oxo-4 [(thiényl-2)-3 pipérazinyl-1]-8 dihydro-1,4 benzo[b]naphtyridine-1,8-(RS) a été préparée dans les conditions de l'exemple 39, mais à partir de 1,68 g d'éthoxycarbonyl-3 trifluoro-7,8,9 méthyl-1 oxo-4 dihydro-1,4 benzo[b]naphtyridine-1,8 et de 1,01 g de (thiényl-2)-2 pipérazine-(RS). On obtient ainsi 1,68 g d'éthoxycarbonyl-3 difluoro-7,9 méthyl-1 oxo-4 [(thiényl-2)-3 pipérazinyl-1]-8 dihydro-1,4 benzo[b]naphtyridine-1,8-(RS) sous forme d'un solide jaune fondant à 220°C.

EXEMPLE 45

En opérant dans les conditions de l'exemple 39, mais à partir de 3,2 g d'éthoxycarbonyl-3 fluoro-7 (hydroxy-4 pipérazinyl-1)-8 méthyl-1 oxo-4 dihydro-1,4 benzo[b]naphtyridine-1,8, on obtient 2,86 g d'acide fluoro-7 (hydroxy-4 pipérazinyl-1)-8 méthyl-1 oxo-4 dihydro-1,4 benzo[b]naphtyridine-1,8 carboxylique-3 sous forme d'un solide jaune fondant à 290°C.

L'éthoxycarbonyl-3 fluoro-7 (hydroxy-4 pipérazinyl-1)-8 méthyl-1 oxo-4 dihydro-1,4 benzo[b]naphtyridine-1,8 a été préparée dans les conditions de l'exemple 39, mais à partir de 3 g d'éthoxycarbonyl-3 difluoro-7,8 méthyl-1 oxo-4 dihydro-1,4 benzo[b]naphtyridine-1,8, de 1,97 g de dichlorhydrate d'hydroxy-1 pipérazine et de 1,99 g de carbonate de sodium. On obtient 3,25 g d'éthoxycarbonyl-3 fluoro-7 (hydroxy-4 pipérazinyl-1)-8 méthyl-1 oxo-4 dihydro-1,4 benzo[b]naphtyridine-1,8 sous forme d'un solide jaune fondant à 258-260°C.

Le dichlorhydrate d'hydroxy-1 pipérazine a été préparé selon le procédé décrit par Toschio Uno et Coll., J. Het. Chem., 26, 393 (1988).

EXEMPLE 46

En opérant dans les conditions de l'exemple 39, mais à partir de 1,4 g d'éthoxycarbonyl-3 difluoro-7,9 (hydroxy-4 pipérazinyl-1)-8 méthyl-1 oxo-4 dihydro-1,4 benzo [b] napthtyridine-1,8, on obtient 0,5 g d'acide difluoro-7,9 (hydroxy-4 pipérazinyl-1)-8 méthyl-1 oxo-4 dihydro-1,4 benzo[b]naphtyridine-1,8 carboxylique-3 sous forme d'un solide jaune fondant à 285-288°C.

L'éthoxycarbonyl-3 difluoro-7,9 (hydroxy-4 pipérazinyl-1)-8 méthyl-1 oxo-4 dihydro-1,4 benzo [b] napthtyridine-1,8 a été préparé dans les conditions de l'exemple 39, mais à partir de 1,9 g d'éthoxycarbonyl-3 trifluoro-7,8,9 méthyl-1 oxo-4 dihydro-1,4 benzo[b]naphtyridine-1,8, de 1 g de dichlorhydrate d'hydroxy-1 pipérazine et de 1,6 g de carbonate de potassium. On obtient 1,4 g d'éthoxycarbonyl-3 difluoro-7,9 (hydroxy-4 pipérazinyl-1)-8 méthyl-1 oxo-4 dihydro-1,4 benzo[b]naphtyridine-1,8 sous forme d'un solide jaune fondant à 255-258°C.

La présente invention concerne également les compositions pharmaceutiques utilisables en médecine humaine ou vétérinaire qui contiennent comme produit actif au moins un produit de formule générale (I) à l'état pur (sous forme libre ou sous forme de sel) ou sous forme d'une association avec un ou plusieurs diluants ou adjuvants compatibles et pharmaceutiquement acceptables. Ces compositions peuvent être utilisées par voie orale, parentérale ou rectale.

Comme compositions solides pour administration orale, peuvent être utilisés des comprimés, des pilules, des poudres ou des granulés. Dans ces compositions, le produit actif selon l'invention est mélangé à un ou plusieurs diluants ou adjuvants inertes, tels que saccharose, lactose ou amidon. Ces compositions peuvent également comprendre des substances autres que les diluants, par exemple un lubrifiant tel que le stéarate de magnésium.

Comme compositions liquides pour administration orale, on peut utiliser des émulsions pharmaceutiquement acceptables, des solutions, des suspensions, des sirops et des élixirs contenant des diluants inertes tels que l'eau ou l'huile de paraffine. Ces compositions peuvent également comprendre des substances autres que les diluants, par exemple des produits mouillants, édulcorants ou aromatisants.

Les compositions pour administration parentérale, peuvent être des solutions stériles aqueuses ou non aqueuses, des suspensions ou des émulsions. Comme solvant ou véhicule, on peut employer le propylèneglycol, un polyéthylèneglycol, des huiles végétales, en particulier l'huile d'olive, et des esters organiques injectables, par exemple l'oléate d'éthyle. Ces compositions peuvent également contenir des adjuvants, en particulier des agents mouillants, émulsifiants ou dispersants. La stérilisation peut se faire de plusieurs façons, par exemple à l'aide d'un filtre bactériologique, en incorporant à la composition des agents stérilisants, par irradiation ou par chauffage. Elles peuvent également être préparées sous forme de compositions solides stériles qui seront dissoutes au moment de l'emploi dans de l'eau stérile ou tout autre milieu stérile injectable.

Les compositions pour administration rectale sont les suppositoires ou les capsules rectales qui peuvent contenir, outre le produit actif, des excipients tels que le beurre de cacao ou la suppo-cire.

En thérapeutique humaine ou vétérinaire, les compositions selon l'invention sont particulièrement utiles dans le traitement des infections d'origine bactérienne.

D'une façon générale, le médecin déterminera la posologie qu'il estime la plus appropriée en fonction de l'âge, du poids, du degré de l'infection et des autres facteurs propres au sujet à traiter. Généralement, les doses sont comprises entre 0,2 et 1 g de produit actif deux fois par jour, par voie orale ou parentérale pour un adulte.

Les exemples suivants, donnés à titre non limitatif, illustrent des compositions selon l'invention :

Exemple A

On prépare selon les techniques habituelles des comprimés dosés à 250 mg de produit actif, ayant la composition suivante.

- acide fluoro-7 méthyl-1 oxo-4 (phényl-3 pipérazinyl-1)-8 dihydro-1,4 benzo[b]naphtyridine-1,8 carboxylique-3(R) 250 mg
- amidon 50 mg
- lactose 35 mg
- talc 15 mg

Exemple B

On prépare selon les techniques habituelles des comprimés dosés à 250 mg de produit actif, ayant la composition suivante.

- acide fluoro-7 [(furyl-2)-3 pipérazinyl-1]-8 méthyl-1 oxo-4 dihydro-1,4 benzo[b]naphtyridine-1,8 carboxylique-3(RS) 250 mg
- amidon 50 mg
- lactose 35 mg
- talc 15 mg

Les produits de formule générale (I) sont également intéressants dans le domaine de l'agrochimie pour les traitements antibactériens des plantes et des végétaux. Il est entendu que les compositions à usage agrochimique renfermant un produit de formule générale (I) entrent aussi dans le cadre de la présente invention.

Par ailleurs, les produits de formule générale (I) peuvent également être utilisés comme agents de conservation ou de désinfection des matières organiques ou minérales. Notamment dans l'industrie des colorants, de matières grasses, du papier, du bois, des polymères ou encore dans l'industrie textile, l'industrie alimentaire ou le traitement des eaux. Il est également entendu que les compositions renfermant un produit de formule générale (I) à l'état pur ou sous forme d'association avec des diluants ou adjuvants compatibles, entrent aussi dans le cadre de la présente invention.

## Revendications

### Revendications pour les Etats contractants suivants : AT, BE, CH, DE, DK, FR, GB, IT, LI, LU, NL, SE

1. Un dérivé de la benzo[b]naphtyridine-1,8 caractérisé en ce qu'il répond à la formule générale :

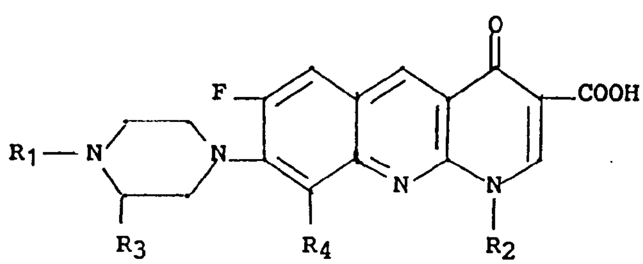

dans laquelle,

- $R_1$ représente un atome d'hydrogène ou un radical hydroxy ou alcoyle,
- $R_2$ représente un atome d'hydrogène ou un radical alcoyle, fluoroalcoyle, cycloalcoyle contenant 3 à 6 atomes de carbone, alcoyloxy ou alcoylamino,
- $R_3$ représente un radical phényle ou phénylalcoyle éventuellement substitué par un ou plusieurs atomes d'halogène ou radicaux alcoyle, cycloalcoyle contenant 3 à 6 atomes de carbone, alcoyloxy, cyano, amino, alcoylamino, dialcoylamino, alcoyloxyalcoyle, hydroxyalcoyle, hydroxyalcoyloxy, méthylènedioxy, aminoalcoyle, alcoylaminoalcoyle ou dialcoylaminoalcoyle dont les parties alcoyle peuvent former avec l'atome d'azote auquel elles sont rattachées, un hétérocycle à 5 ou 6 chaînons, ou représente un radical hétérocyclyle à 5 chaînons contenant 1 ou 2 hétéroatomes choisis parmi l'azote, l'oxygène ou le soufre, ou un radical N-alcoyle pyrrolyle, et

- $R_4$ représente un atome d'hydrogène ou un atome de fluor, ou bien
- $R_1$ est hydroxy, $R_2$ est méthyle, $R_3$ est un atome d'hydrogène et $R_4$ est défini comme ci-dessus,

et dont les radicaux alcoyle sont droits ou ramifiés et contiennent 1 à 4 atomes de carbone, sous ses formes isomères ou leurs mélanges ainsi que ses sels métalliques, ses sels d'addition avec les bases azotées, ses sels d'addition avec les acides et ses formes hydratées.

**2.** Un dérivé de la benzo[b]naphtyridine-1,8 selon la revendication 1, caractérisé en ce que :

- $R_1$ représente un atome d'hydrogène ou un radical hydroxy ou alcoyle,
- $R_2$ représente un atome d'hydrogène ou un radical alcoyle, fluoroalcoyle, cycloalcoyle contenant 3 à 6 atomes de carbone ou alcoyloxy,
- $R_3$ représente un radical phényle ou phénylalcoyle éventuellement substitué par un ou plusieurs atomes d'halogène ou radicaux alcoyle, cycloalcoyle contenant 3 à 6 atomes de carbone, alcoyloxy, cyano, amino, alcoyloxyalcoyle, hydroxyalcoyloxy, méthylènedioxy, ou représente un radical hétérocyclyle à 5 chaînons contenant 1 ou 2 hétéroatomes choisis parmi l'azote, l'oxygène ou le soufre, ou un radical N-alcoyle pyrrolyle, et
- $R_4$ représente un atome d'hydrogène ou un atome de fluor, ou bien
- $R_1$ est hydroxy, $R_2$ est méthyle, $R_3$ est un atome d'hydrogène et $R_4$ est défini comme ci-dessus,

et dont les radicaux alcoyle sont droits ou ramifiés etcontiennent 1 à 4 atomes de carbone, sous ses formes isomères ou leurs mélanges ainsi que ses sels métalliques, ses sels d'addition avec les bases azotées, ses sels d'addition avec les acides et ses formes hydratées.

**3.** Un dérivé de la benzo[b]naphtyridine-1,8 selon la revendication 1, caractérisé en ce qu'il s'agit de l'acide fluoro-7 méthyl-1 oxo-4 (phényl-3 pipérazinyl-1)-8 dihydro-1,4 benzo [b] naphtyridine-1,8 carboxylique-3 sous ses formes isomères ou leurs mélanges ainsi que ses sels métalliques, ses sels d'addition avec les bases azotées, ses sels d'addition avec les acides et ses formes hydratées.

**4.** Un dérivé de la benzo[b]naphtyridine-1,8 selon la revendication 1, caractérisé en ce qu'il s'agit de l'acide fluoro-7 méthyl-1 [(méthyl-4 phényl)-3 pipérazinyl-1]-8 oxo-4 dihydro-1,4 benzo [b] naphtyridine-1,8 carboxylique-3 sous ses formes isomères ou leurs mélanges ainsi que ses sels métalliques, ses sels d'addition avec les bases azotées, ses sels d'addition avec les acides et ses formes hydratées.

**5.** Un dérivé de la benzo[b]naphtyridine-1,8 selon la revendication 1, caractérisé en ce qu'il s'agit de l'acide fluoro-7 [(méthoxy-4 phényl)-3 pipérazinyl-1]-8 méthyl-1 oxo-4 dihydro-1,4 benzo [b] naphtyridine-1,8 carboxylique-3 sous ses formes isomères ou leurs mélanges ainsi que ses sels métalliques, ses sels d'addition avec les bases azotées, ses sels d'addition avec les acides et ses formes hydratées.

**6.** Un dérivé de la benzo[b]naphtyridine-1,8 selon la revendication 1, caractérisé en ce qu'il s'agit de l'acide difluoro-7,9 [(méthoxy-4 phényl)-3 pipérazinyl-1]-8 méthyl-1 oxo-4 dihydro-1,4 benzo [b] naphtyridine-1,8 carboxylique-3 sous ses formes isomères ou leurs mélanges ainsi que ses sels métalliques, ses sels d'addition avec les bases azotées, ses sels d'addition avec les acides et ses formes hydratées.

**7.** Un dérivé de la benzo[b]naphtyridine-1,8 selon la revendication 1, caractérisé en ce qu'il s'agit de l'acide fluoro-7 [(furyl-2)-3 pipérazinyl-1]-8 méthyl-1 oxo-4 dihydro-1,4 benzo[b]naphtyridine-1,8 carboxylique-3 sous ses formes isomères ou leurs mélanges ainsi que ses sels métalliques, ses sels d'addition avec les bases azotées, ses sels d'addition avec les acides et ses formes hydratées.

**8.** Procédé de préparation d'un dérivé de la benzo[b]naphtyridine-1,8 selon la revendication 1, caractérisé en ce que l'on fait agir un dérivé de la pipérazine de formule générale :

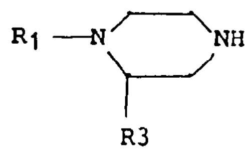

dans laquelle $R_1$ et $R_3$ sont définis comme dans la revendication 1, sur une benzo[b]naphtyridine-1,8 de formule générale :

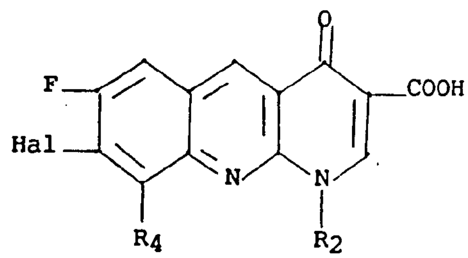

dans laquelle $R_2$ et $R_4$ sont définis comme dans la revendication 1 et Hal est un atome de fluor, de chlore ou de brome, si $R_4$ est un atome d'hydrogène, ou Hal et $R_4$ sont simultanément des atomes de fluor, puis, le cas échéant, lorsque l'on veut obtenir un produit selon la revendication 1 pour lequel $R_1$ est méthyle et lorsque l'on a obtenu le produit correspondant pour lequel $R_1$ est un atome d'hydrogène, transforme le produit obtenu en un dérivé de (méthyl-4 pipérazinyl-1)-8 benzo[b]naphtyridine, puis éventuellement transforme le produit obtenu en un sel.

9. Procédé de préparation d'un dérivé de la benzo[b]naphtyridine-1,8 selon la revendication 1, caractérisé en ce que l'on transforme l'ester de formule générale :

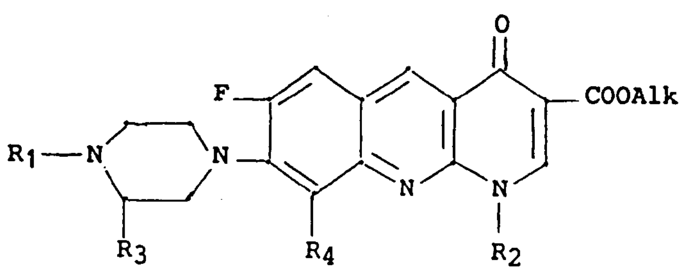

dans laquelle $R_1$, $R_2$, $R_3$ et $R_4$ sont définis comme dans la revendication 1, ou $R_2$ représente un radical alcoylamino protégé et Alk est un radical alcoyle droit ou ramifié contenant 1 à 4 atomes de carbone, par toute méthode connue pour obtenir un acide à partir d'un ester, sans toucher au reste de la molécule, puis, le cas échéant, élimine le groupement protecteur du radical alcoylamino, et/ou lorsque l'on veut préparer un produit selon la revendication 1, pour lequel $R_1$ est méthyle et lorsque l'on a obtenu le produit correspondant pour lequel $R_1$ est un atome d'hydrogène, transforme le produit obtenu en un dérivé de (méthyl-4 pipérazinyl-1)-8 benzo[b]:naphtyridine puis, éventuellement, prépare le sel du dérivé de benzo[b]naphtyridine obtenu.

10. Composition caractérisée en ce qu'elle contient au moins un dérivé selon la revendication 1, à l'état pur ou en association avec un ou plusieurs diluants ou adjuvants compatibles.

**Revendication pour les Etats contractants suivants : ES, GR**

1. Procédé de préparation d'un dérivé de la benzo[b]naphtyridine-1,8 de formule générale :

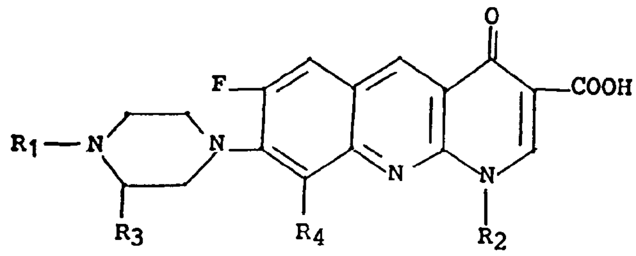

dans laquelle,

- $R_1$ représente un atome d'hydrogène ou un radical hydroxy ou alcoyle,
- $R_2$ représente un atome d'hydrogène ou un radical alcoyle, fluoroalcoyle, cycloalcoyle contenant 3 à 6 atomes de carbone, alcoyloxy ou alcoylamino,
- $R_3$ représente un radical phényle ou phénylalcoyle éventuellement substitué par un ou plusieurs atomes d'halogène ou radicaux alcoyle, cycloalcoyle contenant 3 à 6 atomes de carbone, alcoyloxy, cyano, amino, alcoylamino, dialcoylamino, alcoyloxyalcoyle, hydroxyalcoyle, hydroxyalcoyloxy, méthylènedioxy, aminoalcoyle, alcoylaminoalcoyle ou dialcoylaminoalcoyle dont les parties alcoyle peuvent former avec l'atome d'azote auquel elles sont rattachées, un hétérocycle à 5 ou 6 chaînons, ou représente un radical hétérocyclyle à 5 chaînons contenant 1 ou 2 hétéroatomes choisis parmi l'azote, l'oxygène ou le soufre, ou un radical N-alcoyl pyrrolyle, et
- $R_4$ représente un atome d'hydrogène ou un atome de fluor, ou bien
- $R_1$ est hydroxy, $R_2$ est méthyle, $R_3$ est un atome d'hydrogène et $R_4$ est défini comme ci-dessus,

et dont les radicaux alcoyle sont droits ou ramifiés et contiennent 1 à 4 atomes de carbone, sous ses formes isomères ou leurs mélanges ainsi que ses sels métalliques, ses sels d'addition avec les bases azotées, ses sels d'addition avec les acides et ses formes hydratées, caractérisé en ce que l'on fait agir un dérivé de la pipérazine de formule générale :

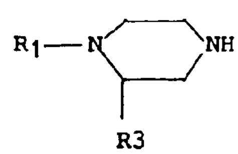

dans laquelle $R_1$ et $R_3$ sont définis comme ci-dessus sur une benzo[b]naphtyridine-1,8 de formule générale :

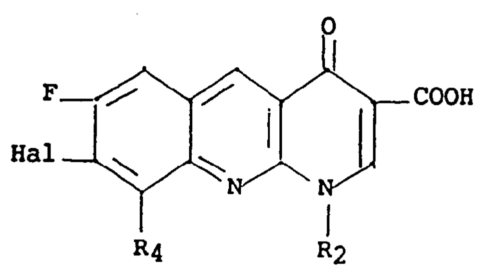

dans laquelle $R_2$ et $R_4$ sont définis comme ci-dessus et Hal est un atome de fluor, de chlore ou de brome, si $R_4$ est un atome d'hydrogène, ou Hal et $R_4$ sont simultanément des atomes de fluor, puis, le cas échéant, lorsque l'on veut obtenir un produit pour lequel $R_1$ est méthyle et lorsque l'on a obtenu le produit correspondant pour lequel $R_1$ est un atome d'hydrogène, transforme le produit obtenu en un dérivé de (méthyl-4 pipérazinyl-1)-8 benzo[b]naphtyridine, ou bien
caractérisé en ce que l'on transforme l'ester de formule générale :

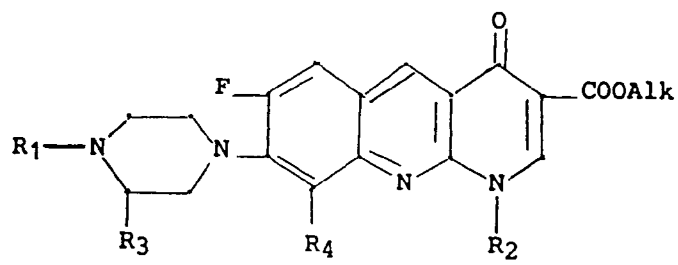

dans laquelle $R_1$, $R_2$, $R_3$ et $R_4$ sont définis comme ci-dessus, ou $R_2$ représente un radical alcoylamino protégé et Alk est un radical alcoyle droit ou ramifié contenant 1 à 4 atomes de carbone, par toute méthode connue pour obtenir un acide à partir d'un ester, sans toucher au reste de la molécule, puis, le cas échéant, élimine le groupement protecteur du radical alcoylamino, et/ou lorsque l'on veut préparer un produit pour lequel $R_1$ est méthyle et lorsque l'on a obtenu le produit correspondant pour lequel $R_1$ est un atome d'hydrogène, transforme le produit obtenu en un dérivé de (méthyl-4 pipérazinyl-1)-8 benzo[b]naphtyridine puis, éventuellement, prépare le sel du dérivé de benzo[b]naphtyridine obtenu.

## Patentansprüche

### Patentansprüche für folgende Vertragsstaaten : AT, BE, CH, DE, DK, FR, GB, IT, LI, LU, NL, SE

1. Benzo[b]-1,8-naphthyridinderivat, dadurch gekennzeichnet, daß es der allgemeinen Formel

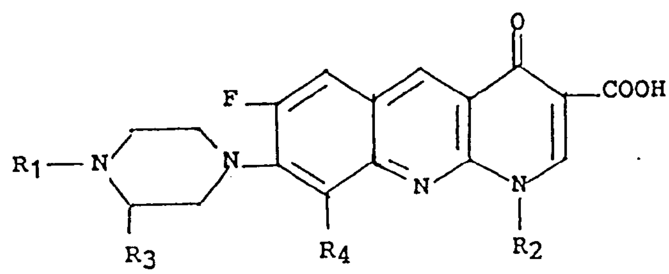

entspricht, worin
- $R_1$ ein Wasserstoffatom oder eine Hydroxy- oder Alkylgruppe bedeutet,
- $R_2$ für ein Wasserstoffatom oder einen Alkyl-, Fluoralkyl-, Cycloalkyl- mit 3 bis 6 Kohlenstoffatomen, Alkoxy- oder Alkylaminorest steht,
- $R_3$ einen Phenyl- oder Phenylalkylrest, gegebenenfalls substituiert durch einen oder mehrere Halogenatome oder Alkyl-, Cycloalkyl- mit 3 bis 6 Kohlenstoffatomen, Alkoxy-, Cyano-, Amino, Alkylamino, Dialkylamino-, Alkoxyalkyl-, Hydroxyalkyl-, Hydroxyalkoxy-, Methylendioxy-, Aminoalkyl-, Alkylaminoalkyl- oder Dialkylaminoalkylreste, deren Alkylteile mit dem Stickstoffatom, an das sie gebunden sind, einen Heterocyclus mit 5 oder 6 Kettengliedern bilden können, bedeutet, oder einen heterocyclischen Rest mit 5 Kettengliedern mit 1 oder 2 Heteroatomen, ausgewählt unter Stickstoff, Sauerstoff oder Schwefel, oder einen N-Alkylpyrrolylrest darstellt, und
- $R_4$ ein Wasserstoffatom oder ein Fluoratom wiedergibt, oder
- $R_1$ Hydroxy bedeutet, $R_2$ Methyl bedeutet, $R_3$ ein Wasserstoffatom bedeutet und $R_4$ wie vorstehend definiert ist,

und deren Alkylreste geradkettig oder verzweigt sind und 1 bis 4 Kohlenstoffatome aufweisen, in ihren isomeren Formen oder deren Gemische sowie deren Metallsalze, deren Additionssalze mit Stickstoffbasen, deren Additionssalze mit Säuren und deren hydratisierte Formen.

2. Benzo[b]-1,8-naphthyridinderivat gemäß Anspruch 1, dadurch gekennzeichnet, daß
- $R_1$ ein Wasserstoffatom oder einen Hydroxy- oder Alkylrest bedeutet,
- $R_2$ ein Wasserstoffatom oder einen Alkyl-, Fluoralkyl, Cycloalkyl- mit 3 bis 6 Kohlenstoffatomen oder Alkoxyrest darstellt,
- $R_3$ einen Phenyl- oder Phenylalkylrest, gegebenenfalls substituiert durch einen oder mehrere Halogenatome oder Alkyl-, Cycloalkyl- mit 3 bis 6 Kohlenstoffatomen, Alkoxy-, Cyano-, Amino-, Alkoxyalkyl-, Hydroxyalkoxy-, Methylendioxyreste, bedeutet, oder einen heterocyclischen Rest mit 5 Kettengliedern mit 1 oder 2 Heteroatomen, ausgewählt unter Stickstoff, Sauerstoff oder Schwefel, oder einen N-Alkylpyrrolylrest wiedergibt und

- $R_4$ ein Wasserstoffatom oder ein Fluoratom darstellt, oder
- $R_1$ Hydroxy bedeutet, $R_2$ Methyl bedeutet, $R_3$ ein Wasserstoffatom bedeutet und $R_4$ wie vorstehend definiert ist,

und deren Alkylreste geradkettig oder verzweigt sind und 1 bis 4 Kohlenstoffatome aufweisen, in dessen isomeren Formen oder deren Gemische sowie dessen Metallsalze, dessen Additionssalze mit Stickstoffbasen, dessen Additionssalze mit Säuren und dessen hydratisierte Formen.

3. Benzo[b]-1,8-naphthyridinderivat gemäß Anspruch 1, dadurch gekennzeichnet, daß es sich um die 7-Fluor-1-methyl-4-oxo-8-(3-phenylpiperazinyl-1)-1,4-dihydro-benzo[b]-1,8-naphthyridin-3-carbonsäure in ihren isomeren Formen oder deren Gemische sowie deren Metallsalze, deren Additionssalze mit Stickstoffbasen, deren Additionssalze mit Säuren und deren hydratisierte Formen handelt.

4. Benzo[b]-1,8-naphthyridinderivat gemäß Anspruch 1, dadurch gekennzeichnet, daß es sich um die 7-Fluor-1-methyl-8-[3-(4-methylphenyl)-piperazinyl-1]-4-oxo-1,4-dihydro-benzo[b]-1,8-naphthyridin-3-carb onsäure in ihren isomeren Formen oder deren Gemische sowie deren Metallsalze, deren Additionssalze mit Stickstoffbasen, deren Additionssalze mit Sauren und deren hydratisierte Formen handelt.

5. Benzo[b]-1,8-naphthyridinderivat gemäß Anspruch 1, dadurch gekennzeichnet, daß es sich um die 7-Fluor-8-[3-(4-methoxyphenyl)-piperazinyl-1]-1-methyl-4-oxo-1,4-dihydro-benzo[b]-1,8-naphthyridin-3-car bonsäure in ihren isomeren Formen oder deren Gemische sowie deren Metallsalze, deren Additionssalze mit Stickstoffbasen, deren Additionssalze mit Säuren und deren hydratisierte Formen handelt.

6. Benzo[b]-1,8-naphthyridinderivat gemäß Anspruch 1, dadurch gekennzeichnet, daß es sich um die 7,9-Difluor-8-[3-(4-methoxyphenyl)-piperazinyl-1]-1-methyl-4-oxo-1,4-dihydro-benzo[b]-1,8-naphthyridin-3-c arbonsäure in ihren isomeren Formen oder deren Gemische sowie deren Metallsalze, deren Additionssalze mit Stickstoffbasen, deren Additionssalze mit Säuren und deren hydratisierte Formen handelt.

7. Benzo[b]-1,8-naphthyridinderivat gemäß Anspruch 1, dadurch gekennzeichnet, daß es sich um die 7-Fluor-8-[3-(furyl-2)-piperazinyl-1]-1-methyl-4-oxo-1,4-dihydro-benzo[b]-1,8-naphthyridin-3-carbonsäure in ihren isomeren Formen oder deren Gemische sowie deren Metallsalze, deren Additionssalze mit Stickstoffbasen, deren Additionssalze mit Säuren und deren hydratisierte Formen handelt.

8. Verfahren zur Herstellung eines Benzo[b]-1,8-naphthyridinderivats gemäß Anspruch 1, dadurch gekennzeichnet, daß man ein Piperazinderivat der allgemeinen Formel

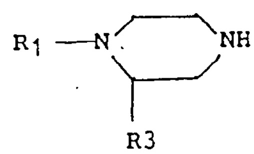

worin $R_1$ und $R_3$ wie in Anspruch 1 definiert sind, mit einem Benzo[b]-1,8-naphthyridin der allgemeinen Formel

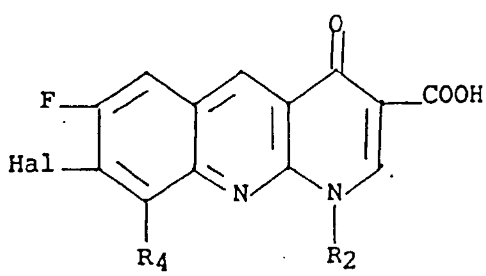

worin $R_2$ und $R_4$ wie in Anspruch 1 definiert sind und Hal für ein Fluor-, Chlor- oder Bromatom steht, wenn

$R_4$ ein Wasserstoffatom bedeutet, oder Hal und $R_4$ gleichzeitig Fluoratome bedeuten, umsetzt, gegebenenfalls danach, wenn man ein Produkt gemäß Anspruch 1 erhalten möchte, worin $R_1$ einen Methylrest bedeutet und wenn man das entsprechende Produkt erhalten hat, worin $R_1$ ein Wasserstoffatom bedeutet, das erhaltene Produkt in ein 8-(4-Methylpiperazinyl-1)-benzo[b]-naphthyridinderivat überführt, wonach man gegebenenfalls das erhaltene Produkt in ein Salz überführt.

9. Verfahren zur Herstellung eines Benzo[b]-1,8- naphthyridins gemäß Anspruch 1, dadurch gekennzeichnet, daß man den Ester der allgemeinen Formel

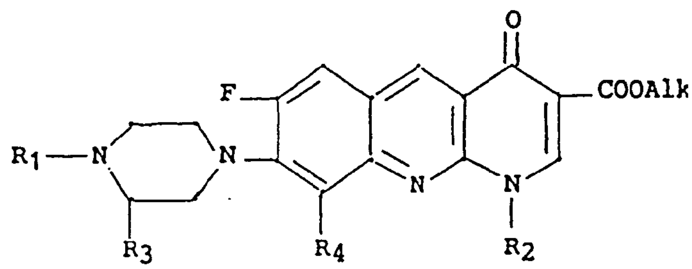

worin $R_1$, $R_2$, $R_3$ und $R_4$ wie in Anspruch 1 definiert sind, oder $R_2$ einen geschützten Alkylaminorest bedeutet und Alk für einen geradkettigen oder verzweigten Alkylrest mit 1 bis 4 Kohlenstoffatomen steht, nach jeder für die Erzielung einer Säure aus einem Ester ohne Beeinträchtigung des Restes des Moleküls bekannten Methode umwandelt, wonach man gegebenenfalls die Schutzgruppe des Alkylaminorestes eliminiert und/oder wenn man ein Produkt des Anspruchs 1 herstellen möchte, worin $R_1$ ein Methylrest ist, und wenn man das entsprechende Produkt erhalten hat, worin $R_1$ ein Wasserstoffatom bedeutet, das erhaltene Produkt in ein 8-(4-Methylpiperazinyl-1)-benzo[b]-naphthyridinderivat überführt, wonach man gegebenenfalls das Salz des erhaltenen Benzo[b]-naphthyridinderivats herstellt.

10. Zusammensetzung dadurch gekennzeichnet, daß sie zumindest ein Derivat gemäß Anspruch 1 in reiner Form oder in Assoziation mit einem oder mehreren verträglichen Verdünnungsmitteln oder Adjuvantien enthält.

**Patentanspruch für folgende Vertragstaaten : ES, GR**

1. Verfahren zur Herstellung eines Benzo[b]-1,8-naphthyridinderivats der allgemeinen Formel

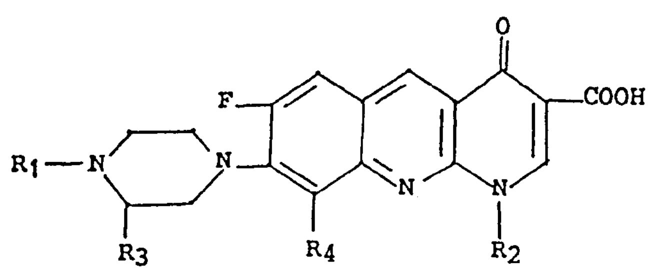

worin

- $R_1$ ein Wasserstoffatom oder eine Hydroxy- oder Alkylgruppe bedeutet,
- $R_2$ für ein Wasserstoffatom oder einen Alkyl-, Fluoralkyl-, Cycloalkyl- mit 3 bis 6 Kohlenstoffatomen, Alkoxy- oder Alkylaminorest steht,
- $R_3$ einen Phenyl- oder Phenylalkylrest, gegebenenfalls substituiert durch einen oder mehrere Halogenatome oder Alkyl-, Cycloalkyl- mit 3 bis 6 Kohlenstoffatomen, Alkoxy-, Cyano-, Amino-, Alkylamino-, Dialkylamino-, Alkoxyalkyl-, Hydroxyalkyl, Hydroxyalkoxy-, Methylendioxy-, Aminoalkyl-, Alkylaminoalkyl- oder Dialkylaminoalkylreste, deren Alkylteile mit dem Stickstoffatom, an das sie gebunden sind, einen Heterocyclus mit 5 oder 6 Kettengliedern bilden können, bedeutet, oder einen Heterocyclus mit 5 Kettengliedern mit 1 oder 2 Heteroatomen, ausgewählt unter Stickstoff, Sauerstoff oder Schwefel, oder einen N-Alkylpyrrolylrest darstellt, und
- $R_4$ ein Wasserstoffatom oder ein Fluoratom bedeutet, oder

- $R_1$ Hydroxy bedeutet, $R_2$ Methyl bedeutet, $R_3$ ein Wasserstoffatom bedeutet und $R_4$ wie vorstehend definiert ist,

und deren Alkylreste geradkettig oder verzweigt sind und 1 bis 4 Kohlenstoffatome aufweisen, in seinen isomeren Formen oder von dessen Gemischen sowie dessen Metallsalzen, dessen Additionssalzenmit Stickstoffbasen, dessen Additionssalzenmit Säuren und dessen hydratisierten Formen, dadurch gekennzeichnet, daß man ein Piperazinderivat der allgemeinen Formel

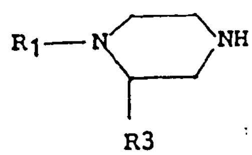

worin $R_1$ und $R_3$ wie vorstehend definiert sind, mit einem Benzo[b]-1,8-naphthyridin der allgemeinen Formel

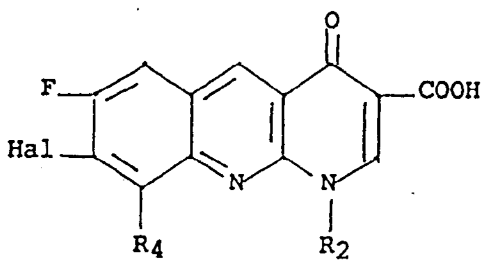

worin $R_2$ und $R_4$ wie vorstehend definiert sind und Hal für ein Fluor-, Chlor- oder Bromatom steht, wenn $R_4$ ein Wasserstoffatom bedeutet, oder Hal und $R_4$ gleichzeitig Fluoratome bedeuten, umsetzt, wonach man gegebenenfalls, wenn man ein Produkt erhalten möchte, worin $R_1$ Methyl bedeutet, und wenn man das entsprechende Produkt erhalten hat, worin R1 ein Wasserstoffatom bedeutet, das erhaltene Produkt in ein 8-(4-Methylpiperazinyl-1)-benzo[b]-naphthyridinderivat überführt, oder

dadurch gekennzeichnet, daß man den Ester der allgemeinen Formel

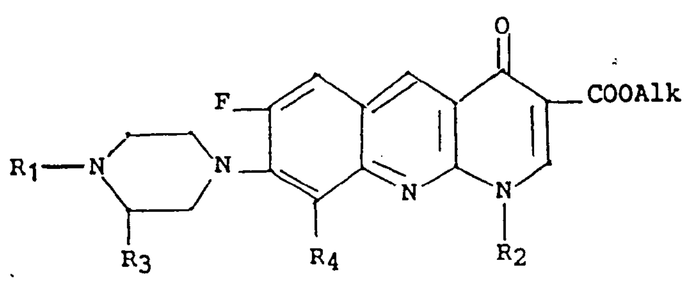

worin $R_1$, $R_2$, $R_3$ und $R_4$ wie vorstehend definiert sind, oder $R_2$ einen geschützten Alkylaminorest bedeutet und Alk für einen geradkettigen oder verzweigten Alkylrest mit 1 bis 4 Kohlenstoffatomen steht, nach jeder für die Erzielung einer Säure aus einem Ester ohne Beeinträchtigung des Restes des Moleküls bekannten Methode umwandelt, wonach man gegebenenfalls die Schutzgruppe des Alkylaminorestes eliminiert und/oder wenn man ein Produkt herstellen möchte, worin $R_1$ Methyl bedeutet, und wenn man das entsprechende Produkt erhalten hat, worin $R_1$ für ein Wasserstoffatom steht, das erhaltene Produkt in ein 8-(4-Methylpiperazinyl-1)-benzo[b]-naphthyridin überführt, wonach man gegebenenfalls das Salz des erhaltenen Benzo[b]-naphthyridinderivats herstellt.

## Claims

**Claims for the following Contracting States : AT, BE, CH, DE, DK, FR, GB, IT, LI, LU, NL, SE**

1. A benzo[b][1,8]naphthyridine derivative, characterised in that it corresponds to the general formula:

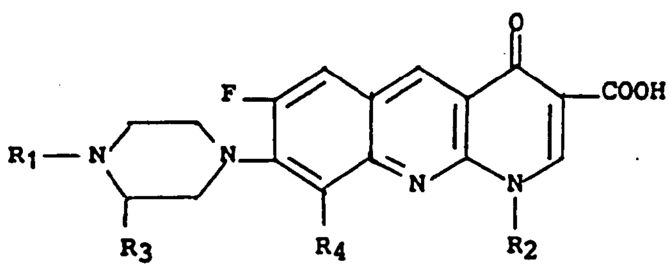

in which,
- $R_1$ represents a hydrogen atom or a hydroxyl or alkyl radical,
- $R_2$ represents a hydrogen atom or an alkyl, fluoroalkyl, cycloalkyl containing 3 to 6 carbon atoms, alkyloxy or alkylamino radical,
- $R_3$ represents a phenyl or phenylalkyl radical optionally substituted by one or a number of halogen atoms or alkyl, cycloalkyl containing 3 to 6 carbon atoms, alkyloxy, cyano, amino, alkylamino, dialkylamino, alkyloxyalkyl, hydroxyalkyl, hydroxyalkyloxy, methylenedioxy, aminoalkyl, alkylaminoalkyl or dialkylaminoalkyl, in which the alkyl parts can form, with the nitrogen atom to which they are attached, a 5- or 6-membered heterocycle, radicals, or represents a 5-membered heterocyclyl radical containing 1 or 2 heteroatoms chosen from nitrogen, oxygen or sulphur, or an N-alkylpyrrolyl radical, and
- $R_4$ represents a hydrogen atom or a fluorine atom, or else
- $R_1$ is hydroxyl, $R_2$ is methyl, $R_3$ is a hydrogen atom and $R_4$ is defined as above,

and the alkyl radicals of which are straight or branched and contain 1 to 4 carbon atoms, in its isomeric forms or mixtures thereof, as well as its metal salts, its addition salts with nitrogenous bases, its addition salts with acids and its hydrated forms.

2. A benzo[b][1,8]naphthyridine derivative according to Claim 1, characterised in that:
- $R_1$ represents a hydrogen atom or a hydroxyl or alkyl radical,
- $R_2$ represents a hydrogen atom or an alkyl, fluoroalkyl, cycloalkyl containing 3 to 6 carbon atoms or alkyloxy radical,
- $R_3$ represents a phenyl or phenylalkyl radical optionally substituted by one or a number of halogen atoms or alkyl, cycloalkyl containing 3 to 6 carbon atoms, alkyloxy, cyano, amino, alkyloxyalkyl, hydroxyalkyloxy or methylenedioxy radicals, or represents a 5-membered heterocyclyl radical containing 1 or 2 heteroatoms chosen from nitrogen, oxygen or sulphur, or an N-alkylpyrrolyl radical, and
- $R_4$ represents a hydrogen atom or a fluorine atom, or else
- $R_1$ is hydroxyl, $R_2$ is methyl, $R_3$ is a hydrogen atom and $R_4$ is as defined above,

and the alkyl radicals of which are straight or branched and contain 1 to 4 carbon atoms, in its isomeric forms or mixtures thereof, as well as its metal salts, its addition salts with nitrogenous bases, its addition salts with acids and its hydrated forms.

3. A benzo[b][1,8 ]naphthyridine derivative according to Claim 1, characterised in that it is 7-fluoro-1-methyl-4-oxo-8-(3-phenyl-1-piperazinyl)-1,4-dihydrobenzo[b][1,8]naphthyridine-3-carboxylic acid in its isomeric forms or mixtures thereof, as well as its metal salts, its addition salts with nitrogenous bases, its addition salts with acids and its hydrated forms.

4. A benzo[b][1,8]naphthyridine derivative according to Claim 1, characterised in that it is 7-fluoro-1-methyl-8-[3-(4-methylphenyl)-1-piperazinyl]-4-oxo-1,4-dihydrobenzo[b][1,8]naphthyridine-3-carboxylic acid in its isomeric forms or mixtures thereof, as well as its metal salts, its addition salts with nitrogenous bases, its addition salts with acids and its hydrated forms.

5. A benzo[b][1,8]naphthyridine derivative according to Claim 1, characterised in that it is 7-fluoro-8-[3-(4-methoxyphenyl)-1-piperazinyl]-1-methyl-4-oxo-1,4-dihydrobenzo[b][1,8]naphthyridine-3-carboxylic acid in its isomeric forms or mixtures thereof, as well as its metal salts, its addition salts with nitrogenous bases, its addition salts with acids and its hydrated forms.

6. A benzo[b][1,8]naphthyridin derivative according to Claim 1, characterised in that it is 7,9-difluoro-8-[3-(4-methoxyphenyl)-1-piperazinyl]-1-methyl-4-oxo-1,4-dihydrobenzo[b][1,8]naphthyridine-3-carboxylic acid in its isomeric forms or mixtures thereof, as well as its metal salts, its addition salts with nitrogenous bases, its addition salts with acids and its hydrated forms.

7. A benzo[b][1,8]naphthyridine derivative according to Claim 1, characterised in that it is 7-fluoro-8-[3-(2-furyl)-1-piperazinyl]-1-methyl-4-oxo-1,4-dihydrobenzo[b][1,8]naphthyridine-3-carboxylic acid in its isomeric forms or mixtures thereof, as well as its metal salts, its addition salts with nitrogenous bases, its addition salts with acids and its hydrated forms.

8. Process for the preparation of a benzo[b][1,8]naphthyridine derivative according to Claim 1, characterised in that a piperazine derivative of general formula:

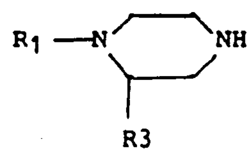

in which $R_1$ and $R_3$ are defined as in Claim 1, is reacted with a benzo[b][1,8]naphthyridine of general formula:

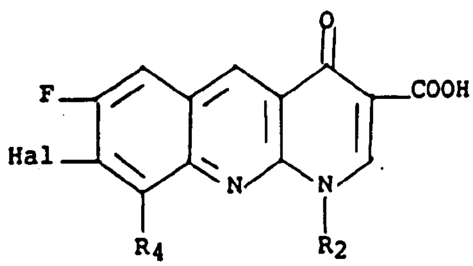

in which $R_2$ and $R_4$ are defined as in Claim 1 and Hal is a fluorine, chlorine or bromine atom, if $R_4$ is a hydrogen atom, or Hal and $R_4$ are simultaneously fluorine atoms, then, if appropriate, when it is desired to obtain a product according to Claim 1 for which $R_1$ is methyl and when the corresponding product for which $R_1$ is a hydrogen atom has been obtained, the product obtained is converted to an 8-(4-methyl-1-piperazinyl)benzo[b]naphthyridine derivative, and the product obtained is then optionally converted to a salt.

9. Process for the preparation of a benzo[b][1,8]naphthyridine derivative according to Claim 1, characterised in that the ester of general formula:

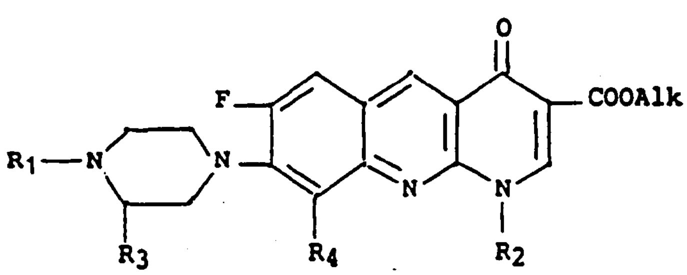

in which $R_1$, $R_2$, $R_3$ and $R_4$ are defined as in Claim 1, or $R_2$ represents a protected alkylamino radical and Alk is a straight or branched alkyl radical containing 1 to 4 carbon atoms, is converted by any method

known for producing an acid from an ester, without affecting the remainder of the molecule, then, if appropriate, the protective group of the alkylamino radical is removed, and/or, when it is desired to prepare a product according to Claim 1 for which $R_1$ is methyl and when the corresponding product for which $R_1$ is a hydrogen atom has been obtained, the product obtained is converted to an 8-(4-methyl-1-piperazinyl)benzo[b]naphthyridine derivative, and then, optionally, the salt of the benzo[b]naphthyridine derivative obtained is prepared.

**10.** Composition, characterised in that it contains at least one derivative according to Claim 1 in the pure state or in combination with one or a number of compatible diluents or adjuvants.

**Claim for the following Contracting states : ES, GR**

**1.** Process for the preparation of a benzo[b][1,8]naphthyridine derivative of general formula:

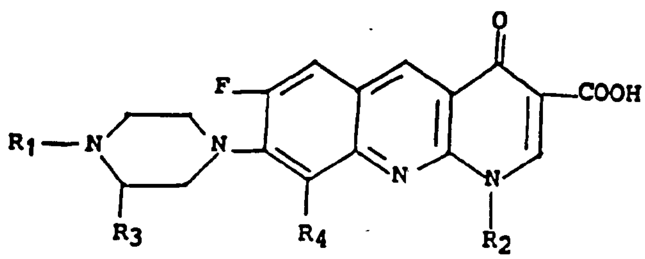

in which,
- $R_1$ represents a hydrogen atom or a hydroxyl or alkyl radical,
- $R_2$ represents a hydrogen atom or an alkyl, fluoroalkyl, cycloalkyl containing 3 to 6 carbon atoms, alkyloxy or alkylamino radical,
- $R_3$ represents a phenyl or phenylalkyl radical optionally substituted by one or a number of halogen atoms or alkyl, cycloalkyl containing 3 to 6 carbon atoms, alkyloxy, cyano, amino, alkylamino, dialkylamino, alkyloxyalkyl, hydroxyalkyl, hydroxyalkyloxy, methylenedioxy, aminoalkyl, alkylaminoalkyl or dialkylaminoalkyl, in which the alkyl parts can form, with the nitrogen atom to which they are attached, a 5- or 6-membered heterocycle, radicals, or represents a 5-membered heterocyclyl radical containing 1 or 2 heteroatoms chosen from nitrogen, oxygen or sulphur, or an N-alkylpyrrolyl radical, and
- $R_4$ represents a hydrogen atom or a fluorine atom, or else
- $R_1$ is hydroxyl, $R_2$ is methyl, $R_3$ is a hydrogen atom and $R_4$ is defined as above,

and the alkyl radicals of which are straight or branched and contain 1 to 4 carbon atoms, in its isomeric forms or mixtures thereof, as well as its metal salts, its addition salts with nitrogenous bases, its addition salts with acids and its hydrated forms, characterised in that a piperazine derivative of general formula:

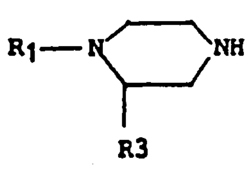

in which $R_1$ and $R_3$ are defined as above, is reacted with a benzo[b][1,8]naphthyridine of general formula:

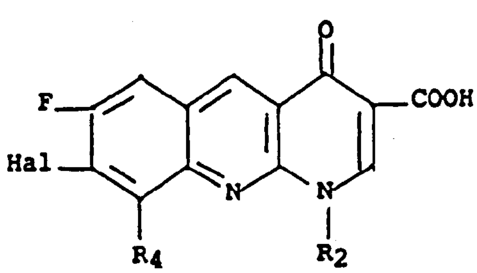

in which $R_2$ and $R_4$ are defined as above and Hal is a fluorine, chlorine or bromine atom, if $R_4$ is a hydrogen atom, or Hal and $R_4$ are simultaneously fluorine atoms, then, if appropriate, when it is desired to obtain a product for which $R_1$ is methyl and when the corresponding product for which $R_1$ is a hydrogen atom has been obtained, the product obtained is converted to an 8-(4-methyl-1-piperazinyl)benzo[b]naphthyridine derivative, or else
characterised in that the ester of general formula:

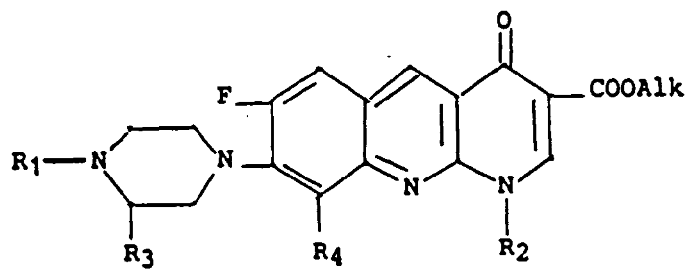

in which $R_1$, $R_2$ $R_3$ and $R_4$ are defined as above, or $R_2$ represents a protected alkylamino radical and Alk is a straight or branched alkyl radical containing 1 to 4 carbon atoms, is converted by any method known for producing an acid from an ester, without affecting the remainder of the molecule, then, if appropriate, the protective group of the alkylamino radical is removed, and/or, when it is desired to prepare a product for which $R_1$ is methyl and when the corresponding product for which $R_1$ is a hydrogen atom has been obtained, the product obtained is converted to an 8-(4-methyl-1-piperazinyl)benzo[b]naphthyridine derivative, and then, optionally, the salt of the benzo[b]naphthyridine derivative obtained is prepared.